# EUROPEAN PATENT APPLICATION

(11) **EP 1 857 440 A1**
(43) Date of publication of application: **21.11.2007**
(21) Application number: 06008140.3
(22) Date of filing: 19.04.2006
(51) Int. Cl.: C07C 311/08, A61K 31/08

(54) **Novel compounds, isomer thereof, or pharmaceutically acceptable salts thereof as vanilloid receptor antagonist; and pharmaceutical compositions containing the same**

(71) Applicant: Amorepacific Corporation, Seoul 140-777 (KR)
(72) Inventor: Kim, Hee-Doo College of Pharmacy, Seoul 140-742 (KR); Suh, Young-Ger College of Pharmacy, Seoul 151-010 (KR); Park, Hyeung, Geun College of Pharmacy, Seoul 151-010 (KR); Oh, Uh, Taek College of Pharmacy, Seoul 151-010 (KR); Park, Seol, Rin, Pusan-si 608-757 (KR); Park, Young-Ho, Seoul 156-775 (KR); Shin, Song, Seok, Gyeonggi-do 449-959 (KR); Kim, Sun-Young, Seoul 137-873 (KR); Kim, Jin, Kwan, Gyeonggi-do 441-350 (KR); Lee, Ki-Wha, Seoul 135-230 (KR); Woo, Byoung, Young, Gyeonggi-do 446-724 (KR); Jeong, Yeon, Su, Kyunggi-do 449-749 (KR)
(74) Representative: Blodig, Wolfgang

(57) **Abstract**

This present invention relates to novel compounds, isomer thereof or pharmaceutically acceptable salts thereof as vanilloid receptor (Vanilloid Receptor 1; VR1; TRPV1) antagonist; and a pharmaceutical composition containing the same.

The present invention provides a pharmaceutical composition for preventing or treating a disease such as pain, migraine, arthralgia, neuralgia, neuropathies, nerve injury, skin disorder, urinary bladder hypersensitiveness, irritable bowel syndrome, fecal urgency, a respiratory disorder, irritation of skin, eye or mucous membrane, stomach-duodenal ulcer, inflammatory diseases, ear disease, and heart disease.

## Description

### Technical Field

The present invention relates to novel compounds, isomer thereof or pharmaceutically acceptable salts thereof as vanilloid receptor (Vanilloid Receptor 1 ; VR1 ; TRPV1) antagonist; and a pharmaceutical composition containing the same.

### Background Art

As diseases associated with the activity of vanilloid receptor (Nagy et al., 2004, Eur. J. Pharmacol. 500, 351-369) pain such as acute pain, chronic pain, neuropathic pain, post-operative pain, rheumatic arthritic pain, osteoarthritic pain, postherpetic neuralgia, neuralgia, headache, and migraine (Petersen et al., 2000, Pain, 88, pp125-133; Walker et al., 2003, J. Pharmacol. Exp. Ther., 304, pp56-62); nerve-related diseases such as neuropathies, HIV-related neuropathy, nerve injury, neurodegeneration, and stroke(Park et al., 1999, Arch. Pharm. Res. 22, pp432-434; Kim et al., 2005, J. Neurosci. 25(3), pp662-671); diabetic neuropathy (Kamei et al., 2001, Eur. J. Pharmacol. 422, pp83-86); fecal urgency; irritable bowel syndrome (Chan et al., 2003, Lancet, 361, pp385-391); inflammatory bowel disease (Yiangou et al., 2001, Lancet, 357, pp1338-1339); disease of digestive organ such as stomach-duodenal ulcer and Crohn's disease (Holzer P, 2004, Eur. J. Pharm. 500, pp231-241; Geppetti et al., 2004, Br. J. Pharmacol., 141, pp1313-1320); respiratory disease such as asthma, chronic obstructive pulmonary disease( Hwang et al., 2002, Curr. Opin. Pharmacol. pp235-242; Spina et al., 2002, Curr. Opin. Pharmacol. pp264-272); urinary incontinence (Birder et al., 2002, Nat. Neuroscience, 5, pp856-860); urinary bladder hypersensitiveness (Birder et al., 2001, Proc. Natl. Acad. Sci. 98, pp13396-13401); neurotic/allergic/inflammatory skin disease such as psoriasis, pruritus and vitiligo(Southall et al., 2003, J. Pharmacol. Exp. Ther., 304, pp217-222); irritation of skin, eye or mucous membrane (Tominaga et al., 1998, Neuron 21 pp531-543); hyperacusis; tinnitus; vestibular hypersensitiveness (Balaban et al., 2003, Hear Res. 175, pp165-70); cardiac disease such as myocardial ischemia etc.(Scotland et al., 2004, Circ. Res. 95, pp1027-1034; Pan et al., 2004, Circulation, 110, pp1826-1831) can be enumerated.

The vanilloid receptor (VR1) is the receptor for capsaicin (8-methyl-N-vanillyl-6-nonenamide), a pungent ingredient in hot peppers. The molecular cloning thereof was also reported in 1997 (Caterina et al., 1997, Nature 389, pp816-824). This receptor is a non-selective cation channel composed of 6 transmembrane domains and belongs to the TRP channel family. Recently, it was named TRPV1. On the other hand, it is known that the vanilloid receptor is activated by stimuli such as capsaicin, resiniferatoxin, heat, acids, anandamide, lipid metabolites or the like; thus it plays a crucial role as a molecular integrator of physico-chemically noxious stimuli in mammals (Tominaga et al., 1998, Neuron 21 pp531-543; Hwang et al., 2000, PNAS, 97, pp6155-6160). Activation of the vanilloid receptor by endogenous/exogenous stimuli leads to not only transmission of noxious stimuli, but also liberation of neuropeptides such as substance P, CGRP (Calcitonin Gene-Related Peptide) and the like, thereby causing neurogenic inflammation. The vanilloid receptor is highly expressed in primary afferent sensory neurons. It is also reportedly expressed in various organs and tissues such as the bladder, kidney, lung, intestine and skin, and in the central nervous system (CNS) including the brain and non-neuronal tissues (Mezey et al., 2000, PNAS, 97, pp3655-3660; Stander et al., 2004, Exp. Dermatol. 13, pp129-139; Cortright et al., 2001, BBRC, 281, pp1183-1189). In particular, TRPV1 receptor knock-out mice exhibit a normal response to harmful physical stimuli, but show a reduced response to painful heat and vanilloid, and exhibit little hyperalgesia to thermal stimuli even in an inflammatory state (Caterina et al., 2000, Science 288, pp306-313; Davis et al., 2000, Nature 405, pp183-187; Karai et al., 2004, J. Clin. Invest., 113, pp1344-1352). Lately, an additional role of the vanilloid receptor is also expected by the report of possibility that the vanilloid receptor may be present in the form of a heteromultimer with TRPV3, another TRP channel (Smith et al., 2002, Nature, 418, pp186-190).

As mentioned above, the vanilloid receptor knock-out mice exhibited reduced responses to thermal or noxious stimuli, thus raising the possibility that vanilloid receptor antagonists may be utilized for prevention or treatment of various pain conditions. Recently, this possibility is supported by the report that the well-known vanilloid receptor antagonist, capsazepine also decreases hyperalgesia caused by physical stimuli in models of inflammatory and neuropathic pain (Walker et al., 2003, JPET, 304, pp56-62; Garcia-Martinez et al., 2002, Proc. Natl. Acad. Sci. 99, 2374-2379). In addition, treatment of the primary culture of afferent sensory neurons with the vanilloid receptor agonist, capsaicin etc., results in damage to nerve functions and furthermore death of nerve cells. The vanilloid receptor antagonist exerts defense actions against such damage to nerve functions and nerve cell death (Holzer P, 1991, Pharmacological Reviews, 43, pp143-201; Mezey et al., 2000, PNAS, 97, 3655-3660). The vanilloid receptor is expressed on sensory neurons distributed in myenteric ganglia, muscle layer, mucosa and epithelial cells in all regions of the gastrointestinal tract. In particular, the vanilloid receptor is highly expressed in inflammatory disorders of the colon and ileum.

In addition, activation of the vanilloid receptor stimulates sensory nerves, which in turn causes release of neuropeptides which are known to play a critical role in pathogenesis of bowel disorders. The role of the vanilloid receptor in development of gastrointestinal disorders is well elucidated and documented in recent scientific papers and journals, for example, Holzer P, 2004, Eur. J. Pharm. 500, pp231-241; Geppetti et al., 2004, Br. J. Pharmacol., 141, pp1313-1320. According to such references, it seems that the vanilloid receptor antagonists will be effective for prevention or treatment of gastrointestinal diseases such as gastro-esophageal reflux disease (GERD) and gastroduodenal ulcer (DU). It has been reported that the number of sensory nerves expressing the vanilloid receptor is increased in patients suffering from irritable bowel syndromes and such increased expression of the vanilloid receptor is known to be involved in the development of the disease (Chan et al., 2003, Lancet, 361, pp385-391). Other investigations showed that expression of the vanilloid receptor is significantly increased in patients suffering from inflammatory bowel disorders. Taken together, it appears that the vanilloid receptor antagonist may also be therapeutically effective for such bowel disorders (Yiangou et al., 2001, Lancet, 357, pp1338-1339). The vanilloid receptor-expressing afferent nerves are abundantly distributed in airway mucosa. Bronchial hypersensitivity is very similar to hyperalgesia, and protons and lipoxygenase products, known as endogenous ligands for the vanilloid receptor, are well known as crucial factors responsible for development of asthma and chronic obstructive pulmonary diseases (Hwang et al., 2002, Curr. Opin. Pharmacol. pp235-242; Spina et al., 2002, Curr. Opin. Pharmacol. pp264-272). Further, it has been reported that air-polluting substances, which are a kind of asthma-causing substances, i.e., particulate matter specifically acts on the vanilloid receptor and such action is inhibited by capsazepine, thus suggesting the possible applicability of vanilloid receptor antagonists to respiratory diseases (Veronesi et al., 2001, NeuroToxicology, 22, pp795-810). Urinary bladder hypersensitiveness and urinary incontinence are caused by various central/peripheral nerve disorders or injury, and capsaicin-responsive sensory nerves play an important role in bladder function control and inflammation. In addition, immunoreactivity of the vanilloid receptor was reported in urinary bladder epithelium (urothelium) in rats and it was found that bladder overactivity induced by capsaicin was due to stimulation of vanilloid receptors present in nerve fibers, or various transmitters which are released by vanilloid receptors (Birder et al., 2001, Proc. Natl. Acad. Sci. 98, pp13396-13401). Further, VR1 (TRPV1) -/- mice are anatomically normal, but exhibit higher frequency of low-amplitude, non-voiding bladder contraction, as compared to wild type mice, thus indicating that the vanilloid receptor affects functions of the bladder (Birder et al., 2002, Nat. Neuroscience, 5, pp856-860). Some of vanilloid agonists are recently under development as therapeutics for treating bladder diseases. Vanilloid receptors are distributed in human epidermal keratinocytes as well as in primary afferent sensory nerves(Denda et al., 2001, Biochem. Biophys. Res. Commun., 285, pp1250-1252; Inoue et al., 2002, Biochem. Biophys. Res. Commun., 291, pp124-129), and are then involved in transmission of various noxious stimuli and pains such as skin irritation and pruritus, thereby having close correlation with etiology of dermatological diseases and disorders such as skin inflammation, due to neurogenic/non-neurogenic factors. This is supported by the report that the vanilloid receptor antagonist, capsazepine inhibits inflammatory factors in human skin cells (Southall et al., 2003, J. Pharmacol. Exp. Ther., 304, pp217-222).

Based on the above-mentioned information, development of various vanilloid receptor antagonists is under way, and some patents and patent applications relating to vanilloid receptor antagonists under development were recently published, in which the above mentioned information is described well (Rami et al., 2004, Drug Discovery Today: Therapeutic Strategies, 1, pp97-104).

As a result of extensive and intensive studies based on the theoretical background discussed above, the present inventors have synthesized novel compounds having antagonistic activity by selective action on a vanilloid receptor and thus completed the present invention.

Consequently, present inventors have surprisingly found that the introduction of oxygen atom to the linker between the two phenyl rings provides novel compounds which exhibit potent and highly selective activity at vanilloid receptors. Therefore, it is an object of the present invention to provide novel compounds useful as a potent antagonist for a vanilloid receptor, isomer thereof and pharmaceutically acceptable salts thereof; and a pharmaceutical composition comprising the same.

### Disclosure of the invention

The present invention provides a novel compound of the formula (I), an isomer and/or a pharmaceutically acceptable salt thereof; and a pharmaceutical composition containing the same. 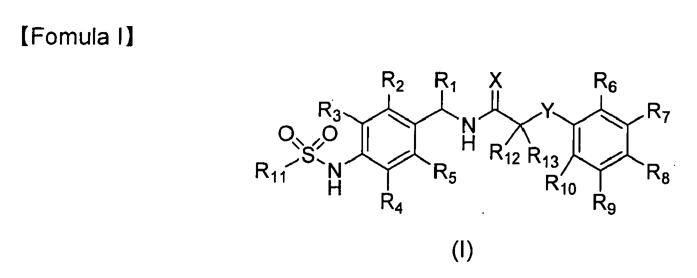 wherein,
X is O, S, or N-CN;
Y is O or S;
R₁ is hydrogen or C1-C5 alkyl;
R₂, R₃, R₄, and R₅ are independently hydrogen, halogen, nitro, cyano, carboxy, C1-C5 alkyl, C2-C5 alkenyl, C2-C5 alkynyl, halo (C1-C5) alkyl, C1-C5 alkylthio, C1-C5 alkoxy, or C1-C5 alkoxycarbonyl;
R₆, R₇, R₉, and R₁₀ are independently hydrogen, hydroxy, halogen, nitro, carboxy, C1-C5 alkyl, C2-C5 alkenyl, C2-C5 alkynyl, halo (C1-C5) alkyl, halo (C1-C5) alkoxy, C1-C5 alkylthio, C1-C5 alkylsulfonyl, C1-C5 alkoxy, C1-C5 alkylcarbonyl, C1-C5 alkoxycarbonyl, C2-C5alkenyloxy, (C1-C5)alkoxy(C1-C5)alkyl, (C1-C5)alkoxycarbonyl(C1-C5)alkyl, (C1-C5)alkylcarbonyloxy(C1-C3)alkyl, (C1-C5)alkoxy(C1-C5)alkylamino, (C1-C5)alkoxy(C1-C5)alkoxy, C3-C6 cycloalkyl, C3-C5 cycloalkylmethyl, piperidinyl, or morpholinyl;
R₈ is halogen, C1-C5 alkyl, C2-C5 alkenyl, C2-C5 alkynyl, halo (C1-C5) alkyl, halo (C1-C5) alkoxy, C1-C5 alkylthio, C1-C5 alkylsulfonyl, C1-C5 alkoxy, C1-C5 alkylcarbonyl, C1-C5 alkoxycarbonyl, C2-C5alkenyloxy, (C1-C5)alkoxy(C1-C5)alkyl, (C1-C5)alkoxycarbonyl(C1-C5)alkyl, (C1-C5)alkoxy(C1-C5)alkylamino, (C1-C5)alkoxy(C1-C5)alkoxy, C3-C6 cycloalkyl, mono- or di- (C3-C5)cycloalkyl(C1-C3)alkyl, piperidinyl, morpholinyl, phenyl, pyridyl, or pyrimidinyl, wherein each (C3-C5)cycloalkyl may be unsubstituted or substituted with one or more methyl groups, and phenyl may be unsubstituted or substituted with one or more substituent selected from halogen, nitro, carboxy, C1-C5 alkyl, C2-C5 alkenyl, halo (C1-C5) alkyl, or C1-C5 alkoxy;
R₈ and R₉ may also form together -OCH₂O-, C2-C4 alkylene, or -NHN=N-;
R₁₁ is C1-C5 alkyl or C2-C5 alkenyl; and
R₁₂ and R₁₃ are hydrogen, halogen, or C1-C5 alkyl.

One preferred aspect of the present invention is a compound of the formula (I), an isomer thereof, and/or a pharmaceutically acceptable salt thereof;
wherein,
X is O, S, or N-CN,
Y is O or S;
R₁ is hydrogen or C1-C3 alkyl;
R₂, R₃, R₄, and R₅ are independently hydrogen, halogen, nitro, cyano, methyl, ethyl, ethenyl, ethynyl, trifluoromethyl, methoxy, ethoxy, or methoxycarbonyl;
R₆, R₇, R₉, and R₁₀ are independently hydrogen, hydroxy, halogen, nitro, carboxy, C1-C5 alkyl, C2-C5 alkenyl, C2-C5 alkynyl, halo (C1-C5) alkyl, halo (C1-C5) alkoxy, C1-C5 alkylthio, C1-C5 alkylsulfonyl, C1-C5 alkoxy, C1-C5 alkylcarbonyl, C1-C5 alkoxycarbonyl, C2-C5alkenyloxy, (C1-C5)alkoxy(C1-C5)alkyl, (C1-C5)alkoxycarbonyl(C1-C5)alkyl, (C1-C5)alkylcarbonyloxy(C1-C3)alkyl, (C1-C5)alkoxy(C1-C5)alkylamino, (C1-C5)alkoxy(C1-C5)alkoxy, C3-C6 cycloalkyl, piperidinyl, or morpholinyl;
R₈ is halogen, C1-C5 alkyl, C2-C5 alkenyl, C2-C5 alkynyl, halo (C1-C5) alkyl, halo (C1-C5) alkoxy, C1-C5 alkoxy, C1-C5 alkylcarbonyl, C1-C5 alkoxycarbonyl, C2-C5alkenyloxy, (C1-C5)alkoxy(C1-C5)alkyl, C3-C6 cycloalkyl, mono- or di- (C3-C5)cycloalkyl(C1-C3)alkyl, piperidinyl, or morpholinyl, wherein each (C3-C5)cycloalkyl may be unsubstituted or substituted with one or more methyl groups;
R₈ and R₉ may also form together -OCH₂O-, C2-C4 alkylene, or -NHN=N-;
R₁₁ is C1-C3 alkyl or C2-C3 alkenyl; and
R₁₂ and R₁₃ are hydrogen, halogen, or C1-C3 alkyl.

Another preferred aspect of the present invention is a compound according to the formula (II), an isomer, and/or a pharmaceutically acceptable salt thereof; 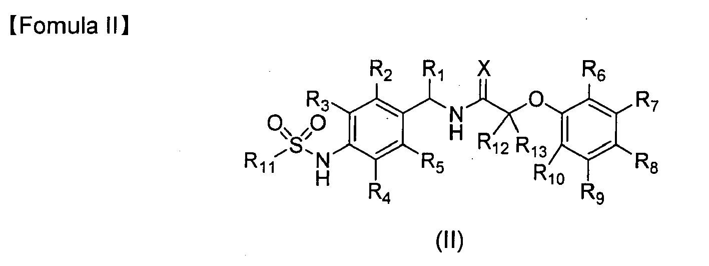 wherein,
X is O, S, or N-CN;
R₁ is hydrogen or C1-C5 alkyl;
R₂, R₃, R₄, and R₅ are independently hydrogen, halogen, nitro, cyano, methyl, ethyl, ethenyl, ethynyl, trifluoromethyl, methoxy, ethoxy, or methoxycarbonyl;
R₆, R₇, R₉, and R₁₀ are independently hydrogen, hydroxy, halogen, nitro, carboxy, C1-C5 alkyl, C2-C5 alkenyl, C2-C5 alkynyl, halo (C1-C5) alkyl, halo (C1-C5) alkoxy, C1-C5 alkylthio, C1-C5 alkylsulfonyl, C1-C5 alkoxy, C1-C5 alkylcarbonyl, C1-C5 alkoxycarbonyl, C2-C5alkenyloxy, (C1-C5)alkoxy(C1-C5)alkyl, (C1-C5)alkoxycarbonyl(C1-C5)alkyl, (C1-C5)alkylcarbonyloxy(C1-C3)alkyl, (C1-C5)alkoxy(C1-C5)alkylamino, (C1-C5)alkoxy(C1-C5)alkoxy, C3-C6 cycloalkyl, C3-C5 cycloalkylmethyl, piperidinyl, or morpholinyl;
R₈ is halogen, C1-C5 alkyl, C2-C5 alkenyl, C2-C5 alkynyl, halo (C1-C5) alkyl, halo (C1-C5) alkoxy, C1-C5 alkoxy, C1-C5 alkylcarbonyl, C1-C5 alkoxycarbonyl, C2-C5alkenyloxy, (C1-C5)alkoxy(C1-C5)alkyl, (C1-C5)alkoxycarbonyl(C1-C5)alkyl, (C1-C5)alkoxy(C1-C5)alkylamino, (C1-C5)alkoxy(C1-C5)alkoxy, C3-C6 cycloalkyl, mono- or di- (C3-C5)cycloalkyl(C1-C3)alkyl, piperidinyl, morpholinyl, or phenyl, wherein each (C3-C5)cycloalkyl may be unsubstituted or substituted with one or more methyl groups, and phenyl may be unsubstituted or substituted with one or more substituent selected from halogen, nitro, carboxy, C1-C5 alkyl, C2-C5 alkenyl, halo (C1-C5) alkyl, or C1-C5 alkoxy;
R₈ and R₉ may also form together -OCH₂O-, C2-C4 alkylene, or -NHN=N-;
R₁₁ is C1-C5 alkyl or C2-C5 alkenyl; and
R₁₂ and R₁₃ are hydrogen, halogen, or C1-C5 alkyl.

One preferred embodiment of the present invention relates to the above described compounds of formula (I) or (II) having one or more of the following features:
- R₁₁ is methyl or ethyl and particularly preferably methyl:
- X is O or N-CN and particularly preferably O;
- R₁ is hydrogen or methyl;
- R₁₂ is hydrogen and R₁₃ is hydrogen or methyl;
- YisO
- R₈ is isopropyl, sec-butyl, t-butyl, isobutyl, t-amyl, trifluoromethyl, pentafluoroethyl, heptafluoropropyl, heptafluoro-iso-propyl, nonafluorobutyl, nonafluoro-t-butyl, 2-propynyl; and even more preferably isopropyl, sec-butyl, t-butyl, isobutyl, pentafluoroethyl, heptafluoropropyl, heptafluoro-iso-propyl, nonafluorobutyl, nonafluoro-t-butyl or 2-propynyl; and particularly preferably isopropyl, sec-butyl, t-butyl or isobutyl
- R₃ and R₄ are independently hydrogen, fluoro, chloro, bromo, nitro, cyano, methyl, ethyl, ethenyl, ethynyl, trifluoromethyl, methoxy, ethoxy, or methoxycarbonyl; particularly preferably R₃ is hydrogen, fluoro, chloro, bromo, nitro, cyano, methyl, ethyl, ethenyl, and R₄ is hydrogen, fluoro, chloro, methyl or ethyl;
R₂, and R₁₀ are hydrogen;
R₅ is hydrogen or fluoro;
R₆, R₇, and R₉ are independently hydrogen, halogen, nitro, methyl, ethyl, propyl, isopropyl, butyl, isobutyl, sec-butyl, t-butyl, ethenyl, propenyl, 2-propynyl, trifluoromethyl, pentafluoroethyl, trifluoromethoxy, methylthio, methoxy, ethoxy, propoxy, allyloxy, acetyl, t-butoxycarbonylmethyl, ethoxycarbonylethyl, t-butylcarbonyloxymethyl, ethylcarbonyloxymethyl, isopropylcarbonyloxymethyl, methoxyethylamino, methoxyethoxy, cyclopropyl, or morpholinyl; and R₆, R₇, and R₉ are particularly preferably independently hydrogen, halogen, nitro, methyl, ethyl, ethenyl, trifluoromethyl, trifluoromethoxy, methoxy, ethoxy, methoxyethylamino, methoxyethoxy, or morpholinyl.

In another preferred embodiment, in the above Formula (I) and (II),
X is O, S, or N-CN;
R₁ is hydrogen, methyl, or ethyl;
R₂, R₃, R₄, and R₅ are independently hydrogen, fluoro, chloro, bromo, nitro, cyano, methyl, ethyl, ethenyl, ethynyl, trifluoromethyl, methoxy, ethoxy, or methoxycarbonyl;
R₆, R₇, R₉, and R₁₀ are independently hydrogen, halogen, nitro, carboxy, methyl, ethyl, propyl, isopropyl, butyl, isobutyl, sec-butyl, t-butyl, ethenyl, propenyl, 2-propynyl, trifluoromethyl, pentafluoroethyl, heptafluoropropyl, hexafluoropropyl, trifluoromethoxy, methylthio, methoxy, ethoxy, propoxy, allyloxy, acetyl, t-butoxycarbonylmethyl, ethoxycarbonylethyl, methoxycarbonylmethyl, t-butylcarbonyloxymethyl, ethylcarbonyloxymethyl, isopropylcarbonyloxymethyl, methoxyethylamino, methoxyethoxy, C3-C6 cycloalkyl, piperidinyl, or morpholinyl;
R₈ is fluoro, chloro, bromo, iodo, C3-C5 alkyl, halo (C1-C5) alkyl, C2-C5 alkynyl, propenyl, butenyl, trifluoromethoxy, pentafluoroethoxy, ethoxy, propoxy, isopropoxy, acetyl, propionyl, allyloxy, methoxyethyl, C3-C5 cycloalkyl, mono-cyclopropylmethyl, dicyclopropylmethyl, 2,2-dimethylcyclopropyl, 2,3-dimethylcyclopropyl, piperidinyl, or morpholinyl;
R₈ and R₉ may also form together-OCH₂O-, C2-C4 alkylene, or -NHN=N-;
R₁₁ is C1-C3 alkyl or C2-C3 alkenyl; and
R₁₂ and R₁₃ are hydrogen, halogen, or C1-C3 alkyl.

In another preferred embodement, in the above Formula (I) and (II),
X is O, S, or N-CN;
R₁ is hydrogen, methyl, or ethyl;
R₃ and R₄ are independently hydrogen, fluoro, chloro, bromo, nitro, cyano, methyl, ethyl, ethenyl, ethynyl, trifluoromethyl, methoxy, ethoxy, or methoxycarbonyl;
R₂, R₅, and R₁₀ are hydrogen;
R₆, R₇, and R₉ are independently hydrogen, halogen, nitro, carboxy, methyl, ethyl, propyl, isopropyl, butyl, isobutyl, sec-butyl, t-butyl, ethenyl, propenyl, 2-propynyl, trifluoromethyl, pentafluoroethyl, heptafluoropropyl, trifluoromethoxy, methylthio, methoxy, ethoxy, propoxy, allyloxy, acetyl, t-butoxycarbonylmethyl, ethoxycarbonylethyl, t-butylcarbonyloxymethyl, ethylcarbonyloxymethyl, isopropylcarbonyloxymethyl, methoxyethylamino, methoxyethoxy, cyclopropyl, cyclobutyl, piperidinyl, or morpholinyl;
R₈ is selected from C3-C5 alkyl, halo (C1-C5) alkyl, or C2-C5 alkynyl;
R₁₁ is methyl; and
R₁₂ and R₁₃ are hydrogen, fluoro, chloro, methyl, or ethyl.

In another preferred embodement, in the above Formula (I) and (II),
X is O, or N-CN;
R₁ is hydrogen, methyl, or ethyl;
R₃ and R₄ are independently hydrogen, fluoro, chloro, bromo, nitro, cyano, methyl, ethyl, ethenyl, ethynyl, trifluoromethyl, methoxy, ethoxy, or methoxycarbonyl;
R₂, R₅, and R₁₀ are hydrogen;
R₆, R₇, and R₉ are independently hydrogen, halogen, nitro, methyl, ethyl, propyl, isopropyl, butyl, isobutyl, sec-butyl, t-butyl, ethenyl, propenyl, 2-propynyl, trifluoromethyl, pentafluoroethyl, trifluoromethoxy, methylthio, methoxy, ethoxy, propoxy, allyloxy, acetyl, t-butoxycarbonylmethyl, ethoxycarbonylethyl, t-butylcarbonyloxymethyl, ethylcarbonyloxymethyl, isopropylcarbonyloxymethyl, methoxyethylamino, methoxyethoxy, cyclopropyl, or morpholinyl;
R₈ is isopropyl, sec-butyl, t-butyl, isobutyl, t-amyl, trifluoromethyl, pentafluoroethyl, heptafluoropropyl, heptafluoro-iso-propyl, nonafluorobutyl, nonafluoro-t-butyl, or 2-propynyl;
R₁₁ is methyl; and
R₁₂ and R₁₃ are hydrogen, methyl, or ethyl.

Preferably, in the above Formula (I) and (II),
X is O or N-CN;
R₁ is hydrogen or methyl;
R₃ and R₄ are independently hydrogen, fluoro, chloro, bromo, nitro, cyano, methyl, ethyl, ethenyl, ethynyl, or trifluoromethyl;
R₂, R₅, and R₁₀ are hydrogen;
R₆, R₇, and R₉ are independently hydrogen, halogen, nitro, methyl, ethyl, propyl, isopropyl, butyl, isobutyl, sec-butyl, t-butyl, ethenyl, propenyl, 2-propynyl, trifluoromethyl, pentafluoroethyl, trifluoromethoxy, methylthio, methoxy, ethoxy, propoxy, allyloxy, acetyl, t-butoxycarbonylmethyl, ethoxycarbonylethyl, t-butylcarbonyloxymethyl, ethylcarbonyloxymethyl, methoxyethylamino, methoxyethoxy, cyclopropyl, or morpholinyl;
R₈ is isopropyl, sec-butyl, t-butyl, isobutyl, t-amyl, trifluoromethyl, pentafluoroethyl, heptafluoropropyl, or 2-propynyl;
R₁₁ is methyl; and
R₁₂ and R₁₃ are hydrogen or methyl.

Preferably, in the above Formula (I) and (II),
X is O or N-CN;
R₁ is hydrogen or methyl;
R₃ and R₄ are independently hydrogen, fluoro, chloro, bromo, nitro, cyano, methyl, ethyl, ethenyl, ethynyl, trifluoromethyl, methoxy, ethoxy, or methoxycarbonyl;
R₂, R₅, and R₁₀ are hydrogen;
R₆ and R₉ are independently hydrogen, halogen, nitro, methyl, ethyl, propyl, isopropyl, butyl, isobutyl, sec-butyl, t-butyl, ethenyl, propenyl, 2-propynyl, trifluoromethyl, pentafluoroethyl, trifluoromethoxy, methylthio, methoxy, ethoxy, propoxy, allyloxy, acetyl, t-butoxycarbonylmethyl, t-butylcarbonyloxymethyl, ethylcarbonyloxymethyl, methoxyethylamino, methoxyethoxy, cyclopropyl, or morpholinyl;
R₇ is hydrogen or halogen;
R₈ is isopropyl, sec-butyl, t-butyl, isobutyl, pentafluoroethyl, or heptafluoropropyl;
R₁₁ is methyl; and
R₁₂ and R₁₃ are hydrogen or methyl.

Preferably, in the above Formula (I) and (II),
X is O;
R₁ is hydrogen or methyl;
R₃ is hydrogen, fluoro, chloro, bromo, nitro, cyano, methyl, ethyl, ethenyl, ethynyl, or methoxy;
R₄ is hydrogen, fluoro, chloro, methyl or ethyl;
R₂, R₅, R₁₀, and R₁₂ are hydrogen;
R₆ and R₉ are independently hydrogen, fluoro, chloro, bromo, iodo, nitro, methyl, ethyl, propyl, isopropyl, butyl, isobutyl, sec-butyl, t-butyl, ethenyl, propenyl, 2-propynyl, trifluoromethyl, pentafluoroethyl, trifluoromethoxy, methylthio, methoxy, ethoxy, propoxy, t-butylcarbonyloxymethyl, methoxyethylamino, methoxyethoxy, or morpholinyl;
R₇ is hydrogen or fluoro;
R₈ is isopropyl, sec-butyl, t-butyl, isobutyl, or pentafluoroethyl;
R₁₁ is methyl; and
R₁₃ is methyl or hydrogen.

More preferably, in the above Formula (I) and (II),
X is O;
R₁ is hydrogen or methyl;
R₃ is hydrogen, fluoro, chloro, methyl, ethyl, ethenyl, or ethynyl;
R₄ is hydrogen, fluoro, chloro, methyl or ethyl;
R₂, R₅, R₁₀, R₁₂, and R₁₃ are hydrogen;
R₆ and R₉ are independently hydrogen, fluoro, chloro, bromo, nitro, methyl, ethyl, ethenyl, trifluoromethyl, trifluoromethoxy, methoxy, ethoxy, methoxyethylamino, methoxyethoxy, or morpholinyl;
R₇ is hydrogen or fluoro; R₈ is isopropyl or t-butyl; and
R₁₁ is methyl.

Preferred examples of compounds according to formula (I) and (II) are selected from the group consisting of;
2-(4-tert-Butylphenoxy)-N-(4-methanesulfonylaminobenzyl)acetamide,
2-(4-tert-Butylphenoxy)-N-(3-fluoro-4-methanesulfonylaminobenzyl)acetamide,
2-(4-tert-Butylphenoxy)-N-(4-methanesulfonylamino-3-vinylbenzyl)acetamide,
2-(4-tert-Butylphenoxy)-N-(3-fluoro-4-methanesulfonylamino-5-vinylbenzyl)acetamide,
2-(4-tert-Butylphenoxy)-N-(3-ethynyl-5-fluoro-4-methanesulfonylaminobenzyl)acetamide,
2-(4-tert-Butylphenoxy)-N-(3-ethyl-5-fluoro-4-methanesulfonylaminobenzyl)acetamide,
2-(4-tert-Butylphenoxy)-N-(3-chloro-4-methanesulfonylamino-5-methylbenzyl)acetamide,
2-(4-tert-Butylphenoxy)-N-(5-fluoro-4-methanesulfonylamino-2-methylbenzyl)acetamide,
2-(4-tert-Butylphenoxy)-N-(3-chloro-4-methanesulfonylaminobenzyl)acetamide,
2-(4-tert-Butylphenoxy)-N-(3-fluoro-4-methanesulfonylamino-5-methylbenzyl) acetamide,
(R)-2-(4-tert-Butylphenoxy)-N-[1-(4-methanesulfonylaminophenyl)ethyl]acetamide,
(R)-2-(4-tert-Butylphenoxy)-N-[1-(3-fluoro-4-methanesulfonylaminophenyl)ethyl]acetamide,
(R)-2-(4-tert-Butylphenoxy)-N-(3-fluoro-4-methanesulfonylaminobenzyl)propionamide,
(R)-2-(4-tert-Butylphenoxy)-N-[1-(4-methanesulfonylaminophenyl)ethyl]-N'-cyanoacetamidine,
2-(4-tert-Butylphenoxy)-N-(3-fluoro-4-methanesulfonylaminobenzyl)-N'-cyanoacetamidine,
2-(4-tert-Butylphenylsulfanyl)-N-(3-ethynyl-5-fluoro-4-methanesulfonylaminobenzyl)acetamide,
2-[4-(*tert*-butyl)phenoxy]-N-(3-cyano-5-fluoro-4-methanesulfonylaminobenzyl)acetamide,
2-(4-tert-Butylphenoxy)-2-fluoro-N-(3-fluoro-4-methanesulfonylaminobenzyl)acetamide,
N-(3-Fluoro-4-methanesulfonylaminobenzyl)-2-(4-isopropylphenoxy)acetamide, and
N-(3-Fluoro-4-methanesulfonylaminobenzyl)-2-(4-sec-butylphenoxy)acetamide.

The compounds of formula (I) or (II) according to the present invention can chemically be synthesized by the following reaction schemes. However, these are given only for illustration of the invention and not intended to limit them.

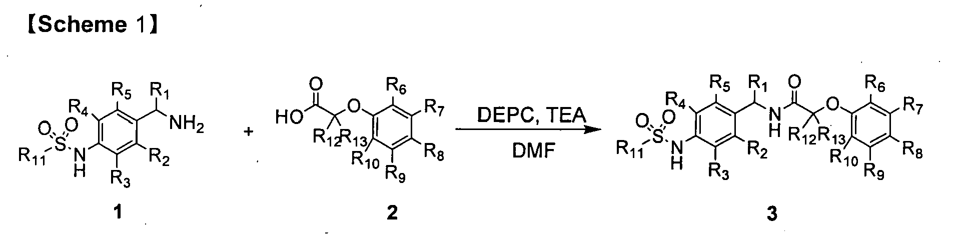

The Scheme 1 shows a proposed process for synthesizing the acetamide compound with phenoxy groups (3). The phenoxyacetic acid compound (2) is reacted with the benzylamine compound (1) and diethylcyanophosphate (DEPC) to yield the phenoxyacetamide compound (3).

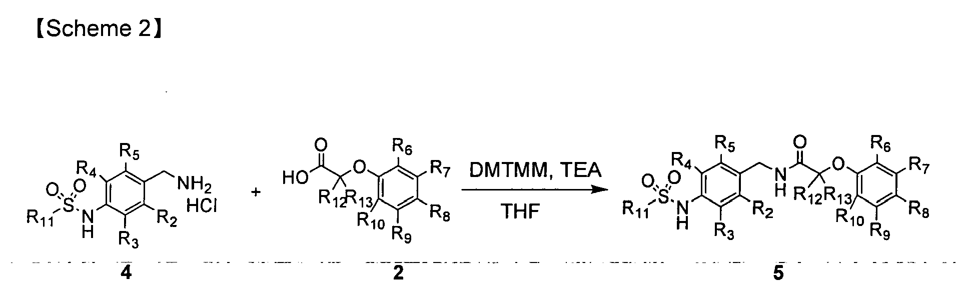

The Scheme 2 shows another process for synthesizing the acetamide compound (5). The benzylamine compound (4) is reacted with the α-phenoxyacetic acid compound (2) and DMTMM {4-(4,6-dimethoxy-1,3,5-triazin-2-yl)-4-methylmorpholinium chloride} (Tetrahedron Lett., 1999, 40, 5327) instead of DEPC to yield the benzyl phenoxyacetamide compound (5).

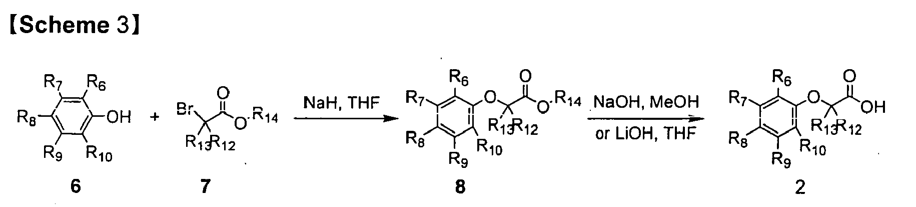

The Scheme 3 shows a proposed process for synthesizing α-phenoxyacetic acid compound (2) with various substituents. Phenol compound (6) is reacted with alkyl α-bromoacetate compound (7) to yield alkyl α-phenoxyacetate compound (8). The compound (8) is hydrolyzed with sodium hydroxide in methanol or lithium hydroxide in THF to yield α-phenoxyacetic acid compound (2) with various substituents.

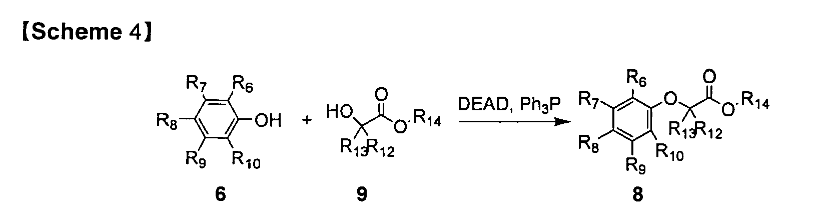

The Scheme 4 shows another process for synthesizing alkyl 2-phenoxyacetate compound (8). Phenol compound (6) is reacted with alkyl α-hydroxyacetate compound (9) under Mitsunobu reaction (Synthesis, 1981, 1) to yield alkyl α-phenoxyacetate compound (8). The compound (8) is hydrolyzed using base in aqueous solvent according to the same procedure as described in scheme 3.

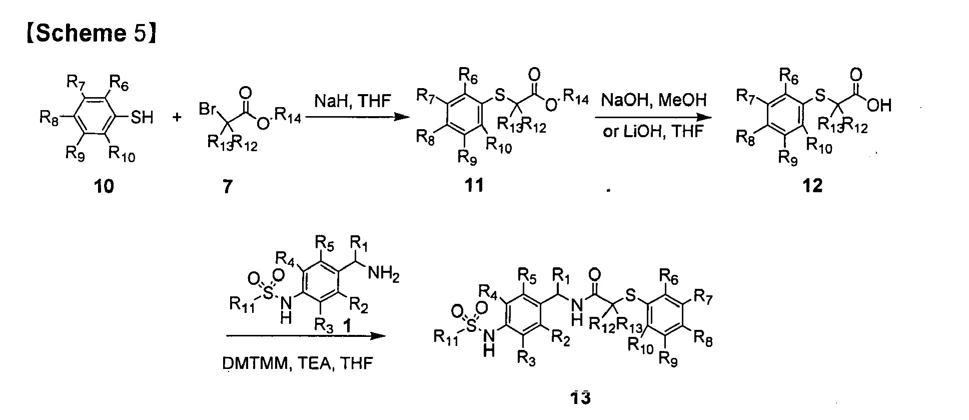

The Scheme 5 shows a proposed process for synthesizing the amide compound with phenylthio group (13). The thiophenol compound (10) is reacted with the alkyl α-bromoacetate compound (7) to yield the alkyl α-phenylthioacetate compound (11). The compound (11) is hydrolyzed to yield the phenythioacetic acid compound (12). The acid compound (12) is reacted with benzyl amine compound (1) to yield the acetamide compound with phenylthio group (13).

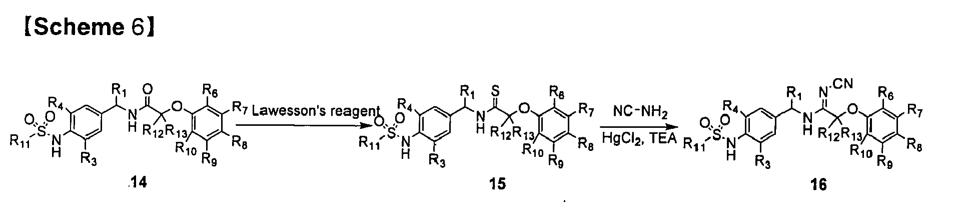

The Scheme 6 shows a proposed process for synthesizing the cyanoacetamide compound with phenoxy group (16). The compound (14) is reacted with Lawesson's reagent to yield the thioacetamide compound with phenoxy moiety (15). The thioacetatmide compound (15) is reacted with cyanamide and HgCl₂ to yield the cyanamide compound (16).

The present invention also provides a pharmaceutical composition comprising a compound of formula (I) or (II), an isomer thereof, or a pharmaceutically acceptable salt thereof as an active ingredient together with a pharmaceutically acceptable carrier.

In said pharmaceutical composition, a compound of formula (I) or (II), an isomer thereof, or a pharmaceutically acceptable salt thereof as an active ingredient together with an pharmaceutically acceptable carrier is present in an effective amount for preventing or treating pain, acute pain, neuropathic pain, post-operative pain, migraine, arthralgia, neuropathies, nerve injury, diabetic neuropathy, neurodegeneration, stroke, neurotic/allergic/inflammatory skin disease, psoriasis, pruritus, prurigo, urinary bladder hypersensitiveness, irritable bowel syndrome, fecal urgency, Crohn's disease, respiratory disorder such as asthma or chronic obstructive pulmonary disease, irritation of skin, eye or mucous membrane, stomach-duodenal ulcer, inflammatory bowel disease or inflammatory diseases.

The present invention also provides a pharmaceutical composition for preventing and treating a disease associated with the pathological stimulation and/or aberrant expression of vanilloid receptor, wherein said composition comprises a compound of formula (I) or (II), an isomer thereof or a pharmaceutically acceptable salt thereof; and pharmaceutically acceptable carrier.

The present invention also provides a pharmaceutical composition for preventing and treating a condition related to vanilloid receptor, wherein said composition comprises a compound of formula (I) or (II), an isomer thereof or a pharmaceutically acceptable salt thereof and pharmaceutically acceptable carrier.

In the above, said condition related to vanilloid receptor is pain, migraine, arthralgia, neuralgia, neuropathies, nerve injury, skin disorder, urinary bladder hypersensitiveness, irritable bowel syndrome, fecal urgency, a respiratory disorder, irritation of skin, eye or mucous membrane, stomach-duodenal ulcer, inflammatory diseases, ear disease, or heart disease.

More specifically, said condition related to vanilloid receptor is acute pain, chronic pain, neuropathic pain, post-operative pain, rheumatic arthritic pain, osteoarthritic pain, postherpetic neuralgia, diabetic neuropathy, HIV-related neuropathy, neurodegeneration, stroke, neurotic/allergic/inflammatory skin disease, psoriasis, pruritus, prurigo, asthma, chronic obstructive pulmonary disease, urinary incontinence, inflammatory bowel disease, hyperacusis, tinnitus, vestibular hypersensitiveness, or myocardial ischemia.

In one preferred aspect, the present invention provides a pharmaceutical composition for treating a condition selected from pain, inflammatory disease of the joints including inflammatory autoimmune diseases of the joints, urinary bladder hypersensitivity including urinary incontinence, stomach duodenal ulcer, irritable bowel syndrome (IBS) and inflammatory bowel disease (IBD), neurotic/allergic/inflammatory skin disease, psoriasis, asthma, chronic obstructive pulmonary disease (COPD), pruritus, or prurigo, wherein the composition comprises a compound, an isomer thereof or a pharmaceutically acceptable salt thereof according to anyone of formula (I) or (II), as defined further above.

More specific, the inventive compounds can be used in a pharmaceutical composition for treating pain, wherein the pain is or is associated with a condition selected from osteoarthritis ("OA"), rheumatoid arthritis ("RA"), Ankylosing Spondylitis ("AS"), diabetic neuropathic pain, post-operative pain, non-inflammatory musculoskeletal pain (including fibromyalgia, myofascial pain syndrome and back pain), migraine and other types of headaches.

If the compounds of the present invention are said to be useful to treat pain associated with osteoarthritis, it shall not be excluded that this also comprises the treatment of other signs and symptoms of osteoarthritis. Besides reducing the pain associated with osteoarthritis, the pharmacological intervention of osteoarthritis may be aimed at maintaining the mobility and minimizing the disability of the joints.

The term "inflammatory disease of the joints" includes diseases that involve to a more or less degree inflammatory processes in the joints, e.g. in knees, fingers, hips etc. An example for such a disease is osteoarthritis. The term "inflammatory disease of the joints" does also include diseases or conditions which may involve autoimmune processes, such as e.g. rheumatoid arthrtitis or ankylosing spondylitis. The inventive treatment of "inflammatory diseases of the joints" is primarily aimed at treating pain associated with these conditions but may also be aimed at improving the function of the affected joints, either directly or indirectly, e.g. by reducing the pain associated with the movement of said joints.

One outcome of the administration of the compounds of the present invention to patients suffering from an inflammatory disease of the joints may thus be reducing the pain experienced by the subject suffering from said disease relative to the pain experienced by the subject immediately before the administration of the compounds or combinations of the present invention. Another outcome of said treatment may be preventing the re-occurence of pain which has previously been reduced as a result of pharmaco- or other therapy. Another outcome of treatment may be decreasing the occurrence of and/or the severity of manifestations related to an inflammatory disease of the joints, including particularly osteoarthritis, rheumatoid arthritis ankylosing spondylitis. The treatment may suitably result in an improved functionality of the joints, such as decreased stiffness, improved mobility.

The term "osteoarthritis (OA)" as used herein typically includes diseases with a failure of a diarthrodial (movable, synovial-lined) joint. In idiopathic (primary) OA, the most common form of the disease, no predisposing factor is apparent. Secondary OA is attributable to an underlying cause. Pain and joint dysfunction are the cardinal symptoms of OA. The joint pain of OA is often described as a deep ache and is localized to the involved joint. Typically, the pain of OA is aggravated by joint use and relieved by rest, but, as the disease progresses, it may become persistent. Nocturnal pain, interfering with sleep, is seen particularly in advance OA of the hip and may be enervating. Stiffness of the involved joint on arising in the morning or after a period of inactivity may be prominent but usually lasts less than 20 minutes.

The term "RA'' refers to Rheumatoid Arthritis. RA is a chronic inflammatory autoimmune disease that causes the immune system to attack the joints, and particularly the synovium in the joint. The synovium becomes inflamed and causes swelling and pain. Cardinal symptoms of RA are joint pain and stiffness but additional symptoms include muscle aches, anemia and fever. Diagnosis of RA can be confirmed by detecting an antibody in the blood called the "rheumatic (or "rheumatoid") factor" and/or by a blood sedimentation test. Other useful and common tests are the detection of the "antinuclear antibody" or the "C-reactive protein".

"AS" stands for Ankylosing Spondylitis, which is a chronic, progressive autoimmune disease characterized by arthritis, inflammation and eventual immobility of the joints, particularly the spinal joints. It causes pain and stiffness in the back (often in the morning hours) as a result of ongoing swelling and irritation of the spinal joints (vertebrae). Inflammation of the tendons and ligaments that connect and provide support to the vertebrae can lead to pain and tenderness in the ribs, shoulder blades, hips, thighs, shins, heels and along the bony points of the spines.

If the compounds according to the present invention are said to be of use in

treating pain associated with an inflammatory autoimmune disease of the joints, this refers to the administration of the compounds or combinations of the compounds of the present invention to reduce at least one pain symptom experienced by a subject suffering from an inflammatory autoimmune disease of the joints including back pain, joint pain and muscle pain associated with RA or AS. Besides the pain relief, treatment of an inflammatory autoimmune disease of the joints may also include a decrease of the inflammation and/or swelling of the synovium and may help to improve the functionality (i.e. maintaining mobility, and minimizing disability) of the joints, in particular in patients suffering from RA or AS.

Treatment of "non-inflammatory musculoskeletal pain" refers to the administration of the compounds or combinations of the compounds of the present invention to reduce the pain experienced by a subject suffering from non-inflammatory musculoskeletal pain including back pain, fibromyalgia, and myofascial pain syndrome. One outcome of treatment may be reducing the pain experienced by the subject relative to the pain experienced by the subject immediately before the administration of the compounds or combinations of the present invention. Another outcome of treatment may be preventing reoccurence of pain which has previously been reduced as a result of pharmacotherapy. Another outcome of treatment may be decreasing the occurrence of and/or the severity of manifestations related to non-inflammatory musculoskeletal pain including back pain, fibromyalgia, and myofascial pain syndrome. The treatment may suitably result in a reduction of increased muscle sensitivity characterized by pain evoked by a normally non-nociceptive stimulus (allodynia) or increased pain intensity evoked by nociceptive stimuli (hyperalgesia). Finally, the treatment of non-inflammatory musculoskeletal pain can also improve the associated symptoms of back pain, fibromyalgia, and myofascial pain syndrome.

The terms "fibromyalgia" or "FMS" relates to a syndrome that causes widespread pain and stiffness throughout the tissue that supports and moves bones and joints. Fibromyalgia can be diagnosed by the presence of excessive tenderness on applying pressure to at least 11 of 18 specific muscle-tendon sites.

"Myofascial pain syndrome" is a chronic non-degenerative, non-inflammatory musculoskeletal pain condition. Distinct areas within muscles or their delicate connective tissue coverings (fascia) become abnormally thickened or tight. When the myofascial tissues tighten and lose their elasticity, neurotransmitter ability to send and receive messages between the brain and body is damaged. Symptoms include muscle stiffness and aching and sharp shooting pains or tingling and numbness in areas distant from the trigger point. Most commonly trigger points are in the neck, back, or buttocks.

"Back pain" is a common non-infiammatory musculoskeletal pain condition that may be either acute or chronic. It may be caused by a variety of diseases and disorders that affect the lumbar spine. Low back pain is often accompanied by sciatica, which is pain that involves the sciatic nerve and is felt in the lower back, the buttocks, and the backs of the thighs.

The compounds of the present invention are also useful for treating signs and symptoms of an overactive bladder such as urinary incontinence, more specific urinary urge incontinence, urinary stress incontinence, urinary urgency, nocturia and/or urinary frequency.

The pharmaceutical compositions according to the present invention are preferably adapted for oral administration. Alternatively, if skin diseases are to be treated the pharmaceutical composition containing the inventive compounds may be also formulated for topical or transcutaneous use.

In another aspect, the present invention relates to a method for inhibiting vanilloid ligand from binding to vanilloid receptor in a patient, comprising contacting cells expressing vanilloid receptor in the patient with a compound of formula (I) or (II), an isomer thereof, or a pharmaceutically acceptable salt thereof.

In another aspect, the present invention relates to a method for preventing or treating a disease selected from pain, migraine, arthralgia, neuropathies, nerve injury, skin disorder, urinary bladder hypersensitiveness, irritable bowel syndrome, fecal urgency, a respiratory disorder, irritation of skin, eye or mucous membrane, stomach-duodenal ulcer, inflammatory diseases, which comprises administering to a mammal including a person in need thereof a therapeutically effective amount of a compound of formula (I) or (II), an isomer thereof, or a pharmaceutically acceptable salt thereof.

In the above method, the disease is also selected from acute pain, chronic pain, neuropathic pain, post-operative pain, diabetic neuropathy, neurodegeneration, stroke, neurotic/allergic/inflammatory skin disease, psoriasis, pruritus, prurigo, asthma, chronic obstructive pulmonary disease, urinary incontinence or inflammatory bowel disease.

In one preferred aspect of the invention, the above method is treating pain that is or that is associated with a condition selected from osteoarthritis ("OA"), rheumatoid arthritis ("RA"), Ankylosing Spondylitis ("AS"), diabetic neuropathic pain, non-inflammatory musculoskeletal pain (including fibromyalgia, myofascial pain syndrome and back pain), post-operative pain, migraine and other types of headache.

In another aspect, the present invention relates to the use of a compound of formula (I) or (II), an isomer thereof, or a pharmaceutically acceptable salt thereof as an antagonist of vanilloid receptor.

In another aspect, the present invention relates to the use of a compound of formula (I) or (II), an isomer thereof, or a pharmaceutically acceptable salt thereof for prevention or treatment of a condition related to vanilloid receptor, which is more specifically associated with the aberrant expression and/or aberrant activation of a vanilloid receptor.

In another aspect, the present invention relates to the use of a compound of formula (I) or (II), an isomer thereof, or a pharmaceutically acceptable salt thereof, in preparation of a medicament for prevention or treatment of a condition related to vanilloid receptor.

In a preferred aspect, the present invention relates to the use of a compound of formula (I) or (II), an isomer thereof, or a pharmaceutically acceptable salt thereof in the preparation of a medicament for the prevention or the treatment of a condition that is selected from pain, inflammatory autoimmune disease of the joints, urinary bladder hypersensitivity including urinary incontinence, stomach duodenal ulcer, irritable bowel syndrome (IBS) and inflammatory bowel disease (IBD), neurotic/allergic/inflammatory skin disease, psoriasis, asthma, chronic obstructive pulmonary disease (COPD), pruritus, or prurigo.

In a particularly preferred aspect, the present invention relates to the use of a compound for treating pain as described above, wherein the pain is or is associated with a condition that is selected from osteoarthritis ("OA"), rheumatoid arthritis ("RA"), Ankylosing Spondylitis ("AS"), diabetic neuropathic pain, post-operative pain, non-inflammatory musculoskeletal pain (including fibromyalgia, myofascial pain syndrome and back pain), migraine and other types of headaches.

Hereinafter, the formulating methods and kinds of excipients will be described, but the present invention is not limited to them.

A compound of formula (I) or (II), an isomer thereof or a pharmaceutically acceptable salt thereof according to the present invention can be prepared as a pharmaceutical composition containing pharmaceutically acceptable carriers, adjuvants, diluents and the like. For instance, the compounds of the present invention can be dissolved in oils, propylene glycol or other solvents which are commonly used to produce an injection. Suitable examples of the carriers include physiological saline, polyethylene glycol, ethanol, vegetable oils, isopropyl myristate, etc., but are not limited to them. For topical administration, the compounds of the present invention can be formulated in the form of ointment or cream.

The compound according to the present invention may also be used in the forms of pharmaceutically acceptable salts thereof, and may be used either alone or in combination or in admixture with other pharmaceutically active compounds.

The compounds of the present invention may be formulated into injections by dissolving, suspending or emulsifying in water-soluble solvent such as saline and 5% dextrose, or in water-insoluble solvents such as vegetable oils, synthetic fatty acid glyceride, higher fatty acid esters and propylene glycol. The formulations of the invention may include any of conventional additives such as dissolving agents, isotonic agents, suspending agents, emulsifiers, stabilizers and preservatives.

The preferable dose level of the compounds according to the present invention depends upon a variety of factors including the condition and body weight of the patient, severity of the particular disease, dosage form, and route and period of administration, but may appropriately be chosen by those skilled in the art. The compounds of the present invention are preferably administered in an amount ranging from 0.001 to 100 mg/kg of body weight per day, and more preferably from 0.01 to 30 mg/kg of body weight per day. Doses may be administered once a day, or several times a day with each divided portions. The compounds of the present invention are used in a pharmaceutical composition in an amount of 0.0001 ~ 10% by weight, and preferably 0.001 ~ 1% by weight, based on the total amount of the composition.

The pharmaceutical composition of the present invention can be administered to a mammalian subject such as rat, mouse, domestic animals, human being and the like via various routes. The methods of administration which may easily be expected include oral and rectal administration; intravenous, intramuscular, subcutaneous, intrauterine, duramatral and intracerebroventricular injections.

### Detailed description of the invention definitions

When describing the compounds, pharmaceutical compositions containing such compounds, methods of using such compounds and compositions, and use of such compounds and compositions, all terms used in the present application shall have the meaning usually employed by a relevant person skilled in the art, e.g. by a medicinal chemists, pharmacist or physician. By the way of example some definitions of specific groups are given below:

"Alkyl" includes monovalent saturated aliphatic hydrocarbyl groups. The hydrocarbon chain may be either straight-chained or branched. This term is exemplified by groups such as methyl, ethyl, n-propyl, isopropyl, n-butyl, iso-butyl, tert-butyl sec-butyl, tert-amyl.

"Alkoxy" includes the group-OR where R is alkyl. Particular alkoxy groups include, by way of example, methoxy, ethoxy, n-propoxy, isopropoxy, n-butoxy, tert-butoxy, iso-butoxy, sec-butoxy, n-pentoxy, 1,2-dimethylbutoxy, and the like.

"Alkenyl" includes monovalent olefinically unsaturated hydrocarbyl groups being straight-chained or branched and having at least 1 double bond. Particular alkenyl groups include ethenyl (-CH=CH₂), n-propenyl (-CH₂CH=CH₂), isopropenyl (C (CH₃) =CH₂), and the like. A preferred "alkenyl" group is ethenyl (vinyl).

"Alkynyl" includes acetylenically unsaturated hydrocarbyl groups being straight-chained or branched and having at least 1 triple bond. A preferred alkynyl group is ethynyl (acetylene).

"Alkylamino" includes the group -NR'R", wherein R' is alkyl and R" is selected from hydrogen or alkyl

"Alkylsulfonyl" includes a radical-S(O)₂R where R is an alkyl group as defined herein. Representative examples include, but are not limited to, methanesulfonyl, ethylsulfonyl, propylsulfonyl, butylsulfonyl and the like.

"Alkylthio" includes a radical-S-R where R is an alkyl group as defined herein that may be optionally substituted as defined herein. Representative examples include, but are not limited to, methylthio, ethylthio, propylthio, butylthio, and the like.

"Carboxy" refers to the radical -C(=O)OH.

"Ethenyl" refers to -CH=CHₛ which in the present application is also designated "vinyl".

"Ethynyl" refers to -C≡CH.

"Halo" or "halogen" refers to fluoro, chloro, bromo and iodo. Preferred halo groups are either fluoro or chloro.

"Haloalkyl" includes an "alkyl" group as defined further above which is substituted with one or more haiogens which may be the same, e.g. in trifluoromethyl, pentafluoroethyl, heptafluoropropyl, heptafluoro-isopropyl, or nonafluorobutyl, or which may be different.

"Hydroxy" refers to the radical-OH.

"Nitro" refers to the radical-NO₂.

"(C1-C5)alkoxy(C1-C5)alkyl" includes the group-ROR where R is "alkyl" with up to five carbon atoms. Particular alkoxyalkyl groups include, by way of example, methoxymethyl, methoxyethyl, methoxypropyl, ethoxyethyl, ethoxyethyl, n-propoxymethyl, and the like.

"(C1-C5)alkoxycarbonyl(C1-C5)alkyl" includes the group RO(C=O)R where R is "alkyl" with up to five carbon atoms. Particular alkoxycarbonylalkyl groups include, by way of example, buthoxycarbonylmethyl, ethoxycarbonylmethyl, methoxycarbonylmethyl, and the like.

"(C1-C5)alkoxy(C1-C5)alkylamino" includes the group RORNH- where R is "alkyl" with up to five carbon atoms. Particular alkoxyalkylamino groups include, by way of example, methoxymethylamino, methoxyethylamino, ethoxymethylamino, and the like.

"(C1-C5)alkoxy(C1-C5)alkoxy" includes the group RORO- where R is "alkyl" with up to five carbon atoms. Particular alkoxyalkoxyl groups include, by way of example, methoxymethoxy, methoxyethoxy, methoxypropoxy, ethoxyethoxy, and the like.

"(C1-C5)alkylcarbonyloxy(C1-C3)alkyl" includes the group RC=OOR- where R is "alkyl" with up to five carbon atoms. Particular alkylcarbonyloxyalkyl groups include, by way of example, t-butylcarbonyloxymethyl, ethylcarbonyloxymethyl, isopropylcarbonyloxymethyl, and the like.

"Pharmaceutically acceptable" means approved by a regulatory agency of the Federal or a state government or listed in the U. S. Pharmacopoeia or other generally recognized pharmacopoeia for use in animals, and more particularly in humans.

"Pharmaceutically acceptable salt" refers to a salt of a compound of the invention that is pharmaceutically acceptable and that possesses the desired pharmacological activity of the parent compound. Such salts include: (1) acid addition salts, formed with inorganic acids such as hydrochloric acid, hydrobromic acid, sulfuric acid, nitric acid, phosphoric acid, and the like; or formed with organic acids such as acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid,3- (4-hydroxybenzoyl) benzoic acid, cinnamic acid, mandelic acid, methanesulfonic acid, ethanesulfonic acid, 1,2-ethanedisulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, 4methylbicyclo [2.2.2]-oct-2-ene-1-carboxylic acid, glucoheptonic acid,3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, and the like; or (2) salts formed when an acidic proton present in the parent compound is replaced.

"Pharmaceutically acceptable carrier" refers to a diluent, adjuvant, excipient or carrier with which a compound of the invention is administered.

"Preventing" or "prevention" refers to a reduction in risk of acquiring a disease or disorder (i. e., causing at least one of the clinical symptoms of the disease not to develop in a subject that may be exposed to or predisposed to the disease but does not yet experience or display symptoms of the disease).

"Subject" includes humans. The terms "human," "patient" and "subject" are used interchangeably herein.

"Therapeutically effective amount" means the amount of a compound that, when administered to a subject for treating a disease, is sufficient to effect such treatment for the disease. The"therapeutically effective amount" can vary depending on the compound, the disease and its severity, and the age, weight, etc., of the subject to be treated.

"Treating" or "treatment" of any disease or disorder refers, in one embodiment, to ameliorating the disease or disorder (i. e., arresting or reducing the development of the disease or at least one of the clinical symptoms thereof). In another embodiment "treating" or "treatment" refers to ameliorating at least one physical parameter, which may not be discernible by the subject. In yet another embodiment, "treating" or "treatment" refers to modulating the disease or disorder, either physically, (e. g., stabilization of a discernible symptom), physiologically, (e. g., stabilization of a physical parameter), or both. In yet another embodiment, "treating" or "treatment" refers to delaying the onset of the disease or disorder.

### Best mode for carrying out invention

The present invention is more specifically explained by following examples and expirical examples. However, it should be understood that the extent of the present invention is not limited to the foiiowing examples and expirical examples.

### Example 1: 2-(4-tert-Butylphenoxy)-N-(4-methanesulfonylaminobenzyl)acetamide

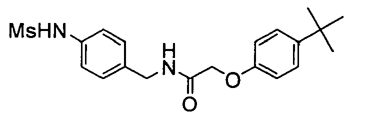

### Step 1: (4-tert-Butylphenoxy)acetic acid tert-butyl ester

4-*tert*-Butylphenol (150mg, 1.00mmol) was added to a suspension of NaH (5eq, 5.00mmol, 200.0mg) in THF and then the resulting mixture was heated at 50~60°C for 1 hr. After cooling, *tert*-butyl bromoacetate (1.20mmol, 177.07µℓ) was added and the reaction mixture was stirred for 2hrs at ambient temperature. After checking the completion of the reaction by TLC, the reaction mixture was quenched by slowly adding water and the mixture was concentrated under reduced pressure to remove THF. The remaining reaction mixture was extracted with methylenechloride, and the organic phase was washed with water and brine, dried over Na₂SO₄, and then concentrated under reduced pressure. The resulting residue was purified by column chromatography (n-Hx: EA= 40: 1) to yield a colorless syrup 255.6 mg (96.75%).
IR (NaCl neat, cm⁻¹): 2926, 1758; ¹H NMR (400MHz, CDCl₃): 7.27 (d, 2H, *J*=8.4Hz), 6.81 (d, 2H, *J*=8.4Hz), 4.46 (s, 2H), 1.47 (s, 9H), 1.27 (s, 9H).

### Step 2: (4-tert-Butylphenoxy)acetic acid

Trifluoroacetic acid (15∼20 drops) was added to a solution of (4-*tert-*butylphenoxy)acetic acid *tert*-butyl ester (153.0mg, 0.58mmol) in methylenechloride and the resulting mixture was stirred for 12hrs at ambient temperature. The reaction mixture was concentrated under reduced pressure, and then toluene was added to the residue. A white solid 188.1mg (100%) was obtained after concentrating the mixture under reduced pressure.
¹H NMR (400MHz, CD₃OD): δ 7.49 (d, 2H, *J* =8.4Hz), 7.28 (d, 2H, *J* =8.4Hz), 4.18 (s, 2H), 1.28(s, 9H).

### Step 3: 2-(4-tert-Butylphenoxy)-N-(4-methanesulfonylaminobenzyl)acetamide

DEPC (1.2eq, 0.13mmol, 20.03µℓ) and TEA (2eq, 0.22mmol, 30.66µℓ) were added to a solution of (4-tert-butyl-phenoxy)acetic acid (1.1eq, 0.12mmol, 38.98mg) and N-(4-aminomethyl-phenyl)-methanesulfonamide (1eq, 0.11mmol, 35mg) in DMF and the resulting mixture was stirred for 12hrs. DMF was concentrated under reduced pressure and the reaction mixture was extracted with Ethyl acetate. The organic phase was washed with H₂O and brine, dried over Na₂SO₄, filtered and then concentrated under reduced pressure. The remaining residue was subjected to column chromatography (n-Hx: EtOAc= 1:1) to give a white solid 34.4mg (80.15%).
Mp: 110~112°C; IR (NaCl neat, cm⁻¹): 3250, 2961, 1649, 1512, 1323, 1146;
¹H NMR (400 MHz, CDCl₃): δ 7.29 (d, 2H, *J* =8.8Hz), 7.21 (d, 2H, *J* =8.8Hz), 7.16 (d, 2H, *J* =8.8Hz), 7.01 (t, 1 H, *J* =5.6Hz), 6.82 (d, 2H, *J* =8.8Hz), 4.52 (s, 2H), 4.48 (d, 2H, *J* =6.0Hz), 2.95 (s, 3H), 1.26 (s, 9H)

### Example 2: 2-(4-tert-Butylphenoxy)-N-(3-fluoro-4-methanesulfonylaminobenzyl)acetamide

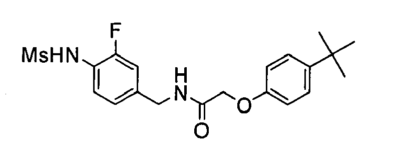

A dried 25ml two-neck round bottom flask was filled with argon and (4-tert-butyl-phenoxy)-acetic acid (1.1eq, 0.09mmol, 30mg) and N-(4-aminomethyl-2-fluorophenyl)-methanesulfonamide (1.0eq, 0.09mmol, 30mg) were dissolved in DMF. To this solution were added DEPC (1.2eq, 0.11mmol, 16.39µℓ) and TEA (2.0eq, 0.18mmol, 25.09µℓ) and stirred for 12 hours. After confirming the completion of the reaction, the solvent was concentrated under reduced pressure, extracted with EtOAc, washed with H₂O and brine, dried over Na₂SO₄, filtered and concentrated under reduced pressure. The obtained liquid was column chromatographed (n-Hexane/EtOAc=1/1) to yield a white solid (27.4 mg, 93.64%).

Mp: 93~95°C; ¹H NMR (400MHz, CDCl₃): δ 7.47 (t, 1H, *J* =8.0Hz), 7.30 (d, 2H, *J* =8.8Hz), 7.04~7.02 (m,1H), 6.83 (d, 2H, *J*=8.8Hz), 6.77 (bs, 1H), 4.52 (s, 2H), 4.48 (d, 2H, *J*=6.0Hz), 2.98 (s, 3H), 1.27 (s, 9H)

### Example 3: 2-(4-tert-Butylphenoxy)-N-(3-chloro-4-methanesulfonylaminobenzyl)-acetamide

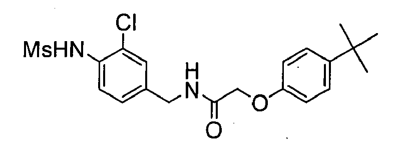

To a solution of (4-*tert*-butylphenoxy)acetic acid (0.144mmol, 30mg) and *N*-(4-aminomethyl-2-chlorophenyl)methanesulfonamide (0.131 mmol, 30.6 mg) in DMF under argon were added DEPC (30µℓ) and TEA (20µℓ). The resulting mixture was stirred for 12 hours, concentrated in vacuo, and diluted with EtOAc. The organic layer was washed with water and brine, dried over anhydrous sodium sulfate, filtered, and concentrated in vacuo. The crude residue was then purified by column chromatography (n-Hx: EtOAc= 1:1) to afford 42.3mg (76%) as a white solid.
Mp: 89~91°C; IR (KBr pellet, cm⁻¹): 3359, 3220, 1667; ¹H NMR (400 MHz, CDCl₃): δ 7.53 (d, 1 H, *J* =8.4Hz), 7.3~7.2 (m, 3H), 7.14 (d, 1 H, *J* =8.4Hz), 7.0∼6.7 (m. 3H), 6.79 (d, 1H, *J* =8.4Hz), 4.48 (s, 2H), 4.44 (d, 2H, *J* =6.4Hz), 2.93 (s, 3H), 1.23 (s, 9H).

### Example 4: 2-(4-tert-Butylphenoxy)-N-(4-methanesulfonylamino-3-vinylbenzyl)acetamide

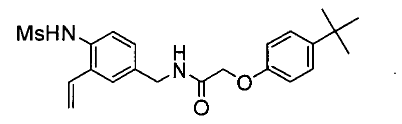

DEPC (1.2eq, 0.16mmol, 23.67µℓ) and TEA (2eq, 0.26mmol, 36.24µℓ) were added to a solution of (4-tert-butyt-phenoxy)-acetic acid (1.1eq, 0.15mmol, 46.97mg) and N-(4-aminomethyl-2-vinyl-phenyl)-methanesulfonamide (1eq, 0.13mmol, 30mg) in DMF and the resulting mixture was stirred for 12hrs. DMF was concentrated under reduced pressure and the reaction mixture was extracted with Ethyl acetate. The organic phase was washed with H₂O and brine, dried over Na₂SO₄, filtered and then concentrated under reduced pressure. The remaining residue was subjected to column chromatography (n-Hx: EtOAc= 1:1) to give a white solid 38.7mg (71.52%).
Mp: 86~88°C; IR (NaCl neat, cm⁻¹): 3286, 2961, 1662, 1512, 1325, 1153; ¹H NMR (400 MHz, CDCl₃): δ 7.37 (s, 1 H), 7.29 (d, 2H, *J*=8.0Hz), 7.17 (dd, 1 H, *J* =8.4, 1.6Hz), 6.96 (t, 1 H, *J* =5.6Hz), 6.88 (dd, 1 H, *J* =17.6, 11.2Hz), 6.82 (d, 2H, *J* =8.0Hz), 6.62 (s, 1 H), 5.67 (d, 1H, *J*=17.6Hz), 5.43 (d, 1H, *J*=11.2Hz), 4.51 (s, 4H), 2.94 (s, 3H), 1.26 (s, 9H)

### Example 5: 2-(4-tert-Butylphenoxy)-N-(3-fluoro-4-methanesulfonylamino-5-vinylbenzyl)acetamide

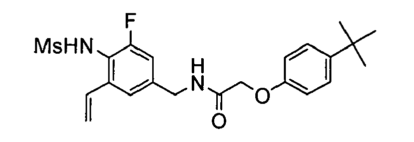

A dried 25ml two-neck round bottom flask was filled with argon and (4-tert-butyl-phenoxy)-acetic acid (1.1eq, 0.11mmol, 34.61mg) and N-(4-aminomethyl-2-fluoro-6-vinylphenyl)methanesulfonamide (1.0eq, 0.10mmol, 35mg) were dissolved in DMF. To this solution were added DEPC (1.2eq, 0.12mmol, 18.21µℓ) and TEA (2.0eq, 0.20mmol, 27.88µℓ) and stirred for 12 hours. After confirming the completion of the reaction, the solvent was concentrated under reduced pressure, extracted with EtOAc, washed with H₂O and brine, dried over Na₂SO₄, filtered and concentrated under reduced pressure. -The- obtained liquid was column chromatographed (n-Hexane/EtOAc=1/1) to yield a white solid (33.7mg, 77.61%).
Mp: 155~157°C; IR (KBr pellet, cm⁻¹): 3286, 3232, 3086, 2962, 1658, 1321, 1155; ¹H NMR (400 MHz, CDCl₃): δ 7.31 (d, 2H, *J*=8.4Hz), 7.12 (dd, 1 H, *J*=17.6, 10.8Hz), 7.01 (t, 1 H, *J* =6.0Hz), 6.97 (dd, 1 H, *J* =10.0, 1.2Hz), 6.84 (d, 2H, *J* =8.4Hz), 6.20 (s, 1 H), 5.73 (d, 1 H, *J* =17.6Hz), 5.41 (d, 1 H, *J* =10.8Hz), 4.53 (s, 2H), 4.50 (d, 2H, *J* =6.0Hz), 3.03 (s, 3H), 1.27 (s, 9H).

### Example 6: 2-(4-tert-Butylphenoxy)-N-(3-ethynyl-5-fluoro-4-methanesulfonylaminobenzyl)acetamide

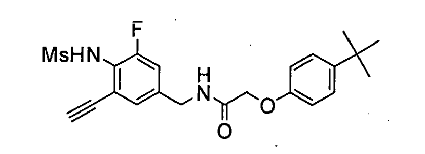

To a solution of (4-*tert*-butylphenoxy)acetic acid (1.1eq, 0.12mmol, 39.78mg) and *N*-(4-aminomethyl-2-ethynyl-6-fluorophenyl)methanesulfonamide (1eq, 0.11mol, 40mg) in DMF under argon were added DEPC (1.2eq, 0.13mmol, 20.03µℓ) and TEA (2eq, 0.22mmol, 30.06µℓ). The resulting mixture was stirred for 12 hours, concentrated in vacuo, and diluted with EtOAc. The organic layer was washed with water and brine, dried over anhydrous sodium sulfate, filtered, and concentrated in vacuo. The crude residue was then purified by column chromatography (n-Hx: EtOAc= 1:1) to afford the title compound (39mg, 77.9%) as a white solid.

Mp: 102~104°C; IR (KBr pellet, cm⁻¹): 3235, 3098, 2961, 1656, 1331, 1148; ¹H NMR (400MHz, CDCl₃): δ 7.30 (d, 2H, *J* =8.8Hz), 7.22 (s, 1 H), 7.06 (dd, 1 H, *J* =10.8, 2.0Hz), 7.01 (s, 1H), 6.83 (d, 2H, *J*=8.8Hz), 6.49 (s, 1H), 4.52 (s, 2H), 4.45 (d, 2H, *J*=6.4Hz), 3.44 (s, 1H), 3.20 (s, 3H), 1.27 (s, 9H)

### Example 7: 2-(4-tert-Butylphenoxy)-N-(3-ethyl-5-fluoro-4-methanesulfonylaminobenzyl)acetamide

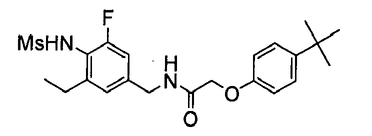

Into a dried 25ml two-neck round bottom flask was put 2-(4-*tert*-butylphenoxy)-*N*-(3-fluoro-4-methanesulfonylamino-5-vinylbenzyl)acetamide (27.9mg, 0.06mmol) and was dissolved in methanol. And this mixture was put into Palladium 10wt.% on activated carbon. After then the flask was substituted with H₂ gas, stirred for 5 hours. After confirming the completion of this reaction, the reaction mixture was filtered with celite, concentrated under reduced pressure and column chromatographed (n-Hx: EtOAc= 1: 1) to yield a white solid (22.3mg, 85.4%).
Mp: 102~104°C; IR (KBr pellet, cm⁻¹): 3235, 3079, 2963, 1671, 1321, 1154 ; ¹H NMR(400MHz, CDCl₃): 7.30 (d, 2H, *J* =8.4Hz), 7.00 (t, 1H, *J* =6.0Hz), 6.97 (s, 1 H), 6.88 (d, 1 H, *J* =10.8Hz), 6.83 (d, 2H, *J* =8.4Hz), 6.17 (s, 1 H), 4.53 (s, 2H), 4.48 (d, 2H, *J* =6.0Hz), 3.04 (s, 3H), 2.81 (q, 2H, *J*=8.0Hz), 1.27 (s, 9H), 1.17 (t, 3H, *J* =7.6Hz),

### Example 8: 2-(4-tert-Butyl-phenoxy)-N-(3-chloro-4-methanesulfonylamino-5-methylbenzyl)acetamide

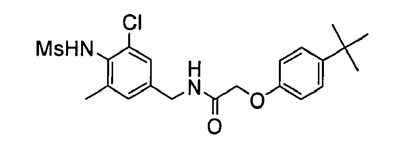

To a solution of (4-*tert*-butylphenoxy)acetic acid (1.1 eq, 0.11mmol, 34.25mg) and *N*-(4-aminomethyl-2-chloro-6-methyl-phenyl)-methanesulfonamide (1eq, 0.10mmol, 35mg) in DMF under argon were added DEPC (1.2eq, 0.12mmol, 27.88µℓ) and TEA (2eq, 0.20mmol, 18.22µℓ). The resulting mixture was stirred for 12 hours, concentrated in vacuo, and diluted with EtOAc. The organic layer was washed with water and brine, dried over anhydrous sodium sulfate, filtered, and concentrated in vacuo. The crude residue was then purified by column chromatography (n-Hx: EtOAc= 1:1) to afford the title compound (42.56mg, 88.3%) as a white solid.
Mp: 105~107°C; IR (KBr pellet, cm⁻¹): 3275, 3039, 2961, 1664, 1320; ¹H NMR (400 MHz, CDCl₃): δ 7.31 (d, 2H, *J*=8.4Hz), 7.18 (s, 1H), 7.07 (s, 1H), 6.95 (t, 1H, *J*=5.6Hz), 6.83 (d, 2H, *J* =8.4Hz), 6.13 (s, 1 H), 4.53 (s, 2H), 4.45 (d, 2H, *J* =6.4Hz), 3.05 (s, 3H), 2.44 (s, 3H), 1.27 (s, 9H)

### Example 9: 2-(4-tert-Butylphenoxy)-N-(5-fluoro-4-methanesulfonylamino-2-methylbenzyl)acetamide

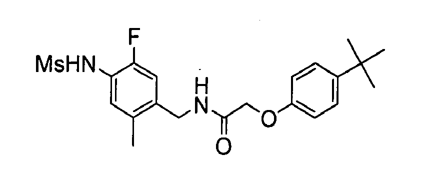

To a solution of (4-*tert*-butylphenoxy)acetic acid (1.1eq, 0.13mmol, 41.98mg) and *N*-(4-aminomethyl-2-fluoro-5-methyl-phenyl)-methanesulfonamide (1eq, 0.12mmol, 41 mg) in DMF under argon were added DEPC (1.2eq, 0.14mmol, 21.85µℓ) and TEA (2eq, 0.24mmol, 33.45µℓ). The resulting mixture was stirred for 12 hours, concentrated in vacuo, and diluted with EtOAc. The organic layer was washed with water and brine, dried over anhydrous sodium sulfate, filtered, and concentrated in vacuo. The crude residue was then purified by column chromatography (n-Hx: EtOAc= 1:1) to afford the title compound (45.61 mg, 83.1 %) as a white solid.

Mp: 84~86°C; IR (KBr pellet, cm⁻¹): 3258, 3022, 2962, 1668, 1511, 1331; ¹H NMR (400 MHz, CDCl₃): δ 7.30 (d, 2H, *J* =8.4Hz), 7.29 (s, 1H), 6.94 (d, 1H, *J* =11.2Hz), 6.83 (d, 2H, *J* =8.4Hz), 6.65 (s, 1H), 4.53 (s, 2H), 2.97 (s, 3H), 2.23 (s, 2H), 1.26 (s, 9H)

### Example 10: 2-(4-tert-Butylphenoxy)-N-(3-fluoro-4-methanesulfonylamino-5-methylbenzyl) acetamide

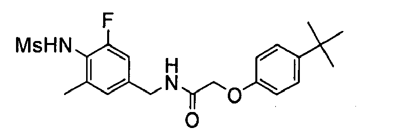

A dried 25ml two-neck round bottom flask was filled with argon, and (4-tert-butylphenoxy)-acetic acid (1.1eq, 0.19mmol, 61.07mg) and N-(4-aminomethyl-2-fluoro-6-methylphenyl)methanesulfonamide (1.0eq, 0.17mmol, 40mg) were dissolved in DMF. To this solution were added DEPC (1.2eq, 0.20mmol, 30.95µℓ) and TEA (2.0eq, 0.34mmol, 47.39µℓ) and stirred for 12 hours. After confirming the completion of the reaction, the solvent was concentrated under reduced pressure, extracted with EtOAc, washed with H₂O and brine, dried over Na₂SO₄, filtered and concentrated under reduced pressure. The obtained liquid was column chromatographed (n-Hexane/EtOAc=1/1) to yield a white solid (68.50mg, 85.4%).
Mp: 85~87°C; IR (KBr pellet, cm⁻¹): 3274, 3019, 2963, 1666, 1512, 1326, 1186; ¹H NMR (400 MHz, CDCl₃): δ 7.29 (d, 2H,*J*=8.8Hz), 7.16~7.09 (m, 1H), 7.14 (s, 1H), 7.01 (t, 1H, *J* =6.0Hz), 6.88 (bs, 1 H), 6.82 (d, 2H, *J* =8.8Hz), 4.52 (d, 2H, *J* =6.0Hz), 4.48 (s, 2H), 2.98 (s, 3H), 2.22 (s, 3H), 1.26 (s, 9H)

### Exam ple 11: (R)-2-(4-tert-Butylphenoxy)-N-[1-(4-methanesulfonylaminophenyl)ethyl]acetamide

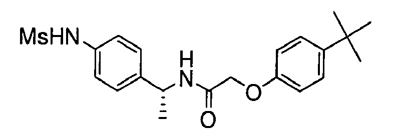

A dried 25ml two-neck round bottom flask was filled with argon, and (4-tert-butyl-phenoxy)-acetic acid (1.1eq, 0.10mmol, 32.40mg) and (R)-N-[4-(1-amino-ethyl)-phenyl]-methanesulfonamide (1.0eq, 0.09mmol, 30mg) were dissolved in DMF. To this solution were added DEPC (1.2eq, 0.11mmol, 16.39µℓ) and TEA (2.0eq, 0.18mmol, 25.08µℓ), and stirred for 12 hours. After confirming the completion of the reaction, the solvent was concentrated under reduced pressure, extracted with EtOAc, washed with H₂O and brine, dried over Na₂SO₄, filtered and concentrated under reduced pressure. The obtained liquid was column chromatographed (n-Hexane/EtOAc=1/1) to yield a white solid (29.42mg, 80.9%)
Mp: 86~88°C; [α]_{D}²⁰ -4.36 (CHCl₃, *c* 0.11); IR (KBr pellet, cm⁻¹): 3262, 3056, 2963, 1660, 1512, 1329, 1154; ¹H NMR (400MHz, CDCl₃): δ 7.34 (s, 1H), 7.30 (d, 2H, *J* =8.8Hz), 7.22 (d, 2H, *J* =8.0), 7.13 (d, 2H, *J* =8.0), 6.83 (d, 2H, *J* =7.2Hz), 4.47 (s, 2H), 2.93 (s, 3H), 1.48 (d, 3H, *J* =6.8Hz), 1.27 (s, 9H)

### Example 12: (R)-2-(4-tert-Butylphenoxy)-N-[1-(3-fluoro-4-methanesuffonylaminophenyl)ethyl]acetamide

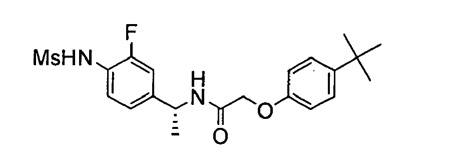

To a solution of (4-*tert*-butylphenoxy)acetic acid (1.1eq, 0.16mmol, 53.44mg) and (R)-N-[4-(1-aminoethyl)-2-fluoro-phenyl]methanesulfonamide (1eq, 0.15mmol, 35mg) in DMF under argon were added DEPC (1.2eq, 0.18mmol, 27.31µℓ) and TEA(2eq, 0.30mmol, 41.81µℓ). The resulting mixture was stirred for 12 hours, concentrated in vacuo, and diluted with EtOAc. The organic layer was washed with water and brine, dried over anhydrous sodium sulfate, filtered, and concentrated in vacuo. The crude residue was then purified by column chromatography (n-Hx: EtOAc= 1:1) to afford the title compound (52.07mg, 77.1%) as a white solid.
Mp: 130~132°C; [α]_{D}²⁰-14.0 (CHCl₃, c 0.52); IR (NaCl neat, cm⁻¹): 3277, 3034, 2967, 1671, 1166; ¹H NMR (400MHz, CDCl₃): δ 7.46 (t, 1H, *J*=8.0Hz), 7.30 (d, 2H, *J* =8.8Hz), 7.06~7.01 (m, 2H), 6.83 (d, 2H, *J* =8.8Hz), 6.78 (s, 1 H), 5.14 (q, 1H, *J*=7.2Hz), 4.47 (s, 2H), 2.98 (s, 3H), 1.48 (d, 3H, *J* =6.8Hz), 1.27 (s, 9H)

### Example 13: (R)-2-(4-tert-Butylphenoxy)-N-(3-fluoro-4-methanesulfonylaminobenzyl)propionamide

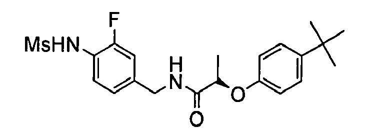

### Step 1: (R)-2-(4-tert-Butylphenoxy)propionic acid ethyl ester

4-tert-Butyl-phenol (1.2eq, 1.02mmol, 168.06mg) and (S)-lactic acid acid ethyl ester (1eq, 0.85mmol, 100mg) were dissolved in Toluene and the mixture was cooled to 0°C. PPh₃ (1.2eq, 1.02mmol, 267.53mg) and DEAD (1.2eq, 1.02mmol, 185.82µℓ) were added to the reaction mixture and then stirred for 12hrs. The liquid obtained after concentrating Toluene under reduced pressure was subjected to column chromatography (n-Hx: EtOAc= 25:1) to yield a colorless syrup 138.43mg (65.1%).
[α]_{D}²⁰ +30.89 (CHCl₃, c 0.69); IR (NaCl neat, cm⁻¹): 2963, 1755, 1734, 1608, 1346, 1135; ¹H NMR (400MHz, CDCl₃): δ 7.26 (d, 2H, *J* =8.4Hz), 6.79 (d, 2H, *J* =8.4Hz), 4.69 (q, 1H, *J* =6.8Hz), 4.20 (q, 1 H, *J* =6.8Hz), 1.58 (d, 3H, *J* =7.2Hz), 1.27 (s, 9H), 1.24 (d, 3H, *J* =7.2Hz)

### Step 2: (R)-2-(4-tert-Butylphenoxy)propionic acid

Small amount of water was added to a solution of (R)-2-(4-tert-butyl-phenoxy)-propionic acid ethyl ester (1eq, 0.23mmol, 56.6mg) in MeOH followed by MeOH. After cooling to 0°C, NaOH (5eq, 1.13mmol, 45.25 mg) was slowly added, and the reaction mixture was stirred for 5hrs. After completion of the reaction, the aqueous phase was washed with methylenechloride, acidified, and then extracted with Ether. The ethereal phase was concentrated to give a pale yellow syrup 35.8mg (70.26%).
¹H NMR (400MHz, CD₃OD) δ 7.25 (d, 2H, *J* =8.8Hz), 6.77 (d, 2H, *J* =8.8Hz), 4.72 (q, 1H, *J* =7.2Hz), 1.53 (d, 3H, *J*=7.2Hz), 1.25 (s, 9H)

### Step 3: (R)-2-(4-tert-Butylphenoxy)-N-(3-fluoro-4-methanesulfonylaminobenzyl)propionamide

To a solution of (R)-2-(4-tert-butyl-phenoxy)propionic acid (1.1eq, 0.10mmol, 22.08mg) and N-(4-aminomethyl-2-fluoro-phenyl)methanesulfonamide (1 eq, 0.09mmol, 30mg) in DMF under argon were added DEPC (1.2eq, 0.11mmol, 16.39µℓ) and TEA(2eq, 0.18mmol, 25.09µℓ). The resulting mixture was stirred for 12 hours, concentrated in vacuo, and diluted with EtOAc. The organic layer was washed with water and brine, dried over anhydrous sodium sulfate, filtered, and concentrated in vacuo. The crude residue was then purified by column chromatography (n-Hx: EtOAc= 1:1) to afford the title compound (32.2mg, 84.74%) as a white solid.
Mp: 69-71 °C; [α]_{D}²⁰ +15.29 (CHCl₃, *c* 0.51); IR (KBr pellet, cm⁻¹): 3264, 2963, 1664, 1510, 1335, 1159; ¹H NMR (400 MHz, CDCl₃): δ 7.42 (t, 1 H, *J*=8.0Hz), 7.29 (d, 2H, *J* =8.8Hz), 6.93~6.89 (m, 2H), 6.87 (bs, 1 H), 6.81 (d, 2H, *J* =8.8Hz), 6.73 (bs, 1 H), 4.71 (q, 1H, *J* =6.8Hz), 4.46∼4.35 (m, 2H), 2.96 (s, 3H), 1.57 (d, 3H, *J* =6.8Hz), 1.26 (s, 9H)

### Example 14: (S)-2-(4-tert-Butylphenoxy)-N-(3-fluoro-4-methanesulfonylaminobenzyl)propionamide

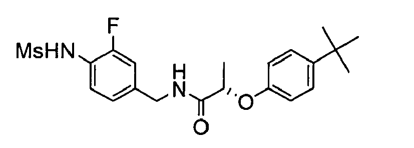

### Step 1: (S)-2-(4-tert-Butylphenoxy)propionic acid ethyl ester

4-tert-Butyl-phenol (1.2eq, 0.71mmol, 106.82mg) and (R)-2-hydroxypropionic acid ethyl ester (1eq, 0.59mmol, 70mg) were dissolved in Toluene and the mixture was cooled to 0°C. PPh₃ (1.2eq, 0.71mmol, 185.70mg) and DEAD (1.2eq, 0.71mmol, 129.34µℓ) were added to the reaction mixture and then stirred for 12hrs. The liquid obtained after concentrating Toluene under reduced pressure was subjected to column chromatography (n-Hx: EtOAc= 25:1) to yield a colorless syrup 89.5mg (60.64%).
[α]_{D}²⁰ -30.51 (CHCl₃, *c* 0.59); IR (NaCl neat, cm⁻¹): 2963, 1755, 1734, 1608, 1346, 1135 ¹H NMR (400MHz, CDCl₃): δ 7.25 (d, 2H, *J* =8.8Hz), 6.79 (d, 2H, *J* =8.8Hz), 4.69 (q, 1 H, *J* =6.8Hz), 4.19 (q, 1 H, *J* =7.2Hz), 1.58 (d, 3H, *J* =6.8Hz), 1.26 (s, 9H), 1.23 (d, 3H, *J* =7.2Hz)

### Step 2: (S)-2-(4-tert-Butylphenoxy)propionic acid

Small amount of water was added to a solution of (S)-2-(4-tert-butylphenoxy)propionic acid ethyl ester (1eq, 0.36mmol, 89.5mg) in MeOH followed by MeOH. After cooling to 0°C, NaOH (5eq, 1.79 mmol, 71.55 mg) was slowly added, and the reaction mixture was stirred for 5hrs. After completion of the reaction, the aqueous phase was washed with Methylenechloride, acidified, and then extracted with Ether (150 × 3). The ethereal phase was concentrated to give a pale yellow syrup 70.05 mg (87.6%).
¹H NMR (400MHz, CD₃OD): δ 7.25 (d, 2H, *J* =8.8Hz), 6.77 (d, 2H, *J* =8.8Hz), 4.71 (q, 1 H, *J* =6.8Hz), 1.53 (d, 3H, *J* =6.8Hz), 1.24 (s, 9H)

### Step 3: (S)-2-(4-tert-butylphenoxy)-N-(3-fluoro-4-methanesulfonylaminobenzyl)propionamide

To a solution of (S)-2-(4-tert-Butyl-phenoxy)propionic acid (1.1eq, 0.12mmol, 25.75mg) and N-(4-aminomethyl-2-fluoro-phenyl)methanesulfonamide (1 eq, 0.11mmol, 35mg) in DMF under argon were added DEPC (1.2eq, 0.13mmol, 20.03µℓ) and TEA (2eq, 0.22mmol, 30.66µℓ). The resulting mixture was stirred for 12 hours, concentrated in vacuo, and diluted with EtOAc. The organic layer was washed with water and brine, dried over anhydrous sodium sulfate, filtered, and concentrated in vacuo. The crude residue was then purified by column chromatography (n-Hx: EtOAc= 1:1) to afford the title compound (41.98mg, 90.4%) as a white solid.
Mp: 55~57 °C; [α]_{D}²⁰-10.93 (CHCl₃, c 0.28); IR (NaCl neat, cm⁻¹): 3263, 3025, 2963, 1663, 1510, 1335, 1159; ¹H NMR (400MHz, CDCl₃): δ 7.43 (t, 1 H, *J*=8.0Hz), 7.29 (d, 2H, *J* =8.4Hz), 6.94∼6.90 (m, 2H), 6.86~6.79 (m, 1 H), 6.81 (d, 2H, *J* =8.4Hz), 6.60 (bs, 1 H), 4.71 (q, 1 H, *J* =6.8Hz), 4.47∼4.35 (m, 2H), 2.97 (s, 3H), 1.57 (d, 3H, *J* =6.8Hz), 1.27 (s, 9H)

### Example 15: (R)-2-(4-tert-Butylphenoxy)-N-[1-(4-methanesulfonylaminophenyl)ethyl]thioacetamide

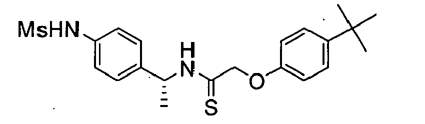

(R)-2-(4-tert-butyl-phenoxy)-N-[1-(4-methanesulfonylaminophenyl)ethyl]acetamide (Ex 11, 1eq, 0.10mmol, 41.4mg) was dissolved in toluene under argon atmosphere. Lawesson's reagent (2eq, 0.20mmol, 82.86mg) was added into the mixture. The reaction mixture was stirred for 12 hrs in reflux. The reaction solvent was removed in vacuo. The residue was purified with column chromatography (n-Hx: EtOAc= 3:1) to yield the title compound (37.1 mg, 88.30%) as a yellow solid.
Mp: 63~65°C; [α]_{D}²⁰ +90.43(CHCl₃, c 0.28); IR (KBr pellet, cm⁻¹): 3281, 2963, 1610, 1512, 1325, 1155; ¹H NMR (400MHz, CDCl₃): δ 8.40 (d, 1H, *J*=8.0Hz), 7.29 (d, 2H, *J* =8.4Hz), 7.23 (d, 2H, *J* =8.4Hz), 7.19 (d, 2H, *J* =8.4Hz), 7.04 (bs, 1 H), 6.81 (d, 2H, *J* =8.8Hz), 5.77 (q, 1H, *J*=7.2Hz), 4.83 (s, 2H), 2.97 (s, 3H), 1.56 (d, 3H, *J*=7.2Hz), 1.26 (s, 9H)

### Example 16: 2-(4-tert-Butylphenoxy)-N-(3-fluoro-4-methanesulfonylaminobenzyl)thioacetamide

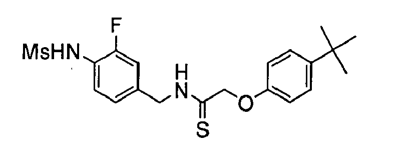

2-(4-*tert*-Butylphenoxy)-*N*-(3-fluoro-4-methanesulfonylaminobenzyl)acetamide (0.10 mmol, 44.89 mg) was dissolved in toluene. Lawesson's reagent (0.20 mmol, 82.86 mg) was added into the mixture. The reaction mixture was stirred for 12 hrs in reflux. The solvent was eliminated under reduced pressure. The residue was purified with column chromatography (n-Hx: EtOAc= 3:1) to yield the title compound (34.9 mg, 82.28%) as a yellow syrup.
IR (NaCl neat, cm⁻¹): 3291, 3037, 2961, 1511, 1331, 1159: ¹H NMR (400MHz, CDCl₃): δ 7.47 (t, 1 H, *J* =8.0Hz), 7.28 (d, 2H, *J* =8.8Hz), 7.06 (d, 1H, *J* =3.2Hz), 7.03 (s, 1 H), 6.82 (d, 2H, *J* =8.8Hz), 6.75 (bs, 1H), 4.91 (d, 2H, *J* =6.0Hz), 4.89 (s, 2H), 2.98 (s, 3H), 1.25 (s, 9H)

### Example 17: (R)-2-(4-tert-Butylphenoxy)-N-[1-(4-methanesulfonylaminophenyl)ethyl]-N'-cyanoacetamidine

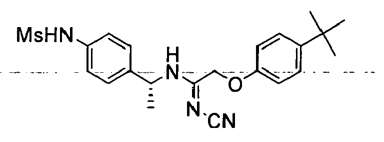

(R)-2-(4-tert-Butyl-phenoxy)-N-[1-(4-methanesulfonylamino-phenyl)-ethyl]thioacetamide (0.08 mmol, 35.0 mg) was dissolved in DMF under argon atmosphere. Cyanamide (0.08 mmol, 3.5 mg), HgCl₂ (0.08 mmol, 21.72 mg), and TEA (0.08 mmol, 11.15 µℓ) were added into the mixture. The reaction mixture was stirred for 12hrs. The reaction mixture was extracted with ethyl acetate. A combined organic layer was washed with H₂O and brine, dried with Na₂SO₄, and concentrated in vacuo. The residue was purified with column chromatography (n-Hx: EtOAc= 1:1) to yield the title compound (33.7 mg, 98.37%) as a white solid.

Mp: > 200°C; [α]_{D}²⁰+27.2(CHYCl₃, *c* 0.1); IR (KBr pellet, cm⁻¹): 3325, 3183, 2952, 2177, 1605, 1334, 1154; ¹H NMR (400MHz, CDCl₃): δ 8.40 (d, 1H, *J* =8.0Hz), 7.29 (d, 2H, *J* =8.4Hz), 7.23 (d, 2H, *J* =8.4Hz), 7.19 (d, 2H, *J* =8.4Hz), 7.04 (bs, 1 H), 6.81 (d, 2H, *J* =8.8Hz), 5.77 (q, 1H, *J* =7.2Hz), 4.83 (s, 2H), 2.97 (s, 3H), 1.56 (d, 3H, *J* =7.2Hz), 1.26 (s, 9H)

### Example 18: 2-(4-tert-Butylphenoxy)-N-(3-fluoro-4-methanesulfonylaminobenzyl)-N'-cyanoacetamidine

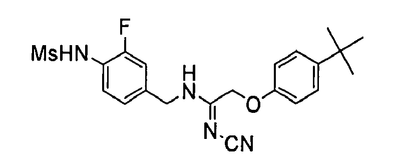

2-(4-*tert*-Butyl-phenoxy)-*N*-(3-fluoro-4-methanesulfonylaminobenzyl)-thioacetamide (0.08 mmol, 34.9 mg) was dissolved in DMF. Cyanamide (0.08 mmol, 3.5mg), HgCl₂(0.08 mmol, 21.72 mg), and TEA (0.08 mmol, 11.15 µℓ) were added into the mixture. The reaction mixture was stirred for 12 hrs in reflux. The reaction mixture was extracted with ethyl acetate. A combined organic layer was washed with H₂O and brine, dried with Na₂SO₄, and concentrated in vacuo. The residue was purified with column chromatography (n-Hx: EtOAc= 1:1) to yield the title compound (33.12 mg, 95.8%) as a white solid.
TLC : R*_{f}*= 0.33 (n-Hx: EtOAc= 1:1/ KMnO₄); Mp: 96~98°C; IR (KBr pellet, cm⁻¹): 3259, 3142, 2965, 2927, 2179, 1610, 1330, 1164; ¹H NMR (400MHz, CDCl₃): δ 7.52 (t, 1H, *J* =8.0Hz), 7.32 (d, 2H, *J* =8.8Hz), 7.08∼7.05 (m, 3H), 6.82 (d, 2H, *J* =8.8Hz), 6.56 (bs, 1 H), 4.97 (s, 2H), 4.55 (d, 2H, *J*=6.0Hz), 3.01 (s, 3H), 1.27 (s, 9H)

### Example 19: 2-(4-tert-Butyl-phenylsulfanyl)-N-(3-fluoro-4-methanesulfonylaminobenzyl)-acetamide

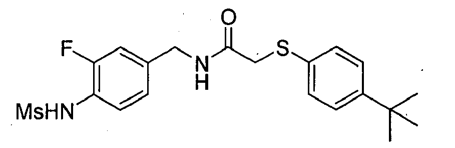

### Step 1: (4-tert-Butyl-phenylsulfanyl)-acetic acid

4-tert-Butylthiophenol (1.0eq, 1.0g, 6.01 mmol) was put into the 100 ml one-neck round bottom flask and was filled in 30ml of EtOH. To the solution was poured K₂CO₃ (1.5eq, 1.25g) slowly and stirred for 10 minutes at room temperature. After then 4-tert-butylbromoacetate (1.0eq, 1.07ml) was added in this mixture. The reaction mixture was stirred for 6 hours. After confirming the completion of the reaction with TLC, the reaction solution was concentrated in vacuo, extracted with EtOAc, washed with water and brine, dried over MgSO₄, filtered and concentrated under reduced pressure. The obtained liquid was column chromatographed (n-hexane/EtOAc=8/1). The obtained liquid was dissolved in methylenechloride; added trifluoroacetic acid 3ml and stirred for 24 hours. After confirming the completion of the reaction with TLC, the reaction solution was extracted with methylene, washed with 1 N HCl and brine, dried over MgSO₄, filtered and concentrated under reduced pressure to yield a liquid (1.3g, 73.0%).
¹H NMR (300MHZ, DMSO-d₆): δ 1.30 (s, 9H), 3.83 (s, 2H), 7.45 (m, 4H)

### Step 2: 2-(4-tert-Butyl-phenylsulfanyl)-N-(3-fluoro-4-methanesulfonylaminobenzyl)-acetamide

(4-tert-Butyl-phenylsulfanyl)-acetic acid (0.28g, 0.95mmol) and DMTMM (1.1eq, 288mg) were put into the 50 ml round bottom flask and was filled in 20ml THF and stirred for 10 minutes. After then to this reaction mixture were added N-(4-aminomethyl-2-fluoro-phenyl)methanesulfonamide hydrochloride (1.0eq, 0.24g) and TEA (excess, 0.5ml) and stirred for 18 hours. After confirming the completion of the reaction with TLC, the reaction mixture was extracted with ethylacetate, washed with 1 N HCl solution and brine, dried over MgSO4, filtered and concentrated under reduced pressure. The obtained liquid was column chromatographed (n-hexane/EtOAc=3/2) to yield a solid (195mg, 48.3%).
Mp: 133-134 °C; ¹H NMR (300MHZ, CDCl₃): δ 1.31 (s, 9H), 3.00 (s, 3H), 3.69 (s, 2H), 4.42 (d, 2H, *J*=6.3Hz), 6.41 (bs, 1 H), 6.89 (m, 2H), 7.21 (m, 2H), 7.34 (m, 2H), 7.45 (t, 1 H, J=8.7Hz)

### Example 20: 2-(4-tert-Butyl-phenylsulfanyl)-N-(3-ethynyl-5-fluoro-4-methanesulfonylamino-benzyl)-acetamide

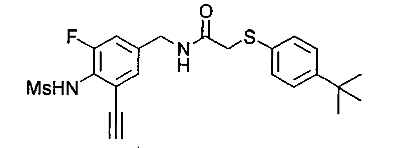

(4-tert-Butyl-phenylsulfanyl)-acetic acid (0.28g, 0.95mmol) and DMTMM (1.2eq, 129mg) were put into the 25 ml round bottom flask and was filled in 20ml THF and stirred for 10 minutes. After then to this reaction mixture were added N-(4-aminomethyl-2-ethynyl-6-fluoro-phenyl)methanesulfonamide hydrochloride (1.0eq, 0.108g) and TEA (excess, 0.3ml) and stirred for 18 hours. After confirming the completion of the reaction with TLC, the reaction mixture was extracted with ethylacetate, washed with 1 N HCl solution and brine, dried over MgS0₄, filtered and concentrated under reduced pressure. The obtained liquid was column chromatographed (n-hexane/EtOAc=1/1) to yield a solid (136mg, 77.7%).
Mp: 139~140 °C; ¹H NMR (300MHZ, CDCl₃): δ 1.30 (s, 9H), 3.25 (s, 3H), 3.47 (s, 1H), 3.68 (s, 2H), 4.40 (d, 2H, J=6Hz), 6.36 (bs, 1 H), 6.92 (m, 1 H), 7.21 (m, 2H), 7.25 (m, 1 H), 7.34 (m, 2H)

### Example 21: N-(3-Chloro-4-methanesulfonylamino-benzyl)-2-methyl-2-(4-trifluoromethyl-phenoxy)-propionamide

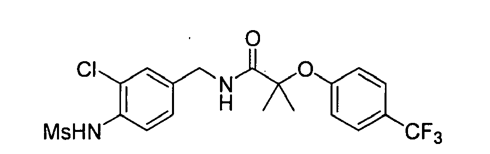

### Step 1: 2-Methyl-2-(4-trifluoromethyl-phenoxy)-propionic acid

4-Trifluoromethylphenol (1.2eq, 0.582g) was put into the 100 ml one-neck round bottom flask and was filled in 30ml THF. To the solution was poured 60% NaH (2.5eq, 0.3g) slowly at 0°C and stirred for 30 minutes at room temperature. After then 2-bromo-2-methyl propionic acid (0.5g, 2.99mmol) was added in this mixture. The reaction mixture was stirred for 3 hrs. After confirming the completion of the reaction with TLC, the reaction solution was extracted with EtOAc, washed with water and brine, dried over MgSO₄, filtered and concentrated under reduced pressure. Without any purification the obtained liquid was proceeded to next step.

### Step 2: N-(3-Chloro-4-methanesulfonylamino-benzyl)-2-methyl-2-(4-trifluoromethyl-phenoxy)-propionamide

2-Methyl-2-(4-trifluoromthyl-phenoxy)-propionic acid (0.1 g, 0.4mmol) and DMTMM (1.2eq, 134mg) were put into the 25 ml round bottom flask and was filled in 15ml THF and stirred for 10 minutes. After then to this reaction mixture were added 3-fluoro-4-methanesulfonylbenzylamine hydrochloride (1.0eq, 0.12g) and TEA (excess, 0.3ml) and stirred for 18 hours. After confirming the completion of the reaction with TLC, the reaction mixture was extracted with ethylacetate, washed with 1 N HCl solution and brine, dried over MgSO₄, filtered and concentrated under reduced pressure. The obtained liquid was column chromatographed (n-hexane/EtOAc=1/1) to yield a white solid (88mg, 47.3%)
Mp: 122~123 °C; ¹H NMR (300 MHZ, CDCl₃): δ 1.99 (s, 6H), 3.01 (s, 3H), 4.42 (d, 2H, J=6Hz), 6.76 (bs, 1 H), 6.92 (m, 1 H), 7.08 (bs, 1 H), 7.24 (m, 2H), 7.36 (bs, 1 H), 7.57 (m, 2H)

### Example 22: 2-(2,4-Dichloro-phenoxy)-N-(3-fluoro-4-methanesulfonylaminobenzyl)-acetamide

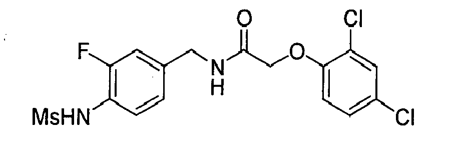

### Step 1: (2,4-Dichloro-phenoxy)-acetic acid

2,4-Dichlorophenol (1.0eq, 0.5g, 3.07mmol) was put into the 100 ml one-neck round bottom flask and was filled in 30ml acetonitrile. To the solution was poured Cs₂CO₃ (1.5eq, 1.45g) slowly and stirred for 10 minutes at room temperature. After then tert-butylbromoacetate (1.0eq, 0.43ml) was added in this mixture. The reaction mixture was stirred for 3 hrs. After confirming the completion of the reaction with TLC, the reaction solution was extracted with EtOAc, washed with water and brine, dried over MgSO₄, filtered and concentrated under reduced pressure. The obtained solid was column chromatographed (n-hexane/EtOAc=4/1). The obtained solid was dissolved in methylenechloride, added trifluoroacetic acid 3ml and stirred for 24 hours. After confirming the completion of the reaction with TLC, the reaction solution was extracted with methylene, washed with 1 N HCl and brine, dried over MgSO₄, filtered and concentrated under reduced pressure to yield a solid (0.67g, 95.8%).
Mp: 138~140 °C; ¹H NMR (300MHZ, DMSO-d₆): δ 4.83 (s, 2H), 7.06 (m, 1 H), 7.35 (m, 1H), 7.58 (s, 1 H)

### Step 2: 2-(2,4-Dichloro-phenoxy)-N-(3-fluoro-4-meyhanesulfonylamino-benzyl)-acetamide

(2,4-Dichloro-phenoxy)-acetic acid (155mg, 0.70mmol) and DMTMM (1.2eq, 233mg) were put into the 25 ml round bottom flask and was filled in 15ml THF and stirred for 10 minutes. After then to this reaction mixture were added 3-fluoro-4-methanesulfonylbenzylamine hydrochloride (1.0eq, 179mg) and TEA (excess, 0.3ml) and stirred for 18 hours. After confirming the completion of the reaction with TLC, the reaction mixture was extracted with ethylacetate, washed with 1 N HCl solution and brine, dried over MgSO₄, filtered and concentrated under reduced pressure. The obtained liquid was column chromatographed (n-hexane/EtOAc=1/1) to yield a white solid (225mg, 76.3%)
Mp: 149∼150 °C; ¹H NMR (300MHZ, CDCl₃) : δ 3.02 (s, 3H), 4.54 (d, 2H, J=6Hz), 4.58 (s, 2H), 6.49 (s, 1 H), 6.85 (d, 1H, *J*=8.7Hz), 7.10 (s, 1 H), 7.13 (bs, 1 H), 7.22 (m, 1 H), 7.41 (d, 1H, *J*=2.7Hz), 7.55 (t, 1H, *J*=8.1 Hz)

### Example 23: N-(3-Fluoro-4-methanesulfonylamino-benzyl)-2-methyl-2-(4-trifluoromethyl-phenoxy)-propionamide

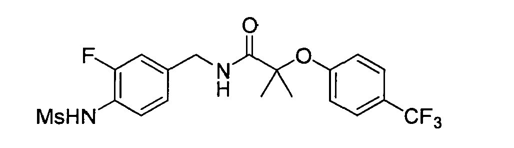

2-Methyl-2-(4-trifluoromethyl-phenoxy)-propionic acid (0.4g, 1.61 mmol) and DMTMM (1.0eq, 446mg) were put into the 25 ml round bottom flask and were filled in 15ml THF and stirred for 10 minutes. After then to this reaction mixture were added 3-fluoro-4-methanesulfonylbenzylamine hydrochloride (1.0eq, 0.41g) and TEA (excess, 1.0ml) and stirred for 18 hours. After confirming the completion of the reaction with TLC, the reaction mixture was extracted with ethylacetate, washed with 1 N HCl solution and brine, dried over MgSO₄, filtered and concentrated under reduced pressure. The obtained liquid was column chromatographed (n-hexane/EtOAc=5/4) to yield a liquid (375mg, 51.9%).
¹H NMR (300 MHZ, CDCl₃): δ 1.99 (s, 6H), 3.03 (s, 3H), 4.44 (d, 2H, *J*=5.7Hz), 6.52 (bs, 1 H), 6.95 (m, 1H), 7.10 (m, 2H), 7.52 (m, 1H), 7.56 (m, 2H)

### Example 24: N-(3-Fluoro-4-methanesulfonylamino-benzyl)-2-(4-trifluoromethylphenoxy)-acetamide

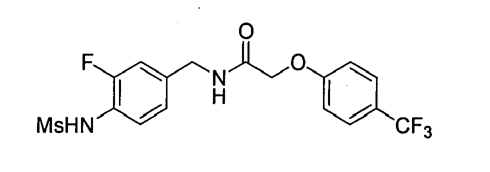

### Step 1: (4-Trifluoromethyl-phenoxy)-acetic acid

To a suspension of α,α,α-trifluoro-p-cresol (500 mg, 3.08 mmol) and ethyl bromoacetate (0.373 ml, 3.38 mmol) in CH₃CN (10 ml) was added Cs₂CO₃ (1.5 g, 4.62 mmol). The mixture was stirred overnight at room temperature. The reaction mixture was diluted with EtOAc (20 ml) and then washed three times with H₂O (20 ml) and brine, dried over MgSO₄, filtered and concentrated under reduced pressure. To a suspension of the crude residue in THF (10 ml) was added a solution of 0.5 N-LiOH (12.32 ml), and the mixture was stirred for 1.5 hrs at room temperature. The resulting residue was dissolved in H₂O (10 ml) and then washed three times with EtOAc (10 ml), acidified with 1 N HCl to pH 1~2. The solution was extracted three times with methylenechloride and then dried over anhyd. Na₂SO₄ and concentrated in vacuo to give (4-Trifluoromethyl-phenoxy)-acetic acid (475 mg, 70%).
¹H NMR (300 MHz, CDCl₃): δ 9.45 (s, 1 H, br), 7.58 (d, 2H, *J* = 9.0 Hz), 6.99 (d, 2H, *J* = 9.0Hz),4.75(s,2H)

### Step 2: N-(3-Fluoro-4-methanesulfonylamino-benzyl)-2-(4-trifluoromethylphenoxy)-acetamide

To a suspension of 3-fluoro-3-methanesulfonylamino-benzylamine HCl salt (233 mg, 0.914 mmol) in THF (4 ml) at 5 °C was added TEA (0.153 ml, 1.101 mmol). The mixture was stirred for 5 minutes at the same temperature, to which were added (4-trifluoromethyl-phenoxy)-acetic acid ethyl ester (200 mg, 0.918 mmol) and DMTMM (279 mg, 1.01 mmol). The mixture was stirred overnight at room temperature and concentrated in vacuo. The residue was diluted with EtOAc (20 ml) and water (20 ml). The organic layer was washed with saturated sodium bicarbonate, 1 N HCl, and brine, dried over anhydrous magnesium sulfate, and concentrated in vacuo to give a solid. The solid was purified by recystallization from EtOAc/hexane to afford the title compound (200 mg, 60 %).
Mp: 126 ~ 132°C; ¹H NMR (300 MHz, CDCl₃): δ 7.55 (d, 2H, *J =* 9.0 Hz), 7.45 (t, 1 H, *J* = 8.1Hz), 7.04 (d, 2H, *J* = 9.0 Hz), 6.96 (d, 2H, *J* = 8.7 Hz), 6.88 (s, 1 H, br), 6.58 (s, 1H), 4.56 (s, 2H), 4.48 (d, 2H, *J =* 6.3 Hz), 2.97 (s, 3H)

### Example 25: 2-(3,4-Dichloro-phenoxy)-N-(3-fluoro-4-methanesulfonylaminobenzyl)-acetamide

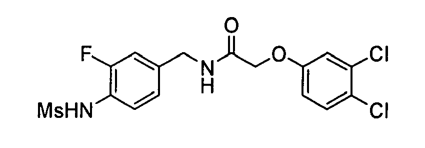

### Step 1: 2-Bromo-N-(3-fluoro-4-methanesulfonylamino-benzyl)-acetamide

3-Fluoro-4-methanesulfonylamino-benzylamine HCl salt (278 mg, 1.17 mmol), pyridine (0.2 ml) and 2-bromoacetylbromide (0.15 ml) were added in THF (50 ml). The reaction mixture was stirred overnight. A reaction solvent was removed in vacuo. The residue was extracted with methylenechloride (30 ml x 3) and H₂O (50 ml). A combined organic layer was washed with brine, dried with MgSO₄, and then concentrated in vacuo. The residue was column chromatographed to yield the title compound (317 mg)
¹H NMR (300 MHz, CDCl₃): δ 7.55 (t, 1H, J = 8.7 Hz), 7.08 (m, 2H), 6.53 (br, 1 H), 4.47 (m, 2H), 3.95 (s, 1H), 3.08 (s, 3H).

### Step 2: 2-(3,4-Dichloro-phenoxy)-N-(3-fluoro-4-methanesulfonylamino-benzyl)-acetamide

2-Bromo-N-(3-fluoro-4-methanesulfonylamino-benzyl)-acetamide (165 mg, 0.486 mmol), 3,4-dichlorophenol (96 mg, 0.59 mmol) and sodium hydride (60 wt% 57 mg, 1.4 mmol) were added in THF (20 ml). The reaction mixture was stirred overnight. The reaction solvent was removed in vacuo. The residue was extracted with methylenechloride (30 ml x 3) and H₂O (30 ml). The combined organic layer was dried with MgSO₄ and concentrated in vacuo. The residue was purified with column chromatography to yield the title compound (14 mg).
¹H NMR (300 MHz, CDCl₃): δ 7.53 (m, 1 H), 7.41 (m, 1 H), 7.08 (m, 4H), 6.97 (br, 1 H), 6.83 (m, 1 H), 4.56 (s, 2H), 4.50 (m, 2H), 3.05 (s, 3H)

### Example 26: N-(3-Fluoro-4-methanesulfonylamino-benzyl)-2-(4-trifluoromethylbenzyloxy)-acetamide

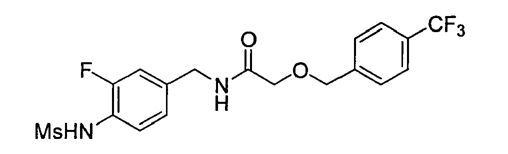

### Step 1: (4-Trifluoromethyl-benzyloxy)-acetic acid

To a suspension of 4-(trifluoromethyl)-benzyl alcohol (100 mg, 0.567 mmol) and ethyl bromoacetate (0.076 ml, 0.681 mmol) in CH₃CN (2ml) was added Cs₂CO₃ (277 mg, 0.850 mmol). The mixture was stirred overnight at room temperature. The reaction mixture was diluted with EtOAc (10 ml) and then washed three times with H₂O (10 ml) and brine, dried over MgSO₄, filtered and concentrated under reduced pressure. To a suspension of the crude residue in THF (2 ml) was added a solution of 0.5 N-LiOH (2.26 ml) and the mixture was stirred for 1.5 hrs at room temperature. The resulting residue was dissolved in H₂O (5 ml) and then washed three times with EtOAc (5 ml), acidified with 1 N HCI to pH 1~2. The solution was extracted three times with methylenechloride (5 ml) and then dried over anhyd. Na₂SO₄ and concentrated in vacuo to give (4-trifluoromethyl-benzyloxy)-acetic acid (106 mg, 80%).
¹H NMR (300 MHz, CDCl₃): δ 7.63 (d, 2H, *J* = 8.1 Hz), 7.49 (d, 2H, *J* = 8.1 Hz), 4.71 (s, 2H), 4.21 (s, 2H)

### Step 2: N-(3-Fluoro-4-methanesulfonylamino-benzyl)-2-(4-trifluoromethylbenzyloxy)-acetamide

To a suspension of 3-fluoro-4-methanesulfonylamino-benzylamine HCl salt (43.5 mg, 0.170 mmol) in THF (2 ml) at 5 °C was added TEA (0.029 ml, 0.204 mmol). The mixture was stirred for 5 minutes at the same temperature, to which were added (4-trifluoromethyl-benzyloxy)-acetic acid (40 mg, 0.170 mmol) and DMTMM (47.04 mg, 0.170 mmol). The mixture was stirred overnight at room temperature and concentrated in vacuo. The residue was diluted with EtOAc (5 ml) and water (5 ml). The organic layer was washed with saturated sodium bicarbonate, 1 N HCl, and brine, dried over anhydrous magnesium sulfate, and concentrated in vacuo to give a solid (68 mg, 92 %).
Mp: 99 ∼ 106°C; ¹H NMR (300 MHz, DMSO-d⁶): δ 9.53 (s. 1 H), 8.50 (t, 1 H, *J =* 6.0 Hz), 7.73 (d, 2H, *J* = 8.4 Hz), 7.62 (d, 2H, *J =* 8.4 Hz), 7.33 (t, 1H, *J =* 8.4Hz), 7.12 (d, 2H, *J* = 11.1 Hz), 7.07(d, 2H, *J* = 0.6Hz), 4.67 (s, 2H), 4.30 (d, 2H, *J* = 6.0 Hz), 4.03 (s, 2H), 2.99 (s, 3H)

### Example 27: 2-(Biphenyl-4-yloxy)-N-(3-fluoro-4-methanesulfonylamino-benzyl)-acetamide

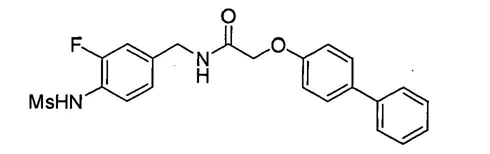

### Step 1: (Biphenyl-4-yloxy)-acetic acid

To a suspension of 4-Phenylphenol (200 mg, 1.175 mmol) and ethyl bromoacetate (0.155 ml, 1.41 mmol) in CH₃CN (5ml) was added Cs₂CO₃ (574 mg, 1.762 mmol). The mixture was stirred overnight at room temperature. The reaction mixture was diluted with EtOAc (15 ml) and then washed three times with H₂O (15 ml) and brine, dried over MgSO₄, filtered and concentrated under reduced pressure. To a suspension of the crude residue in THF (5 ml) was added a solution of 0.5 N-LiOH (4.7 ml), and the mixture was stirred for 1.5 hrs at room temperature. The resulting residue was dissolved in H₂O (5 ml) and then washed three times with EtOAc (5 ml), acidified with 1 N HCl to pH 1~2. The solution was extracted three times with methylenechloride (5 ml) and then dried over anhyd. Na₂SO₄ and concentrated in vacuo to give the title compound (217 mg, 81%).
¹H NMR (300 MHz, DMSO-d⁶): δ 7.62 - 7.58 (m, 4H), 7.43 (t, 1 H, *J* = 7.8 Hz), 7.33 - 7.28 (m, 1 H), 7.00 (d, 2H, *J* = 8.1 Hz), 4.72 (s, 2H)

### Step 2: 2-(Biphenyl-4-yloxy)-N-(3-fluoro-4-methanesulfonylamino-benzyl)-acetamide

To a suspension of 3-fluoro-4-methanesulfonylamino-benzylamine HCl salt (100 mg, 0.393 mmol) in THF (10 ml) at 5 °C was added TEA (0.066 ml, 0.471 mmol). The mixture was stirred for 5 minutes at the same temperature, to which were added (biphenyl-4-yloxy)-acetic acid (90 mg, 0.393 mmol) and DMTMM (119.5 mg, 0.432 mmol). The mixture was stirred overnight at room temperature and concentrated in vacuo. The residue was diluted with EtOAc (15 ml) and water (15 ml). The organic layer was washed with saturated sodium bicarbonate, 1 N HCl and brine, dried over anhydrous magnesium sulfate, and concentrated in vacuo to give a solid. The solid was purified by recrystallization from EtOAc/hexane to afford the title compound (68 mg, 92 %).
Mp: 187 ~ 195°C; ¹H NMR (300 MHz, DMSO-d⁶): δ 9.53 (s. 1H), 8.73 (t, 1H, *J* = 6.0 Hz), 7.61 (d, 4H, *J*= 8.1 Hz), 7.43 (t, 2H, *J* = 7.8 Hz), 7.31 (t, 2H, *J* = 7.8 Hz), 7.13 (d, 1 H, *J* = 11.4 Hz), 7.06 (d, 3H, *J* = 8.1 Hz), 4.62 (s, 2H), 4.33 (d, 2H, *J* = 6.0 Hz), 2.98 (s, 3H)

### Example 28: N-(3-Fluoro-4-methanesulfonylaminobenzyl)-2-(4-(trifluoromethoxy)phenoxy)acetamide

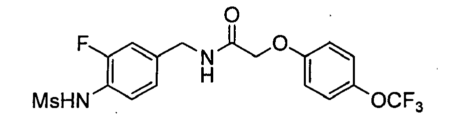

### Step 1: Synthesis of 4-(trifluoromethoxy)phenoxyacetic acid

To a solution of 4-(trifluoromethoxy)phenol (0.50 g, 2.81 mmol) in acetonitrile (10 mL) were added cesium carbonate (1.37 g, 4.2 mmol) and ethyl bromoacetate (0.34 mL, 3.01 mmol). The resulting mixture was stirred for 1.5 hours at room temperature, concentrated under reduced pressure, and diluted with EtOAc and water. The organic layer was washed with brine and concentrated under reduced pressure. The crude residue was diluted with THF (7 mL) and 1 N LiOH (5 mL), and then stirred for 1 hour at room temperature. The mixture was acidified with 6 N HCI, concentrated under reduced pressure, and diluted with EtOAc and water. The organic layer was washed with brine, dried over anhydrous magnesium sulfate, filtered, and concentrated under reduced pressure. The crude residue was then purified by recrystallization from EtOAc/hexane to afford the title compound (0.54 g,72.8%) as a white solid.
¹H NMR (300 MHz, CDCl₃): δ 7.18 (d, 2H, *J =* 8.4 Hz), 6.93 (d, 2H, *J =* 9 Hz), 4.70 (s, 2 H)

### Step 2: Synthesis of N-(3-fluoro-4-methanesulfonylaminobenzyl)-(4-(trifluoromethoxy)phenoxy) acetamide

To a suspension of 3-fluoro-4-methanesulfonylaminobenzylamine hydrochloride (100 mg, 0.39 mmol) in THF (5 mL) was added TEA (82 µL, 0.59 mmol). The mixture was stirred for 5 minutes, to which were added 4-(trifluoromethoxy)phenoxyacetic acid (100 mg, 0.42 mmol) and DMTMM (120 mg, 0.43 mmol). The mixture was stirred overnight at room temperature and was concentrated under reduced pressure. The residue was diluted with EtOAc and water. The organic layer was washed with 3N HCl, saturated sodium bicarbonate and brine, dried over anhydrous magnesium sulfate, filtered, and concentrated under reduced pressure. The crude residue was purified by recrystallization from EtOAc/hexane to afford the title compound (153 mg,89.3%) as a white solid.
Mp: 123 ~ 124°C; ¹H NMR (300MHz, CDCl₃): δ 7.54 (t, 1 H, *J* = 8.4 Hz), 7.19 (d, 2 H, *J* = 9 Hz), 7.09 (d, 2 H, *J* = 9 Hz), 6.95 ∼ 6.86 (m, 3 H), 6.46 (br s, 1 H), 4.56 (s, 2 H), 4.53 (d, 2 H, *J* = 6.3 Hz)

### Example 29: 2-(Benzo[1,3]dioxol-5-yloxy)-N-(3-fluoro-4-methanesulfonylaminobenzyl)-acetamide

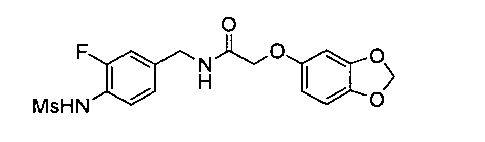

### Step 1: Ethyl (Benzo[1,3]dioxol-5-yloxy)-acetate

Sesamol (144 mg, 1.04 mmol) and sodium hydride (60 wt%, 61 mg, 1.52 mmol) were added in THF (20ml). After stirring for 10 mins, ethyl 2-bromoacetate (0.12 ml, 1.08 mmol) was added into the mixture. The reaction mixture was stirred for 6 hrs. The reaction solvent was removed in vacuo. The residue was extracted with methylenechloride (50 ml). A combined organic layer was washed with 1 M K₂CO₃ aqueous solution, dried with MgSO₄, and concentrated in vacuo. The residue was purified with column chromatography to yield the title compound (137 mg)
¹H NMR (300MHz, CDCl₃): δ 6.64 (d, 1 H), 6.48 (d, 1 H), 6.26 (dd, 1 H), 4.94 (s, 2H), 4.21 (q, 2H), 1.24 (t, 3H).

### Step 2: (Benzo[1,3]dioxol-5-yloxy)-acetatic acid

Ethyl (Benzo[1,3]dioxol-5-yloxy)-acetate (137 mg, 0.611 mmol) was dissolved in methanol (30 ml). 1 M KOH aqueous solution (1.2 ml) was added into the mixture. The reaction mixture was stirred overnight. A reaction solvent was removed in vacuo. A residue was extracted with methylenechloride (30 ml x 3) and H₂O (30 ml). A combined organic layer was dried with MgSO₄ and then concentrated in vacuo. A residue was purified with column chromatography to yield the title compound (114mg, 0.582 mmol)
¹H NMR (300 MHz, CDCl₃): δ 6.62 (d, 1 H), 6.54 (d, 1 H), 6.35 (dd, 1 H), 5.94 (s, 2H), 4.61 (s, 2H).

### Step 3: 2-(Benzo[1,3]dioxol-5-yloxy)-N-(3-fluoro-4-methanesulfonylaminobenzyl)-acetamide

3-Fluoro-4-(methanesulfonylamino)benzylamine HCl salt (71 mg, 0.28 mmol), (Benzo[1,3]dioxol-5-yloxy)-acetatic acid (52 mg, 0.26 mmol) and NMP (0.2 ml) were added in THF (20 ml). DMTMM (97 mg, 0.35 mmol) was added into the mixture. The reaction mixture was stirred overnight at room temperature. The reaction solvent was removed in vacuo. The residue was extracted with ethylacetate (50 ml x 2) and H₂O (40 ml). A combined organic layer was washed with sat K₂CO₃ aqueous solution (30 ml x 2), 1 M KOH solution (30 ml) and then brine. A organic layer was concentrated in vacuo and purified with column chromatography to yield the title compound (64mg)
Mp: 152 ~ 153 °C; ¹H NMR (300 MHz, CDCl₃): δ 7.53 (m, 1 H), 7.26 (m, 1 H), 7.07 (d, 2H), 6.92 (br, 1 H), 6.71 (d, 1 H, J = 8.4 Hz), 6.49 (d, 1 H, J = 2.4 Hz), 6.32 (dd, 1 H, J = 2.4, 8.4 Hz), 4.52 (m, 2H), 4.49 (s, 2H), 3.02 (s, 3H)
IR (neat, cm⁻¹): 1661, 1488, 1309, 1182.

### Example 30: 2-(Benzo[1,3]dioxol-5-yloxy)-N-(3-fluoro-4-methanesulfonylamino-5-vinyl-benzyl)-acetamide

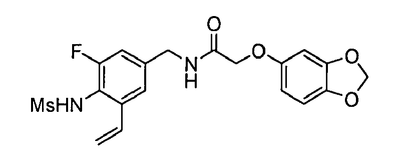

3-Fluoro-4-(methanesulfonylamino)-5-vinyl-benzylamine HCl salt (83 mg, 0.29 mmol), (Benzo[1,3]dioxol-5-yloxy)-acetatic acid (527.4 mg, 0.293 mmol) and NMP (0.2 ml) were added in THF (20 ml). DMTMM (82 mg, 0.29 mmol) was added into the mixture. The reaction mixture was stirred overnight at room temperature. The reaction solvent was removed in vacuo. The residue was extracted with ethylacetate (50 ml x 2) and H₂O (40 ml). A combined organic layer was washed with sat K₂CO₃ aqueous solution (30 ml x 2), 1 M KOH solution (30 ml) and then brine. A organic layer was concentrated in vacuo and purified with column chromatography to yield the title compound (30.3 mg)
Mp: 174 ~ 176 °C; ¹H NMR (300 MHz, CDCl₃): δ 7.28 (s, 1 H), 7.18 (d, 1 H), 7.10 (d, 1H), 6.99, (dd, 1 H), 6.90 (br, 1H), 6.67 (d, 1 H, J = 8.4 Hz), 6.46 (d, 1H, J = 2.4 Hz), 6.28 (dd, 1H, J = 2.4, 8.4 Hz), 5.89 (s, 2H), 5.73 (d, 1H, d = 18 Hz), 5.42 (d, 1H, J = 10.8 Hz), 4.49 (d, 2H, J = 6.3 Hz), 4.45 (s, 2H), 3.03 (s, 3H)

### Example 31: N-(3-Fluoro-4-methanesulfonylamino-benzyl)-2-(2-methoxy-4-methyl-phenoxy)-acetamide

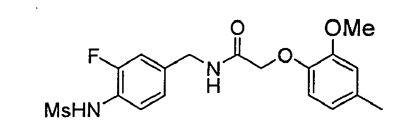

### Step 1: (2-Methoxy-4-methyl-phenoxy)-acetic acid

3-Methoxy-4-methylphenol (378 mg, 2.73 mmol) and sodium hydride (60 wt%, 201 mg) were added in THF (20 ml). After stirring for 20mins, ethyl 2-bromoacetate (0.3 ml, 2.70 mmol) was added into the mixture. The reaction solvent was removed in vacuo. The residue was extracted with methylenechloride (30 ml x 2) and H₂O (30 ml). A combined organic layer was washed with 1 M KOH solution (30 ml x 3), dried with MgSO₄, and concentrated in vacuo. Methanol (40 ml) was added into the residue. 1M KOH solution (2.5 ml) was added into the mixture. A reaction mixture was stirred for 4 hrs. A reaction solvent was removed in vacuo. A residue was acidified with 5 % HCl solution. An aqueous solution was extracted with methylenechloride (30 ml x 2). A combined organic phase was dried with MgSO₄ and then concentrated in vacuo. A residue was crystallized with ethyl acetate and hexane.
¹H NMR (300 MHz, CDCl₃): δ 6.87 (d, 1H, J = 7.8 Hz), 6.75 (m, 2H), 4.62 (s, 2H), 3.89 (s, 2H)

### Step 2: N-(3-Fluoro-4-methanesulfonylamino-benzyl)-2-(2-methoxy-4-methylphenoxy)-acetamide

3-Fluoro-4-methanesulfonylamino-benzylamine HCl salt (108.2 mg, 0.424 mmol), NMP (0.2 ml), and (2-methoxy-4-methyl-phenoxy)-acetic acid (81 mg, 0.41 mmol) were added in THF (50 ml). After the mixture was stirred for 10 mins, DMTMM (151 mg, 0.545 mmol) was added into the mixture. The reaction mixture was stirred for overnight. The reaction solvent was removed in vacuo. The residue was extracted with ethylacetate (60 ml) and H₂O (30 ml). The organic layer was washed with saturated K₂CO₃ (30 ml), 5% HCl solution (30 ml) and brine (30 ml), dried with MgSO₄ and concentrated in vacuo. The residue was purified with column chromatography to yield the title compound (126 mg, 78%) as a white solid.
Mp: 119 ~ 120 °C; ¹H NMR (300 MHz, CDCl₃): δ 7.45 (t, 2H, J = 7.5 Hz), 6.99 (d, 2H), 6.76 (m,, 1 H), 6.66, (m, 2H), 6.60 (br, 1 H), 4.53 (s, 2H), 4.44 (d, 2H, J = 9.0 Hz), 3.76 (s, 3H), 2.96 (s, 3H), 2.77 (s, 3H)

### Example 32: 2-(4-Chloro-2-methoxy-phenoxy)-N-(3-fluoro-4methanesulfonylamino-benzyl)-acetamide

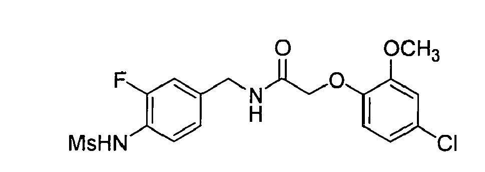

### Step 1: (2,4-Dichloro-phenoxy)-acetic acid

4-Chloro-2-methoxyphenol (1.0eq, 0.2g, 1.26mmol) was put into the 50 ml one-neck round bottom flask and was filled in 20ml acetonitrile. To the solution was poured Cs₂CO₃ (1.2eq, 0.493g) slowly and stirred for 10 minutes at room temperature. After then tert-butylbromoacetate (1.0eq, 0.18ml) was added in this mixture. The reaction mixture was stirred for 3 hrs. After confirming the completion of the reaction with TLC, the reaction solution was extracted with EtOAc, washed with water and brine, dried over MgSO₄, filtered and concentrated under reduced pressure. The obtained solid was column chromatographed (n-hexane/EtOAc=4/1). The obtained solid was dissolved in methylenechloride, added 3ml of trifluoroacetic acid and stirred for 24 hours. After confirming the completion of the reaction with TLC, the reaction solution was extracted with methylene, washed with 1 N HCl and brine, dried over MgSO₄, filtered and concentrated under reduced pressure to yield a solid (0.25g, 91.6%)
Mp: 125-126 °C; ¹H NMR (300MHZ, DMSO-d₆): δ 3.79 (s, 3H), 4.66 (s, 2H), 6.89 (m, 2H), 7.03 (d, 1 H, *J*=2.1 Hz)

### Step 2: 2-(4-Chloro-2-methoxy-phenoxy)-N-(3-fluoro-4-methanesulfonylaminobenzyl)-acetamide

(4-Chloro-2-methoxy-phenoxy)-acetic acid (100 mg, 0.462 mmol) and DMTMM (1.1eq, 141mg) were put into the 25 ml round bottom flask and was filled in 15ml THF and stirred for 10 minutes. After then to this reaction mixture were added 3-fluoro-4-methanesulfonylbenzylamine hydrochloride (1.0eq, 118mg) and TEA (excess, 0.3ml), and stirred for 18 hours. After confirming the completion of the reaction with TLC, the reaction mixture was extracted with ethylacetate, washed with 1 N HCI solution and brine, dried over MgSO₄, filtered and concentrated under reduced pressure. The obtained liquid was column chromatographed (n-hexane/EtOAc=2/3) to yield a white solid (143 mg, 74.3%).
Mp: 114~115 °C; ¹H NMR (300MHZ, CDCl₃) : δ 3.03 (s, 3H), 3.79 (s, 3H), 4.50 (d, 2H, J=6Hz), 4.57 (s, 2H), 6.52 (bs, 1 H), 6.89 (m, 1 H), 7.06 (bs, 1 H), 7.10 (bs, 1 H), 7.32 (bs, 1H), 7.53 (t, 1 H, J=8.4Hz)

### Example 33: N-(3-Fluoro-4-methanesulfonylaminobenzyl)-2-(3,5-dichlorophenoxy)acetamide

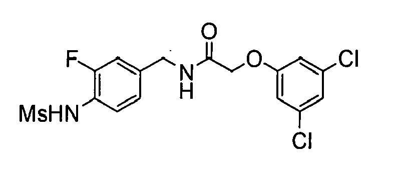

### Step 1: Synthesis of 3,5-di-chlorophenoxyacetic acid

To a solution of 3,5-dichlorophenol (0.46 g, 2.82 mmol) in acetonitrile (10 mL) were added cesium carbonate (1.37 g, 4.2 mmol) and ethyl bromoacetate (0.34 mL, 3.01 mmol). The resulting mixture was stirred for 1.5 hours at room temperature, concentrated under reduced pressure, and diluted with EtOAc and water. The organic layer was washed with brine and concentrated under reduced pressure. The crude residue was diluted with THF (7 mL) and 1 N LiOH (5 mL), and then stirred for 1 hour at room temperature. The mixture was acidified with 6 N HCI, concentrated under reduced pressure, and diluted with EtOAc and water. The organic layer was washed with brine, dried over anhydrous magnesium sulfate, filtered, and concentrated under reduced pressure. The crude residue was then purified by recrystallization from EtOAc/hexane to afford the title compound (0.43 g, 68.9%) as a white solid.
¹H NMR (300MHz, CDCl₃): δ 7.04 (t, 1 H, *J* = 1.5 Hz), 6.83 (d, 2H, *J* = 1.5 Hz), 4.68 (s, 2H)

### Step 2: Synthesis of N-(3-fluoro-4-methanesulfonylaminobenzyl)-(3,5-dichlorophenoxy) acetamide

To a suspension of 3-fluoro-4-methanesulfonylaminobenzylamine hydrochloride (100 mg, 0.39 mmol) in THF (5 mL) was added TEA (82 µL, 0.59 mmol). The mixture was stirred for 5 minutes, to which were added 3,5-dichlorophenoxyacetic acid (93 mg, 0.42 mmol) and DMTMM (120 mg, 0.43 mmol). The mixture was stirred overnight at room temperature and was concentrated under reduced pressure. The residue was diluted with EtOAc and water. The organic layer was washed with 3N HCl, saturated sodium bicarbonate, and brine, dried over anhydrous magnesium sulfate, filtered, and concentrated under reduced pressure. The crude residue was purified by recrystallization from EtOAc/hexane to afford the title compound (125 mg, 75.6%) as a white solid.
Mp: 194 ~ 195°C; ¹H NMR (300MHz, DMSO-d₆): δ 9.54 (s, 1H), 8.72 (t, 1H, *J* = 6.3 Hz), 7.32 (t, 1H, *J* = 8.4 Hz), 7.20 (t, 1H, *J* = 1.8 Hz), 7.15 ∼ 7.04 (m, 4H), 4.67 (s, 2H), 4.32 (d, 2H, *J* = 6 Hz), 2.99 (s, 3H)

### Example 34: 2-(4-Chloro-phenoxy)-N-(3-fluoro-4-methanesulfonylamino-benzyl)-acetamide

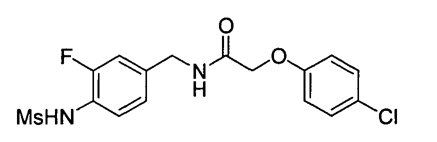

### Step 1: 4-Chloro-phenoxy-acetic acid

4-Chloro-phenol (1.0eq, 0.3g, 2.33mmol) was put into the 50 ml one-neck round bottom flask and was filled in 20ml acetonitrile. To the solution was poured Cs₂CO₃ (1.2eq, 0.91 g) slowly and stirred for 10 minutes at room temperature. After then ethyl bromoacetate (1.0eq, 0.26ml) was added in this mixture, and stirred for 3 hours. After confirming the completion of the reaction with TLC, the reaction solution was extracted with EtOAc, washed with water and brine, dried over MgSO₄, filtered and concentrated under reduced pressure. The obtained solid was column chromatographed (n-hexane/EtOAc=4/1). The obtained solid was dissolved in THF:methanol solution, added 5ml of 1M LiOH solution and stirred for 2 hours. After confirming the completion of the reaction with TLC, the reaction solution was evaporated in vacuo, extracted with EtOAc, washed with 1 N HCl and brine, dried over MgSO₄, filtered and concentrated under reduced pressure to yield a white solid (0.411g, 94.4%).
Mp: 152~153 °C; ¹H NMR (300MHZ, DMSO-d₆): δ 4.68 (s, 2H), 6.94 (m, 2H), 7.32 (m, 2H)

### Step 2: 2-(4-Chloro-phenoxy)-N-(3-fluoro-4-methanesulfonylamino-benzyl)-acetamide

4-Chloro-phenoxy-acetic acid (100mg, 0.536mmol) and DMTMM (1.2eq, 178mg) were put into the 25 ml round bottom flask and was filled in 15ml of THF and stirred for 10 minutes. After then to this reaction mixture were added N-(4-aminomethyl-2-fluoro-phenyl)methanesulfonamide hydrochloride (1.0eq, 137mg) and TEA (excess, 0.3ml) and stirred for 18 hours. After confirming the completion of the reaction with TLC, the reaction mixture was extracted with ethylacetate, washed with 1 N HCl solution and brine, dried over MgSO₄, filtered and concentrated under reduced pressure. The obtained solid was column chromatographed (n-hexane/EtOAc=1/1) to yield a white solid (153mg, 73.8%).
Mp: 114~116 °C; ¹H NMR (300 MHz, CDCl₃): δ 3.03 (s, 3H), 4.52 (d, 2H, *J*=6.3Hz), 4.54 (s, 2H), 6.49 (bs, 1 H), 6.86 (m, 2H), 6.91 (bs, 1H), 7.09 (d, 2H, J=9Hz), 7.28 (m, 2H), 7.53 (t, 1H, *J*=8.1Hz)

### Example 35: N-(3-Fluoro-4-methanesulfonylaminobenzyl)-2-(3-methyl-4-nitrophenoxy)acetamide

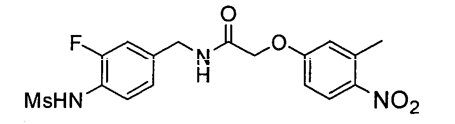

### Step 1: Synthesis of 3-methyl-4-nitrophenoxyacetic acid

To a solution of 3-methyl-4-nitrophenol (0.43 g, 2.81 mmol) in acetonitrile (10 mL) were added cesium carbonate (1.37 g, 4.2 mmol) and ethyl bromoacetate (0.34 mL, 3.01 mmol). The resulting mixture was stirred for 1.5 hours at room temperature, concentrated under reduced pressure, and diluted with EtOAc and water. The organic layer was washed with brine and concentrated under reduced pressure. The crude residue was diluted with THF (7 mL) and 1 N LiOH (5 mL), and then stirred for 1 hour at room temperature. The mixture was acidified with 6 N HCl, concentrated under reduced pressure, and diluted with EtOAc and water. The organic layer was washed with brine, dried over anhydrous magnesium sulfate, filtered, and concentrated under reduced pressure. The crude residue was then purified by recrystallization from EtOAc/hexane to afford the title compound (0.39 g,65.8%) as a pale yellow solid.
¹H NMR (300MHz, CDCl₃): δ 8.09 (dd, 1H, *J* = 7.5, 1.8 Hz), 6.84 (s, 1H), 6.81 (d, 1 H, *J* = 2.7 Hz), 4.78 (s, 2H), 2.64 (s, 3H)

### Step 2: Synthesis of N-(3-Fluoro-4-methanesulfonylaminobenzyl)-2-(3-methyl-4-nitrophenoxy) acetamide

To a suspension of 3-fluoro-4-methanesulfonylaminobenzylamine hydrochloride (100 mg, 0.39 mmol) in THF (5 mL) was added TEA (82 µL, 0.59 mmol). The mixture was stirred for 5 minutes, to which were added 3-methyl-4-nitrophenoxyacetic acid (88 mg, 0.42 mmol) and DMTMM (120 mg, 0.43 mmol). The mixture was stirred overnight at room temperature and was concentrated under reduced pressure. The residue was diluted with EtOAc and water. The organic layer was washed with 3N HCl, saturated sodium bicarbonate, and brine, dried over anhydrous magnesium sulfate, filtered, and concentrated under reduced pressure. The crude residue was purified by recrystallization from EtOAc/hexane to afford the title compound (135 mg,83.6%) as a white solid.

Mp: 160 ~ 161°C; ¹H NMR (300 MHz, CDCl₃): δ 8.10 (d, 1H, *J* = 9.9 Hz), 7.54 (t, 1H, *J* = 8.4 Hz), 7.11 (s, 1 H), 7.09 (d, 2H), 6.86 ∼ 6.80 (m, 3H), 6.46 (br s, 1 H), 4.63 (s, 2H), 4.53 (d, 2H, *J* = 6.3 Hz), 3.03 (s, 3H), 2.64 (s, 3H)

### Example 36: N-(3-Fluoro-4-methanesulfonylamino-benzyl)-2-(4-iodo-phenoxy)-acetamide

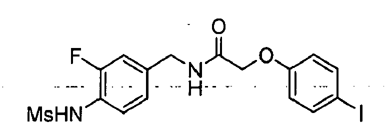

### Step 1: Synthesis of ethyl-(4-iodo-phenoxy)-acetate

4-lodophenol (327.7 mg, 1.48 mmol) and sodium hydride (60 wt%, 85 mg, 2.1 mmol) were added in THF (20 ml). After a mixture was stirred for 20 mins, ethyl 2-bromoacetate (0.2 ml, 1.8 mmol) was added into the mixture. The reaction mixture was stirred overnight. A reaction solvent was removed in vacuo. The residue was extracted with methylenechloride (30 ml x 2) and H₂O (30 ml). The combined organic layer was washed with 1 M KOH (30 ml x 3), dried with MgSO₄, and concentrated in vacuo to yield the title compound.
¹H NMR (300 MHz, CDCl₃): δ 7.53 (d, 2H), 6.64 (d, 2H), 4.53 (s, 2H), 4.22 (q, 2H, J = 7.2 Hz), 1.26 (t, 3H)

### Step 2: Synthesis of (4-iodo-phenoxy)-acetic acid

ethyl-(4-iodo-phenoxy)-acetate was dissolved in methanol (30 mL) and was added 1 M KOH aqueous solution (3 ml). The reaction mixture was stirred for 4 hrs. The reaction solvent was removed in vacuo. The residue was acidified with 5 % HCl solution and then extracted with methylenechloride. The combined organic layer was dried with MgSO₄ and then concentrated to give the title compound.
¹H NMR (300 MHz, CDCl₃): δ 7.60 (d, 2H), 6.71 (d, 2H), 4.66 (s, 2H)

### Step 3: N-(3-Fluoro-4-methanesulfonylamino-benzyl)-2-(4-iodo-phenoxy)-acetamide

3-fluoro-4-methanesulfonylamino-benzylamine HCI salt (120 mg, 0.471 mmol), NMP (0.25 ml), and (4-iodo-phenoxy)-acetic acid (121 mg, 0.435 mmol) were added in THF (50 ml). After the mixture was stirred for 10 mins, DMTMM (138 mg, 0.500 mmol) was added into the mixture. The reaction mixture was stirred overnight. The reaction solvent was removed in vacuo. The residue was extracted with ethylacetate (60 ml) and H₂O (30 ml). The organic layer was washed with saturated K₂CO₃ (30 ml), 5% HCl solution (30 ml) and brine (30 ml). The organic layer was dried with MgSO₄ and concentrated in vacuo. The residue was purified with column chromatography to yield the title compound (172 mg, 83%) as a white solid.
Mp: 172~153 °C; ¹H NMR (300 MHz, CDCl₃): δ 7.60 (d, 2H), 7.53 (m, 1H), 7.08 (d, 2H), 6.89 (br, 1 H), 6.69 (m, 1H), 6.45 (br, 1H), 4.52 (m, 2H), 4.50 (s, 2H), 3.02 (s, 3H)
IR (neat, cm⁻¹): 1674, 1650, 1523, 1482, 1330, 1152.
MS : 478 (M)

### Example 37: N-(3-Fluoro-4-methanesulfonylaminobenzyl)-2-(2,4-difluorophenoxy)acetamide

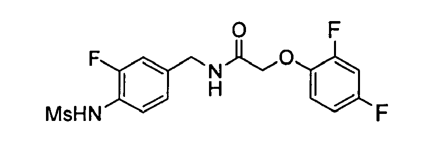

### Step 1: Synthesis of 2,4-difluorophenoxyacetic acid

To a solution of 2,4-difluorophenol (0.37 g, 2.84 mmol) in acetonitrile (10 mL) were added cesium carbonate (1.37 g, 4.2 mmol) and ethyl bromoacetate (0.34 mL, 3.01 mmol). The resulting mixture was stirred for 1.5 hours at room temperature, concentrated under reduced pressure, and diluted with EtOAc and water. The organic layer was washed with brine and concentrated under reduced pressure. The crude residue was diluted with THF (7 mL) and 1 N LiOH (5 mL), and then stirred for 1 hour at room temperature. The mixture was acidified with 6 N HCl, concentrated under reduced pressure, and diluted with EtOAc and water. The organic layer was washed with brine, dried over anhydrous magnesium sulfate, filtered, and concentrated under reduced pressure. The crude residue was then purified by recrystallization from EtOAc/hexane to afford the title compound (0.24 g,44.9%) as a white solid.
¹H NMR (300MHz, CDCl₃): δ 7.01 ∼ 6.86 (m, 2H), 6.84 ∼ 6.77 (m, 1 H), 4.72 (s, 2 H)

### Step 2: Synthesis of N-(3-Fluoro-4-methanesulfonylaminobenzyl)-(3-methyl-4-nitrophenoxy) acetamide

To a suspension of 3-fluoro-4-methanesulfonylaminobenzylamine hydrochloride (100 mg, 0.39 mmol) in THF (5 mL) was added TEA (82 µL, 0.59 mmol). The mixture was stirred for 5 minutes, to which were added 2,4-difluorophenoxyacetic acid (79 mg, 0.42 mmol) and DMTMM (120 mg, 0.43 mmol). The mixture was stirred overnight at room temperature, and concentrated under reduced pressure. The residue was diluted with EtOAc and water. The organic layer was washed with 3N HCl, saturated sodium bicarbonate, and brine, dried over anhydrous magnesium sulfate, filtered, and concentrated under reduced pressure. The crude residue was purified by recrystallization from EtOAc/hexane to afford the title compound (115 mg, 70.5%) as a white solid.

Mp: 108 ~ 109 °C; ¹H NMR (300MHz, CDCl₃): δ 7.55 (t, 1H, *J* = 8.1 Hz), 7.15 ~ 7.02 (m, 3H), 6.98 ∼ 6.79 (m, 3H), 6.47 (br s, 1 H), 4.57 (s, 2H), 4.54 (d, 2H, *J* = 6.3 Hz), 3.03 (s, 3H)

### Example 38: 2-(2,6-Diiodo-4-trifluoromethyl-phenoxy)-N-(3-fluoro-4-methanesulfonylamino-benzyl)-acetamide

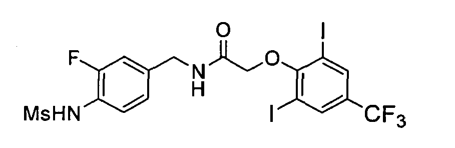

### Step 1: 2,6-Diiodo-4-trifluoromethyl-phenol

To a solution of iodine (411 mg, 3.24 mmol) in EtOH was added a solution of α,α,α-trifluoro-p-cresol (263 mg, 1.62 mmol) and Ag₂SO₄ (607 mg, 1.94 mmol) in EtOH, and the mixture was stirred for 3hrs at ambient temperature under dark. The reaction mixture was filtered through celite pad and then concentrated. The resulting residue was dissolved in EtOAc and then washed three times with 10% Na₂S₂O₃ solution, washed with brine, dried over anhyd. MgSO₄, filtered and concentrated under reduced pressure. The obtained residue was short column chromatographed to yield 2,6-diiodo-4-trifluoromethyl-phenol (100 mg, 21 %).
¹H NMR (300MHz, CDCl₃): δ 7.92 (s, 2H), 6.06 (s, 1H)

### Step 2: (2,6-Diiodo-4-trifluoromethyl-phenoxy)-acetic acid

To a suspension of 2,6-diiodo-4-trifluoromethyl-phenol (40 mg, 0.096 mmol) and ethyl bromoacetate (0.013 ml, 0.116 mmol) in CH₃CN (1ml) was added Cs₂CO₃ (47.23 mg, 0.145 mmol). The mixture was stirred overnight at room temperature. The reaction mixture was diluted with EtOAc (5 ml) and then washed three times with H₂O (5 ml) and brine, dried over MgSO₄, filtered and concentrated under reduced pressure. To a suspension of the crude residue in THF (5 ml) was added a solution of 0.5 N-LiOH (0.4 ml), and the mixture was stirred for 1.5 hrs at room temperature. The resulting residue was dissolved in H₂O (5 ml) and then washed three times with EtOAc (2 ml), acidified with 1 N HCl to pH 1~2. The solution was extracted three times with methylenechloride (2 ml) and then dried over anhyd. Na₂SO₄ and concentrated in vacuo to give (2,6-diiodo-4-trifluoromethyl-phenoxy)-acetic acid (41 mg, 90%).
¹H NMR (300MHz, CDCl₃): δ 8.04 (s, 2H), 4.71 (s, 2H)

### Step 3: 2-(2,6-Diiodo-4-trifluoromethyl-phenoxy)-N-(3-fluoro-4-methanesulfonylamino-benzyl)-acetamide

3-fluoro-4-methanesulfonylamino-benzylamine HCl salt (22.1 mg, 0.087mmol) was suspended in THF (2mL) and treated with TEA (0.012mL). The resulting mixture was stirred for 10mins. (2,6-diiodo-4-trifluoromethyl-phenoxy)-acetic acid (41 mg, 0.087 mmol) was added to the reaction mixture followed by DMTMM (24 mg, 0.095 mmol) after 10 mins. The resulting mixture was stirred overnight at ambient temperature and then diluted with EtOAc. The resulting solution was washed successively with water, sat'd NaHCO₃ (x3), 1 N HCl, and brine, and then dried over anhyd. MgSO₄, filtered and concentrated under reduced pressure. The crude residue was recrystallized from EtOAC/n-hexane to yield the title compound (35 mg, 60%).
Mp: 190 ~ 192°C; ¹HNMR (300MHz, CDCl₃): δ 8.03 (s. 2H), 7.57 (t, 1H, *J* = 8.4Hz), 7.22 ∼ 7.17 (m, 2H), 6.55 (s, 1H), 4.61 (d, 2H, *J =* 7.2 Hz), 4.59 (s, 2H), 3.03 (s, 3H)

### Example 39: N-(3-Fluoro-4-methanesulfonylamino-benzyl)-2-(2-iodo-4-trifluoromethyl-phenoxy)-acetamide

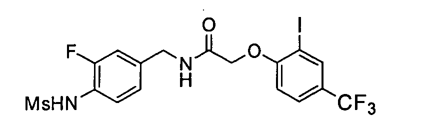

### Step 1: 2-lodo-4-trifluoromethyl-phenol

To a solution of iodine (214 mg, 1.96 mmol) in EtOH was added a solution of α,α,α-Trifluoro-p-cresol (274 mg, 1.69 mmol) and Ag₂SO₄ (632 mg, 2.03 mmol) in EtOH, and the mixture was stirred for 3hrs at ambient temperature under dark. The reaction mixture was filtered through celite pad and then concentrated. The resulting residue was dissolved in EtOAc and then washed three times with 10% Na₂S₂O₃ solution, washed with brine, dried over anhyd. MgSO₄, filtered and concentrated under reduced pressure. The obtained residue was short column chromatographed to yield the title compound (156 mg, 21 %).
¹H NMR (300MHz, CDCl₃): δ 7.92 (s, 2H), 7.53 ∼ 7.49 (m, 1 H), 6.88 (d, 1 H, *J* = 0.6 Hz)

### Step 2: (2-lodo-4-trifluoromethyl-phenoxy)-acetic acid

To a suspension of 2-iodo-4-trifluoromethyl-phenol (70 mg, 0.243 mmol) and ethyl bromoacetate (0.032 ml, 0.291 mmol) in CH₃CN (1ml) was added Cs₂CO₃ (118 mg, 0.364 mmol). The mixture was stirred for overnight at room temperature. The reaction mixture was diluted with EtOAc (5 ml) and then washed three times with H₂O (5 ml) and brine, dried over MgSO₄, filtered and concentrated under reduced pressure. To a suspension of the crude residue in THF (5 ml) was added a solution of 0.5 N-LiOH (0.5 ml), and the mixture was stirred for 1.5 hrs at room temperature. The resulting residue was dissolved in H₂O (5 ml) and then washed three times with EtOAc (2 ml), acidified with 1 N HCl to pH 1 ~ 2. The solution was extracted three times with methylenechloride (2 ml) and then dried over anhyd. Na₂SO₄ and concentrated in vacuo to give (2-iodo-4-trifluoromethyl-phenoxy)-acetic acid (25 mg, 30%).
¹H NMR (300MHz, CDCl₃): δ 9.35 (s, 1H, br), 8.06 (d. 1H, *J* = 2.4 Hz), 7.59 ∼ 7.56 (m, 2H), 6.77 (d, 2H, *J* = 8.7 Hz), 4.81 (s, 2H)

### Step 3: N-(3-Fluoro-4-methanesulfonylamino-benzyl)-2-(2-iodo-4-trifluoromethyl-phenoxy)-acetamide

The compound 3-fluoro-4-methanesulfonylamino-benzylamine HCl salt (18.4 mg, 0.072mmol) was suspended in THF (2mL), treated with TEA (0.01mL) and stirred for 10mins. (2-lodo-4-trifluoromethyl-phenoxy)-acetic acid (25 mg, 0.072 mmol) was added to the reaction mixture followed by DMTMM (22 mg, 0.079 mmol) after 10 mins. The resulting mixture was stirred overnight at ambient temperature and then diluted with EtOAc. The resulting solution was washed successively with water, sat'd NaHCO₃ (x3), 1 N HCl and brine, and then dried over anhyd. MgSO₄, filtered and concentrated under reduced pressure. The crude residue was recrystallized from EtOAC/n-hexane to yield the title compound (35 mg, 89%).
Mp: 152 ~ 155°C; ¹H NMR (300MHz, CDCl₃): δ 8.04∼8.03 (m. 1H), 7.65 ~ 7.61 (m, 1 H), 7.55 (t, 1 H, *J* = 8.1 Hz), 7.28∼7.26 (m, 1 H), 7.17 ∼ 7.13 (m, 1H), 6.86 (d, 1 H, *J* = 8.7 Hz), 6.55 (s, 1H), 4.64 (s, 2H), 4.57 (d, 2H, *J* = 6.3 Hz), 3.03 (s, 3H)

### Example 40: N-(3-Fluoro-4-methanesulfonylaminobenzyl)-2-(3-tert-butylphenoxy)acetamide

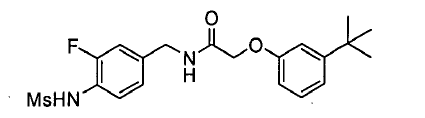

### Step 1: Synthesis of 3-tert-butylphenoxyacetic acid

To a solution of 3-tert-butylphenol (0.42 g, 2.81 mmol) in acetonitrile (10 mL) were added cesium carbonate (1.37 g, 4.2 mmol) and ethyl bromoacetate (0.34 mL, 3.01 mmol). The resulting mixture was stirred for 1.5 hours at room temperature, concentrated under reduced pressure, and diluted with EtOAc and water. The organic layer was washed with brine and concentrated under reduced pressure. The crude residue was diluted with THF (7 mL) and 1 N LiOH (5 mL), and then stirred for 1 hour at room temperature. The mixture was acidified with 6 N HCl, concentrated under reduced pressure, and diluted with EtOAc and water. The organic layer was washed with brine, dried over anhydrous magnesium sulfate, filtered, and concentrated under reduced pressure. The crude residue was then purified by recrystallization from EtOAc/hexane to afford the title compound (0.45 g, 77.3%) as a white solid.
¹H NMR (300MHz, CDCl₃): δ 7.25 (t, 1H, *J* = 8.1 Hz), 7.08~7.05 (m, 1H), 7.01 (t, 1H, *J* = 2.4), 6.72 ∼ 6.80 (m, 1H), 4.69 (s, 2H), 1.31 (s, 9H)

### Step 2: Synthesis of N-(3-fluoro-4-methanesulfonylaminobenzyl)-(3-tert-butylphenoxy) acetamide

To a suspension of 3-fluoro-4-methanesulfonylaminobenzylamine hydrochloride (100 mg, 0.39 mmol) in THF (5 mL) was added TEA (82 µL, 0.59 mmol). The mixture was stirred for 5 minutes, to which were added 3-tert-butylphenoxyacetic acid (87 mg, 0.42 mmol) and DMTMM (120 mg, 0.43 mmol). The mixture was stirred overnight at room temperature and was concentrated under reduced pressure. The residue was diluted with EtOAc and water. The organic layer was washed with 3N HCl, saturated sodium bicarbonate, and brine, dried over anhydrous magnesium sulfate, filtered, and concentrated under reduced pressure. The crude residue was purified by recrystallization from EtOAc/hexane to afford the title compound (136 mg, 77.7%) as a white solid.
Mp: 126 ~ 127°C; ¹H NMR (300 MHz, CDCl₃): δ 7.53 (t, 1H, *J* = 8.1 Hz), 7.29 ~ 7.23 (m, 1 H), 7.11 ∼ 6.98 (m, 4H), 6.96 (t, 1 H, *J* = 2.4 Hz), 6.74 ~ 6.70 (m, 1H), 6.50 (br s, 1 H), 4.58 (s, 2H), 4.53 (d, 2H, *J* = 6 Hz), 3.02 (s, 3H), 1.30 (s, 9H)

### Example 41: N-(3-Fluoro-4-methanesulfonylaminobenzyl)-2-(4-n-propoxyphenoxy)acetamide

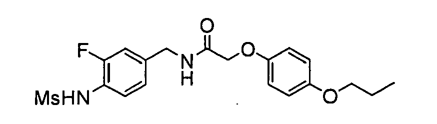

### Step 1: Synthesis of 4-propoxyphenoxyacetic acid

To a solution of 4-propoxyphenol (0.42 g, 2.76 mmol) in acetonitrile (10 mL) were added cesium carbonate (1.37 g, 4.2 mmol) and ethyl bromoacetate (0.34 mL, 3.01 mmol). The resulting mixture was stirred for 1.5 hours at room temperature, concentrated under reduced pressure, and diluted with EtOAc and water. The organic layer was washed with brine and concentrated under reduced pressure. The crude residue was diluted with THF (7 mL) and 1 N LiOH (5 mL), and then stirred for 1 hour at room temperature. The mixture was acidified with 6 N HCl, concentrated under reduced pressure, and diluted with EtOAc and water. The organic layer was washed with brine, dried over anhydrous magnesium sulfate, filtered, and concentrated under reduced pressure. The crude residue was then purified by recrystallization from EtOAc/hexane to afford the title compound (0.48 g, 82.7%) as a white solid.
¹H NMR (300MHz, CDCl₃): δ 6.90 ∼ 6.86 (m, 4H), 4.63 (s, 2H), 3.87 (t, 2H, *J* = 6.6 Hz), 1.79 (tq, 2H, *J* = 7.5, 6.6 Hz), 1.02 (t, 3H, *J* = 7.5 Hz)

### Step 2: Synthesis of N-(3-fluoro-4-methanesulfonylaminobenzyl)-(4-propoxyphenoxy) acetamide

To a suspension of 3-fluoro-4-methanesulfonylaminobenzylamine hydrochloride (100 mg, 0.39 mmol) in THF (5 mL) was added TEA (82 µL, 0.59 mmol). The mixture was stirred for 5 minutes, to which were added 4-propoxyphenoxyacetic acid (88 mg, 0.42 mmol) and DMTMM (120 mg, 0.43 mmol). The mixture was stirred overnight at room temperature and was concentrate under reduced pressure. The residue was diluted with EtOAc and water. The organic layer was washed with 3N HCl, saturated sodium bicarbonate and brine, dried over anhydrous magnesium sulfate, filtered, and concentrated under reduced pressure. The crude residue was purified by recrystallization from EtOAc/hexane to afford the title compound (136 mg, 77.2%) as a white solid.
Mp: 148 ∼ 149°C; ¹H NMR (300MHz, CDCl₃): δ 7.52 (t, 1H, *J*= 8.4 Hz), 7.11 ~ 7.08 (m, 1 H), 7.06 (s, 1H), 7.02 ∼ 6.94 (m, 1 H), 6.84 (s, 4H), 6.52 (br s, 1 H), 4.51 (s, 2H), 4.51 (d, 2H, *J* = 6 Hz), 3.87 (t, 2H, *J* = 6.6 Hz), 3.02 (s, 3H), 1.79 (tq, 2H, *J* = 7.5, 6.6 Hz), 1.03 (t, 3H, *J* = 7.5 Hz)

### Example 42: N-(3-Fluoro-4-methanesulfonylaminobenzyl)-2-(3-chlorophenoxy)acetamide

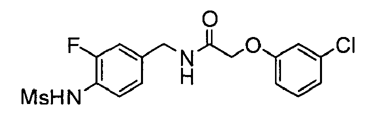

### Step 1: Synthesis of 3-chlorophenoxyacetic acid

To a solution of 3-chlorophenol (0.36 g, 2.80 mmol) in acetonitrile (10 mL) were added cesium carbonate (1.37 g, 4.2 mmol) and ethyl bromoacetate (0.34 mL, 3.01 mmol). The resulting mixture was stirred for 1.5 hours at room temperature, concentrated under reduced pressure, and diluted with EtOAc and water. The organic layer was washed with brine and concentrated under reduced pressure. The crude residue was diluted with THF (7 mL) and 1 N LiOH (5 mL), and then stirred for 1 hour at room temperature. The mixture was acidified with 6 N HCl, concentrated under reduced pressure, and diluted with EtOAc and water. The organic layer was washed with brine, dried over anhydrous magnesium sulfate, filtered, and concentrated under reduced pressure. The crude residue was then purified by recrystallization from EtOAc/hexane to afford the title compound (0.40 g, 76.6%) as a white solid.
¹H NMR (300MHz, CDCl₃): δ 7.26 ∼ 7.21 (m, 1 H), 7.03 ∼ 7.00 (m, 1 H), 6.94 (t, 1 H, *J* = 2.4 Hz), 6.84~6.80 (m, 1H), 4.69 (s, 2H)

### Step 2: Synthesis of N-(3-fluoro-4-methanesulfonylaminobenzyl)-(3-chlorophenoxy)acetamide

To a suspension of 3-fluoro-4-methanesulfonylaminobenzylamine hydrochloride (100 mg, 0.39 mmol) in THF (5 mL) was added TEA (82 µL, 0.59 mmol). The mixture was stirred for 5 minutes, to which were added 3-chlorophenoxyacetic acid (78 mg, 0.42 mmol) and DMTMM (120 mg, 0.43 mmol). The mixture stirred overnight at room temperature and was concentrated under reduced pressure. The residue was diluted with EtOAc and water. The organic layer was washed with 3N HCl, saturated sodium bicarbonate and brine, dried over anhydrous magnesium sulfate, filtered, and concentrated under reduced pressure. The crude residue was purified by recrystallization from EtOAc/hexane to afford the title compound (120 mg, 79.0%) as a white solid.
Mp: 113 ~ 114°C; ¹H NMR (300MHz, CDCl₃): δ 7.53 (t, 1H, *J* = 8.1 Hz), 7.25 (t, 1H, *J* = 8.1 Hz), 7.11 ~ 7.01 (m, 3H), 6.95 ∼ 6.86 (m, 2H), 6.82 (ddd, 1 H, *J =* 8.3, 2.4, 0.9 Hz), 6.51 (br s, 1 H), 4.55 (s, 2H), 4.52 (d, 2H, *J* = 6 Hz), 3.02 (s, 3H)

### Example 43: N-(3-Fluoro-4-methanesulfonylamino-benzyl)-2-(2-morpholin-4-yl-4-trifluoromethyl-phenoxy)-acetamide

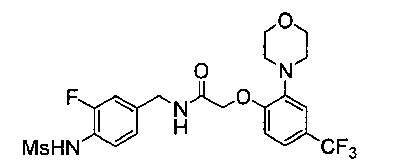

### Step 1: Synthesis of 4-trifluoromethyl-2-morpholinophenol

A mixture of 4-trifluoro-2-iodo-phenol (100 mg, 0.347 mmol), morpholine (36.29 mg, 0.416 mmol) and CsOH·H₂O (117 mg, 0.694 mmol) in DMSO (1.5 mL) were taken in a sealed tube placed in preheated oil bath at 120 °C and then held at that temperature for 3hrs. Then the reaction mixture was cooled to r.t., poured into water containing crushed ice, and stirred for 5 mins. Sat. aq NH₄Cl solution was added to this mixture, and the organic portion was extracted with EtOAc (3 × 2 mL). The combined organic extracts were washed with a sat. NH₄Cl solution and brine, and dried over anhyd Na₂SO₄. The obtained residue was column chromatographed to yield the title compound (36 mg, 42 %).
¹H NMR (300MHz, CDCl₃): δ 8.10 ~ 8.07 (m, 1H), 7.51 ~ 7.47 (m, 1H), 6.88 ∼ 6.86 (m 1 H), 3.82 ∼ 3.56 (m, 8H)

### Step 2: (2-Morpholin-4-yl-4-trifluoromethyl-phenoxy)-acetic acid ethyl ester

To a suspension of 4-trifluoromethyl-2-morpholinophenol (36 mg, 0.124 mmol) and ethyl bromoacetate (61.08 mg, 0.187 mmol) in CH₃CN (1ml) was added Cs₂CO₃ (25.05 mg, 0.149 mmol). The mixture was stirred for overnight at room temperature. The reaction mixture was diluted with EtOAc (2 ml) and then washed three times with H₂O (2 ml) and brine, dried over MgSO₄, filtered and concentrated under reduced pressure. The obtained residue was column chromatographed to yield the title compound (38 mg, 91%).
¹H NMR (300MHz, CDCl₃): δ 7.87 (d, 1 H, *J* = 2.1 Hz), 7.37 (dd. 1 H, *J* = 8.7, 2.4 Hz), 6.72 (d, 2H, *J* = 8.7 Hz), 4.72 (s, 2H), 4.27 (q, 2H, *J* = 7.2 Hz), 3.69 ∼ 3.50 (m, 8H), 1.30 (t, 3H, *J*= 7.2 Hz).

### Step 3: (2-Morpholin-4-yl-4-trifluoromethyl-phenoxy)-acetic acid

To (2-Morpholin-4-yl-4-trifluoromethyl-phenoxy)-acetic acid ethyl ester (38 mg, 0.114 mmol) in THF (1 ml) was added a solution of 0.5 N-LiOH (0.5 ml) and the mixture was stirred for 1.5 hrs at room temperature. The resulting residue was dissolved in H₂O (2 ml) and then washed three times with EtOAc (2 ml), acidified with 1 N HCl to pH 1~2. The solution was extracted three times with methylenechloride (2 ml) and then dried over anhyd. Na₂SO₄ and concentrated in vacuo to give a white solid (34 mg, 97 %).

### Step 4: N-(3-Fluoro-4-methanesulfonylamino-benzyl)-2-(2-morpholin-4-yl-4-trifluoromethyl-phenoxy)-acetamide

3-Fluoro-4-methanesulfonylaminobenzylamine hydrochloride (33.3 mg, 0.131 mmol) was suspended in THF (2mL), treated with TEA (15.9 mg) and stirred for 10mins. (2-Morpholin-4-yl-4-trifluoromethyl-phenoxy)-acetic acid (15.9 mg, 0.157 mmol) was added to the reaction mixture followed by DMTMM (39.8 mg, 0.144 mmol) after 10 mins. The resulting mixture was stirred overnight at ambient temperature and then diluted with EtOAc. The resulting solution was washed successively with water, sat'd NaHCO₃ (x3), 1 N HCl and brine, and then dried over anhyd. MgSO₄, filtered and concentrated under reduced pressure. The crude residue was recrystallized from EtOAC/n-hexane to yield the title compound (27 mg, 41 %).
Mp: 72 ∼ 75 °C; ¹H NMR (300MHz, CDCl₃): δ 7.87 (d, 1 H, *J* = 2.1 Hz), 7.53 (t, 1H, *J* = 8.4 Hz), 7.71 (dd, 1H, *J* = 8.4, 1.8 Hz), 7.31 ∼ 7.26 (m, 1 H), 7.15 (t, 1 H, *J* = 1.8 Hz), 7.12 (s, 1H), 6.81 (d, 1H, *J* = 8.4 Hz), 6.75 (s, 1H), 4.62 (s, 2H), 4.56 (d, 2H, *J* = 6.3 Hz), 3.69 (s, 8H, br), 3.02 (s, 3H).

### Example 44: 2-(2-Acetyl-4-trifluoromethyl-phenoxy)-N-(3-fluoro-4-methanesulfonylamino-benzyl)-acetamide

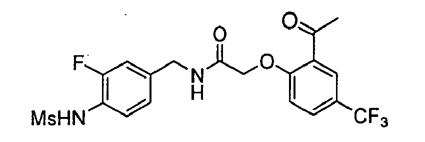

### Step 1: (2-Acetyl-4-trifluoromethyl-phenoxy)-acetic acid ethyl ester

The suspension of Pd(OAc)₂ (7.76 mg, 0.008), ethyl 4-trifluoromethyl-2-iodophenoxyacetate (98.4 mg, 0.262 mmol), DPPP (6.48 mg, 0.015 mmol) and tallium (I) acetate (76 mg, 0.289 mmol), in DMF (2 ml) was stirred for 10mins, and then butyl vinyl ether (52 mg, 0.526 mmol) was added to the mixture. The resulting mixture was stirred at 120°C overnight, and then EtOAC and 1 N HCl were added to the resulting residue. The aqueous phase was separated and extracted with EtOAc. The combined organic phase was dried over anhyd. MgSO₄,filtered and concentrated under reduced pressure. The obtained residue was column-chromatographed to yield the title compound (31 mg, 30%).
¹H NMR (300MHz, CDCl₃): δ 8.05 (d. 1 H, *J* = 2.4 Hz), 7.71 ∼ 7.67 (m, 1 H), 6.92 (d, 1 H, *J* = 8.7 Hz), 4.78 (s, 2H), 4.20 (q, 2H, *J* = 7.2 Hz), 2.73 (s, 3H), 1.32 (t, 3H, *J* = 7.2 Hz).

### Step 2: (2-Acetyl-4-trifluoromethyl-phenoxy)-acetic acid

To (2-Acetyl-4-trifluoromethyl-phenoxy)-acetic acid ethyl ester (31 mg, 0.106 mmol) in THF (1 ml) was added a solution of 0.5 N-LiOH (0.5 ml) and the mixture was stirred for 1.5 hrs at room temperature. The resulting residue was dissolved in H₂O (2 ml) and then washed three times with EtOAc (2 ml), acidified with 1 N HCl to pH 1~2. The solution was extracted three times with methylenechloride (2 ml) and then dried over anhyd. Na₂SO₄ and concentrated in vacuo to give a white solid (25 mg, 97 %).

### Step 3: 2-(2-Acetyl-4-trifluoromethyl-phenoxy)-N-(3-fluoro-4-methanesulfonylamino-benzyl)-acetamide

3-Fluoro-4-methanesulfonylaminobenzylamine hydrochloride (24.28 mg, 0.095 mmol) was suspended in THF (2mL), treated with TEA (11.5 mg) and stirred for 10mins. (2-acetyl-4-trifluoromethyl-phenoxy)-acetic acid (25 mg, 0.095 mmol) was added to the reaction mixture followed by DMTMM (29 mg, 0.104mmol) after 10 mins. The resulting mixture was stirred overnight at ambient temperature and then diluted with EtOAc. The resulting solution was washed successively with water, sat'd NaHCO₃ (x3), 1 N HCl and brine, and then dried over anhyd. MgSO₄, filtered and concentrated under reduced pressure. The crude residue was recrystallized from EtOAC/n-hexane to yield the title compound (35 mg, 79 %).
Mp: 67 ∼ 69 °C; ¹H NMR (300MHz, CDCl₃): δ 8.39 (s, 1H), 8.00 (d, 1H, *J* = 1.8 Hz), 7.78 (dd, 1 H, *J* = 8.4, 2.4 Hz), 7.54 ~ 7.48 (m, 1 H), 7.14 (d, 2H, *J* = 9.3 Hz), 7.05 (d, 1H, *J* = 8.7 Hz), 6.62 (s, 1 H), 4.67 (s, 2H), 4.55 (d, 2H, *J* = 9.0 Hz), 3.94 (d, 2H, *J* = 6.9 Hz), 3.00 (s, 3H), 2.63 (s, 3H).

### Example 45: 2-(4-Bromo-phenoxy)-N-(3-fluoro-4-methanesulfonylamino-benzyl)-acetamide

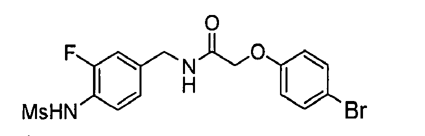

### Step 1: Synthesis of 4-bromophenoxyacetic acid

To a solution of 4-bromophenol (1.03 g, 5.95 mmol) in acetonitrile (35 mL) were added cesium carbonate (2.03 g, 6.23 mmol) and ethyl bromoacetate (0.77 mL, 6.94 mmol). The resulting mixture was stirred for 3 hours at room temperature, concentrated under reduced pressure, and diluted with EtOAc and water. The organic layer was washed with brine and concentrated under reduced pressure. The crude residue was diluted with THF (30 mL) and 1 N KOH (5 mL), and then stirred for 3 hours at room temperature. The mixture was acidified with 6 N HCI, concentrated under reduced pressure, and diluted with EtOAc and water. The organic layer was washed with brine, dried over anhydrous magnesium sulfate, filtered, and concentrated under reduced pressure. The crude residue was then purified by recrystallization from EtOAc/hexane to afford the title compound as a white solid.
¹H NMR (300MHz, CDCl₃): δ 7.34 (d, 2H, J = 8.4 Hz), 6.74 (d, 2H, J = 9Hz), 4.62 (s, 2H)

### Step 2: Synthesis of 2-(4-bromo-phenoxy)-N-(3-fluoro-4-methanesulfonylamino-benzyl)-acetamide

To a suspension of 3-fluoro-4-methanesulfonylaminobenzylamine hydrochloride (137 mg, 0.537 mmol) in THF (15 mL) was added NMP (0.3 ml). The mixture was stirred for 5 minutes, to which were added 4-bromophenoxyacetic acid (123 mg, 0.532 mmol) and DMTMM (157 mg, 0.567 mmol). The mixture was stirred overnight at room temperature and was concentrated under reduced pressure. The residue was diluted with EtOAc and water. The organic layer was washed with 3N HCl, saturated sodium bicarbonate, and brine, dried over anhydrous magnesium sulfate, filtered, and concentrated under reduced pressure. The crude residue was purified by recrystallization from EtOAc/hexane to afford the title compound (137 mg, 60%) as a white solid.
Mp: 136 ~ 137 °C; ¹H NMR (300MHz, CDCl₃): δ 7.45 (t, 1H, *J* = 8.4 Hz), 7.36 (d, 2H, *J* = 9 Hz), 7.00 (d, 2H, *J* = 9 Hz), 6.90 (m, 1 H), 6.72 (dd, 2H, *J* = 6.9, 2.1Hz), 6.70 (br s, 1 H), 4.47 (s, 2H), 4.52 (d, 2H, *J* = 6.3 Hz), 2.96 (s, 3H)

### Example 46: 2-(3-Bromo-phenoxy)-N-(3-fluoro-4-methanesulfonylamino-benzyl)-acetamide

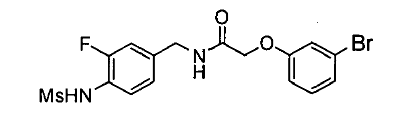

### Step 1: Synthesis of 3-bromophenoxyacetic acid

To a solution of 3-bromophenol (709 mg, 4.09 mmol) in acetonitrile (35 mL) were added cesium carbonate (2.01 g, 6.17 mmol) and ethyl bromoacetate (0.57 mL, 5.1 mmol). The resulting mixture was stirred for 3 hours at room temperature, concentrated under reduced pressure, and diluted with EtOAc and water. The organic layer was washed with brine and concentrated under reduced pressure. The crude residue was diluted with THF (30 mL) and 1 N KOH (5 mL), and then stirred for 3 hours at room temperature. The mixture was acidified with 6 N HCl, concentrated under reduced pressure, and diluted with EtOAc and water. The organic layer was washed with brine, dried over anhydrous magnesium sulfate, filtered, and concentrated under reduced pressure to yield the crude title compound as a white solid.
¹H NMR (300MHz, CDCl₃): δ 7.21 (m, 1 H), 7.04 (m, 1H), 6.82 (m, 1 H), 6.74 (d, 2H, J = 9Hz), 4.63 (s, 2H)

### Step 2: Synthesis of 2-(3-bromo-phenoxy)-N-(3-fluoro-4-methanesulfonylamino-benzyl)-acetamide.

To a suspension of 3-fluoro-4-methanesulfonylaminobenzylamine hydrochloride (79.3 mg, 0.311 mmol) in THF (15 mL) was added NMP (0.2 ml). The mixture was stirred for 5 minutes, to which were added 3-bromophenoxyacetic acid (73 mg, 0.32 mmol) and DMTMM (85.1 mg, 0.307 mmol). The mixture was stirred overnight at room temperature and was concentrated under reduced pressure. The residue was diluted with EtOAc and water. The organic layer was washed with 3N HCl, saturated sodium bicarbonate, and brine, dried over anhydrous magnesium sulfate, filtered, and concentrated under reduced pressure. The crude residue was purified by recrystallization from EtOAc/hexane to afford the title compound (98 mg, 73%) as a white solid.
Mp: 104 ~ 106 °C; ¹H NMR (300MHz, CDCl₃): δ 7.43 (t, 1H, *J* = 8.4 Hz), 7.12 (m, 2H), 7.04 ~ 6.98 (m, 2H), 6.94 (m, 1 H), 6.82 - 6.79 (m, 2H), 4.48 (s, 2H), 4.46 (d, 2H, *J =* 6.3 Hz), 2.95 (s, 3H)

### Example 47: 2-[4-(tert-butyl)phenoxy]-N-(3-cyano-5-fluoro-4-methanesulfonyl Amino-benzyl)acetamide

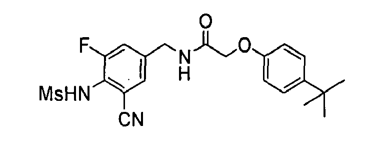

### Step 1: 4-amino-3-fluorobenzenecarbonitrile

2-Fluoro-2-iodoaniline (2.0g, 8.4mmol) and coppercyanide (982mg, 11.0mmol, 1.5eq) was added into DMF(30ml). A reaction mixture was heated to 130°C, stirred for 8hrs. A reaction mixture was diluted with ethyl acetate. A diluted solution was washed with H₂O (2 times) and brine, and then dried with MgSO₄. A residue was purified with column chromatography (n-Hexane: EtOAc =3 : 1) to yield a solid (914mg, 84%).
¹H NMR (300 MHz, CDCl₃): 7.21-7.26 (m, 2H), 6.74 (t, 1H, *J*= 8.3 Hz), 4.20 (bs, 2H)

### Step 2: 4-amino-3-fluoro-5-iodobenzenecarbonitrile

4-amino-3-fluorobenzenecarbonitrile (4.06g, 29.9mmol) and ICI(1.0M in methylene chloride, 40.0 ml, 40.0mmol) was added into methylene chloride (100ml). A reaction mixture was stirred for 20 hrs. A reaction was quenched by adding sodium thiosulfate solution. A aqueous solution was extracted with MC. A combined organic solution was washed with H₂O and brine, dried with Na₂SO₄, concentrated in vacuo. A residue was purified with column chromatography (n-Hexane: EtOAc = 5: 1) to yield a solid (4.80g, 91%).
¹H-NMR (300MHz, CDCl₃): δ 7.69 (t, 1 H, *J* = 1.5 Hz), 7.23 (dd, 1 H, *J* = 10.0, 1.7 Hz), 4.68 (bs, 2H)

### Step 3: 4-aminomethyl-2-fluoro-6-iodophenylamine

A 50 ml two-neck round bottom flask was filled with argon gas and the solution of 4-amino-3-fluoro-5-iodo-benzonitrile 84.4mg(0.322mmol, 1*eq.*) in tetrahydrofuran was put into the flask and then cooled to 0°C. To the solution was added Borane-THF complex solution (1.0M solution in THF, 0.64ml, 0.644mmol, 2*eq.*). The temperature of reaction mixture was raised to room temperature, heated, and refluxed. After confirming the completion of the reaction with TLC, to the solution was added 5% HCl and stirred for 20 minutes. The resulting solution was basified using 1 N KOH, extracted with ether, washed with brine and dried over Na₂SO₄. The obtained liquid was concentrated under reduced pressure to yield a pale yellow solid (78.4mg, 80.5%).
¹H NMR (400MHz, CD₃OD) : 7.33 (s, 1 H), 6.93 (dd, 1 H, *J*=11.6, 2.0Hz), 3.58 (s, 2H),

### Step 4 : (4-amino-3-fluoro-5-iodobenzyl)carbamic acid t-butyl ester

A 25 ml two-neck round bottom flask was filled with argon gas and the solution of 4-aminomethyl-2-fluoro-6-iodophenylamine (31.9mg, 0.120mmol, 1 *eq.*) and triethylamine (18.4µℓ, 0.132mmol, 1.1*eq.*) in methylenechloride was put into the flask and then cooled to 0°C. To the solution were added 4-dimethylaminopyridine (1.47mg, 0.012mmol, 0.1*eq.*) and di-t-butyl dicarbonate (27.6µℓ, 0.120mmol, 1 eq.) and stirred for 5 hours. After confirming the completion of the reaction with TLC, the resulting solution was extracted with methylenechloride, washed with water and brine, dried over Na₂SO₄ and concentrated under reduced pressure. The obtained liquid was column-chromatographed (n-hexane /ethyl acetate = 5/1) to yield a yellow liquid(9.8mg, 22.3%).
¹H NMR (400 MHz, CDCl₃): 7.87 (s, 0.2H), 7.44 (d, 0.2H, *J*=11.2Hz), 7.26 (s, 1H), 6.87 (d, 1H, *J*=11.2Hz), 4.72 (bs, 2H), 4.08 (d, 2H, *J*=4.4Hz), 1.39 (s, 9H)

### Step 5 : (3-fluoro-5-iodo-4-methanesulfonylamino)carbamic acid t-butyl ester

A 25 ml two-neck round bottom flask was filled with argon gas and the solution of (4-amino-3-fluoro-5-iodobenzyl)carbamic acid t-butyl ester (5.8g, 17.2mmol, 1*eq.*) and pyridine (1,65ml, 20.6mmol, 1.2eq.) in methylene chloride (70ml) was put into the flask and then cooled to 0°C. To the solution was added methanesulfonyl chloride (1.73ml, 22.4mmol, 1.3eq.) and heated to reflux for one night. After confirming the completion of the reaction with TLC, the reaction solution was acidified by 10% HCl, extracted with ethylacetate, washed with water and brine, dried over MgSO₄ and evaporated. The obtained solid was column-chromatographed (n-hexane /ethyl acetate = 2/1) to yield a yellow liquid (5.55g, 73%).
¹H-NMR (300MHz, CDCl₃): δ 7.56 (s, 1 H), 7.08 (d, 1 H, *J =* 10.0 Hz), 6.12 (s, 1 H), 4.92 (bs, 1 H), 4.24 (d, 2H, *J =* 5.9 Hz), 3.23 (s, 3H), 1.44 (s, 9H)

### Step 6: (3-cyano-5-fluoro-4-methanesulfonylamino)carbamic acid t-butyl ester

(3-fluoro-5-iodo-4-methanesulfonylamino)carbamic acid t-butyl ester (156 mg, 0.35mmol) and copper cyanide (36mg, 0.42mmol, 1.2eq) were added into DMF(3ml). A reaction mixture was heated to 130°C and stirred for 3hrs. A reaction mixture was diluted with ethyl acetate. A diluted solution was washed with H₂O (2 times) and brine, and then dried with MgSO₄. A residue was purified with column chromatography (n-Hexane: EtOAc =3 : 1) to yield a solid (42mg, 35%).
¹H-NMR (300MHz, CDCl₃): δ 7.34-7.40 (m, 2H), 6.48 (s, 1H), 5.01 (bs, 1H), 4.31 (d, 2H, *J* = 6.4 Hz), 3.28 (s, 3H), 1.44 (s, 9H)

### Step 7: N-(4-aminomethyl-2-cyano-6-fluorophenyl)methanesulfonamide

(3-cyano-5-fluoro-4-methanesulfonylamino)carbamic acid t-butyl ester (30mg, 0.12mmol) was put into 25ml round-bottom flask and dissolved in methylenechloride(3ml). To the solution was added trifluoroacetic acid (0.5ml) and stirred for one night. After confirming the completion of the reaction with TLC, the reaction solution was concentrated under reduced pressure to yield a brown crude liquid (crude 100%).

### Step 8: 2-[4-(tert-butyl)phenoxy]-N-3-(cyano-5-fluoro-4-methanesulfonylaminobenzyl)acetamide

A 25 ml two-neck round bottom flask was filled with argon gas and the solution of 2-[4-(*tert*-butyl)phenoxy]acetic acid (33mg, 0.16mmol, 1.2 *eq.*) in THF was put into the flask. To the solution were added DMTMM (86mg, 0.31 mmol, 2.4 eq) and NMM (20.0µℓ, 0.18mmol, 1.4 *eq.*) and stirred at room temperature for 12 hours. The resulting solution was cooled to 0°C and the solution of 3-cyano-5-fluoro-4-methanesulfonylamino-ammonium, trifluoroacetate (32mg, 0.13mmol, 1*eq.*) in THF was added. The mixture solution was stirred at room temperature for one night. After confirming the completion of the reaction with TLC, methylenechloride was removed under reduced pressure. The remain liquid was column-chromatographed (n-hexane /ethyl acetate = 1/1 (only ethylacetate)) to yield a white solid (37mg, 66%).
Mass (FAB) 434 [M+H]+
IR(KBr pellet, cm⁻¹) : 3433, 2960, 1655, 1548, 1513, 1434, 1333, 1246 ;
¹H NMR (300 MHz, CDCl₃) : 7.40(s, 1H), 7.32∼7.36(m, 3H), 7.089(bs, 1H), 6.85(d, 2H, *J*=9.0Hz), 6.41 (s, 1 H), 4.56(s, 2H), 4.53(d, 2H, *J*=6.2Hz), 3.29( s, 3H), 1.28(s, 9H)

### Example 48: 2-(4-tert-Butylphenoxy)-2-fluoro-N-(3-fluoro-4-methanesulfonylaminobenzyl)acetamide

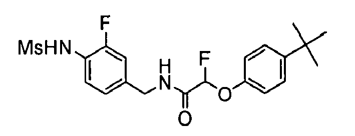

N-(4-Aminomethyl-2-fluorophenyl)methanesulfonamide HCl salt (65.64mg, 0.32mmol) and (4-tert-butylphenoxy)fluoroacetic acid (1.1eq, 0.35mmol, 80.00mg) were added in anhydrous DMF under argon atmosphere. TEA (5eq, 1.60mmol, 222.00µℓ) and diethylcyanophosphonate (1.2eq, 0.38mmol, 58.00µℓ) were added into the mixture. The reaction mixture was stirred for 12 hrs at room temperature. The reaction mixture was extracted with ethylacetate (2 times), washed with brine, dried with Na₂SO₄, and then concentrated in vacuo. The residue was purified with column chromatography (n-Hx: EA= 2: 1) to yield the title compound (35 mg, 46%) as a yellow solid.
IR (NaCl neat, cm⁻¹): 3267, 2962, 1684, 1333, 1219, 1158, 1108, 1028; ¹H NMR (400MHz, CDCl₃):): 1.23 (s, 9H), 2.94 (s, 3H), 4.44 (dd, 2H *J*=4.8, 60.8Hz), 6.76 (s, 1H), 6.98 (d, 2H, *J*=8.8Hz), 4.03 (d, 2H, *J*=8.0Hz), 7.28 (d, 1H, *J*=8.4Hz), 7.46 (dd, 1H, *J*=8.4Hz)

### Example 49: 2-(4-tert-Butylphenoxy)-N-(4-methanesulfonylamino-3-trifluoromethylbenzyl)acetamide

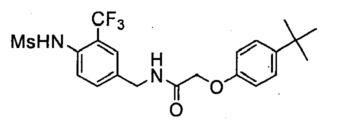

N-(4-Aminomethyl-2-trifluoromethylphenyl)methanesulfonamide (210mg, 0.80mmol) and (4-tert-butylphenoxy)acetic acid (1.1eq, 0.88mmol, 183.26mg) were added in anhydrous DMF under argon atmosphere. TEA (5eq, 4.00mmol, 557.02µℓ) and diethylcyanophosphonate (1.2eq, 0.96mmol, 145.66µℓ) were added into the mixture. The reaction mixture was stirred for 12 hrs at room temperature. The reaction mixture was extracted with ethylacetate (2 times), washed with brine, dried with Na₂SO₄, and then concentrated in vacuo. The residue was purified with column chromatography (n-Hx: EA= 1: 1) to yield the title compound (191 mg, 52%) as a white solid.
Mp: 194~196°C; IR (KBr pellet, cm⁻¹): 3400, 3022, 1664, 1535, 1371, 1323, 1216, 1160, 1057, ¹H NMR (400MHz, CDCl₃): 1.23 (s, 9H), 3.40 (s, 3H), 4.51 (d, 2H, , *J*=4.0Hz), 4.57 (d, 2H, *J*=4.0Hz), 6.80 (d, 2H, , *J*=6.8Hz), 7.27 (d, 2H, *J*=8.8Hz), 7.34 (d, 1 H*J*=8.4Hz), 7.53∼7.44 (m, 1H), 6.54 (s, 1H).

### Example 50: 2-(4-tert-Butylphenoxy)-N-(3-chloro-5-fluoro-4-methanesulfonylaminobenzyl)acetamide

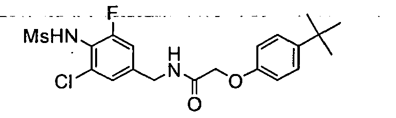

(4-*tert*-Butyl-phenoxy)acetic acid (1.1eq, 0.11mmol, 34.25mg) and N-(4-aminomethyl-2-chloro-6-fluorophenyl)methanesulfonamide (1eq, 0.10mmol, 25mg) were added in anhydrous DMF under argon atmosphere. TEA (2eq, 0.20mmol, 18.22µℓ) and diethylcyanophosphonate (1.2eq, 0.12mmol, 27.88µℓ) were added into the mixture. The reaction mixture was stirred for 12 hrs at room temperature. The reaction mixture was extracted with ethylacetate (2 times), washed with brine, dried with Na₂SO₄, and then concentrated in vacuo. The residue was purified with column chromatography (n-Hx: EA= 1: 1) to yield the title compound (19 mg, 42%) as a white solid.
¹H NMR (400MHz, CDCl₃) δ 1.22 (s, 9H), 3.43 (s, 3H), 4.48 (d, *J*=6.4Hz H), 4.51 (s, 2H), 6.80 (d, J=8.4Hz, 2H), 6.97 (s, 1 H), 7.02 (d, J=9.6Hz, 1 H), 7.28 (d, J=8.8Hz, 2H); IR (NaCI Neat, cm⁻¹): 3406, 2960, 2359, 1492.

### Example 51: 2-(2-trifluoromethyl-phenoxy)-N-(3-fluoro-4-methanesulfonylaminobenzyl)-acetamide

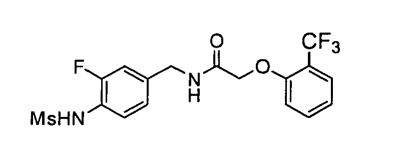

### Step 1: Synthesis of 2-trifluoromethyl phenoxyacetic acid

To a solution of 2-trifluoromethylphenol (100 mg, 0.62 mmol) in tetrahydrofuran (4 mL) and ethyl bromoacetate (0.094 mL, 0.62 mmol) was added sodium hydride (60% in mineral oil, 37 mg, 0.93 mmol) in tetrahydrofuran (2 mL). The resulting mixture was stirred for 3 hours at room temperature, acidified with aqueous NH₄Cl (1 mL), concentrated under reduced pressure, and diluted with EtOAc and water. The organic layer was washed with brine and concentrated under reduced pressure. The crude residue and LiOH (37 mg, 0.92 mmol) were diluted with THF/H₂O (1: 1, 5 mL), and then stirred for 3 hours at room temperature. The mixture was acidified with 1 N HCl, concentrated under reduced pressure, and diluted with CH₂Cl₂ and water. The organic layer was washed with brine, dried over anhydrous magnesium sulfate, filtered, and concentrated under reduced pressure to yield the crude title compound as a white solid. ¹H NMR (300MHz, CDCl₃): δ 7.60 (d, 1 H, *J* = 8.3Hz), 7.49 (t, 1 H, *J* = 7.1 Hz), 7.08 (t, 1H, *J* = 7.4Hz), 6.90 (d, 2H, *J* = 8.4Hz), 4.69 (s, 2H)

### Step 2: Synthesis of 2-(2-trifluoromethyl-phenoxy)-N-(3-fluoro-4-methanesulfonylamino-benzyl)-acetamide.

To a suspension of 3-fluoro-4-methanesulfonylaminobenzylamine hydrochloride (58.1 mg, 0.26 mmol) in THF (10 mL) was added NMM (37.5 mg, 0.37 mmol). The mixture was stirred for 5 minutes, to which were added 2-trifluoromethyl phenoxyacetic acid (70 mg, 0.32 mmol) and DMTMM (175 mg, 0.64 mmol). The mixture was stirred overnight at room temperature and was concentrated under reduced pressure. The residue was diluted with EtOAc and water. The organic layer was washed with 3N HCl, saturated sodium bicarbonate and brine, dried over anhydrous magnesium sulfate, filtered and concentrated under reduced pressure. The crude residue was purified with column chromatography (n-Hexane: EtOAc =1:2) to afford the title compound (45 mg, 42%) as a white solid.
Mass (FAB) 421 [M+H]⁺
¹H NMR (300MHz, CDCl₃): δ 7.60 (d, 1H, *J* = 7.7 Hz), 7.54 (t, 1H, *J* =7.7 Hz), 7.52 (t, 1H, *J* = 8.2 Hz), 7.13 ∼ 7.09 (m, 2H), 7.06 (s, 1 H), 6.96 (d, 1 H, *J* = 8.4 Hz), 6.52 (s, 1 H), 4.61 (s, 2H), 4.51 (d, 2H, *J* = 6.1 Hz), 2.99 (s, 3H)

### Example 52: N-(3-Fluoro-4-methanesulfonylaminobenzyl)-2-(4-isopropylphenoxy)acetamide

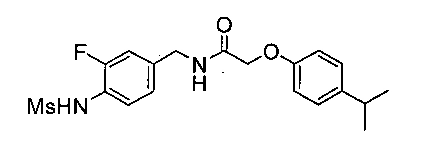

### Step 1: Synthesis of 4-isopropylphenoxyacetic acid

To a solution of 4-isopropylphenol (0.38 g, 2.79 mmol) in acetonitrile (10 mL) were added cesium carbonate (1.37 g, 4.2 mmol) and ethyl bromoacetate (0.34 mL, 3.01 mmol). The resulting mixture was stirred for 1.5 hours at room temperature, concentrated under reduced pressure, and diluted with EtOAc and water. The organic layer was washed with brine and concentrated under reduced pressure. The crude residue was diluted with THF (7 mL) and 1 N LiOH (5 mL), and then stirred for 1 hour at room temperature. The mixture was acidified with 6 N HCl, concentrated under reduced pressure, and diluted with EtOAc and water. The organic layer was washed with brine, dried over anhydrous magnesium sulfate, filtered, and concentrated under reduced pressure. The crude residue was then purified by recrystallization from EtOAc/hexane to afford 0.35 g (64.6%) as a white solid.
¹H NMR (300 MHz, CDCl₃): δ 7.17 (d, 2 H, *J* = 8.7 Hz), 6.86 (d, 2 H, *J* = 8.7 Hz), 4.66 (s, 2 H), 2.87 (sept, 1 H, *J* = 7.2 Hz), 1.22 (d, 6 H, *J* = 7.2 Hz)

### Step 2: Synthesis of N-(3-fluoro-4-methanesulfonylaminobenzyl)-2-(4-isopropylphenoxy)acetamide

To a suspension of 3-fluoro-4-methanesulfonylaminobenzylamine hydrochloride (100 mg, 0.39 mmol) in THF (5 mL) was added triethylamine (82 µL, 0.59 mmol). The mixture was stirred for 5 minutes, to which were added 4-isopropylphenoxyacetic acid (81 mg, 0.42 mmol) and 4-(4,6-dimethoxy[1,3,5]triazin-2-yl)-4-methylmorpholinium chloride hydrate (DMTMM, 120 mg, 0.43 mmol). The mixture was stirred overnight at room temperature and was concentrated under reduced pressure. The residue was diluted with EtOAc and water. The organic layer was washed with 3N HCl, saturated sodium bicarbonate and brine, dried over anhydrous magnesium sulfate, filtered and concentrated under reduced pressure. The crude residue was purified by recrystallization from EtOAc/hexane to afford 110 mg (71.0%) as a white solid.
Melting point: 118 ~ 119.5°C; ¹H NMR (300 MHz, CDCl₃): δ 7.52 (t, 1 H, *J* = 8.1 Hz), 7.18 (d, 2 H, *J* = 8.4 Hz), 7.10 ∼ 7.05 (m, 2 H), 6.97 (br, 1 H), 6.85 (d, 2 H, *J* = 8.7 Hz), 6.48 (br s, 1 H), 4.55 (s, 2 H), 4.52 (d, 2 H, *J* = 6 Hz), 3.02 (s, 3 H), 2.88 (sept, 1 H, *J* = 7.2 Hz), 1.23 (d, 6 H, *J* = 7.2 Hz)

### Example 53: N-(3-Fluoro-4-methanesulfonylaminobenzyl)-2-(4-sec-butylphenoxy)acetamide

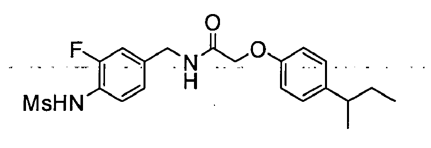

### Step 1: Synthesis of 4-sec-butylphenoxyacetic acid

To a solution of 4-sec-butylphenol (0.42 g, 2.80 mmol) in acetonitrile (10 mL) were added cesium carbonate (1.37 g, 4.2 mmol) and ethyl bromoacetate (0.34 mL, 3.01 mmol). The resulting mixture was stirred for 1.5 hours at room temperature, concentrated under reduced pressure, and diluted with EtOAc and water. The organic layer was washed with brine and concentrated under reduced pressure. The crude residue was diluted with THF (7 mL) and 1 N LiOH (5 mL), and then stirred for 1 hour at room temperature. The mixture was acidified with 6 N HCl, concentrated under reduced pressure, and diluted with EtOAc and water. The organic layer was washed with brine, dried over anhydrous magnesium sulfate, filtered, and concentrated under reduced pressure. The crude residue was then purified by recrystallization from EtOAc/hexane to afford 0.18 g (30.9%) as a white solid.
¹H NMR (300 MHz, CDCl₃): δ 7.12 (d, 2 H, *J* = 8.7 Hz), 6.86 (d, 2 H, *J* = 8.7 Hz), 4.66 (s, 2 H), 2.56 (sext, 1 H, *J* = 6.9 Hz), 1.56 (dq, 2 H, *J* = 7.5 Hz), 1.21 (d, 3 H, *J* = 6.9 Hz), 0.81 (t, 3 H, *J* = 7.5 Hz)

### Step 2: Synthesis of N-(3-fluoro-4-methanesulfonylaminobenzyl)-2-(4-sec-butylphenoxy) acetamide

To a suspension of 3-fluoro-4-methanesulfonylaminobenzylamine hydrochloride (100 mg, 0.39 mmol) in THF (5 mL) was added triethylamine (82 µL, 0.59 mmol). The mixture was stirred for 5 minutes, to which were added 4-sec-butylphenoxyacetic acid (87 mg, 0.42 mmol) and 4-(4,6-dimethoxy[1,3,5]triazin-2-yl)-4-methylmorpholinium chloride hydrate (DMTMM, 120 mg, 0.43 mmol). The mixture was stirred overnight at room temperature and was concentrated under reduced pressure. The residue was diluted with EtOAc and water. The organic layer was washed with 3N HCl, saturated sodium bicarbonate and brine, dried over anhydrous magnesium sulfate, filtered and concentrated under reduced pressure. The crude residue was purified by recrystallization from EtOAc/hexane to afford 120 mg (74.8%) as a white solid.
Melting point: 100 ~ 101 °C; ¹H NMR (300 MHz, CDCl₃): δ 7.52 (t, 1 H, *J* = 7.8 Hz), 7.13 (d, 2 H, *J* = 8.7 Hz), 7.10 ∼ 7.05 (m, 2 H), 6.99 (br, 1 H), 6.85 (d, 2 H, *J* = 8.7 Hz), 6.51 (br s, 1 H), 4.55 (s, 2 H), 4.52 (d, 2 H, *J* = 6.3 Hz), 3.02 (s, 3 H), 2.56 (sext, 1 H, *J* = 6.9 Hz), 1.56 (ddq, 2 H, *J* = 7.5, 1.5 Hz), 1.20 (d, 3 H, *J* = 6.6 Hz), 0.80 (t, 3 H, *J* = 7.5 Hz)

### Example 54: N-(3-Fluoro-4-methanesulfonylaminobenzyl)-2-(3,4-difluorophenoxy)acetamide

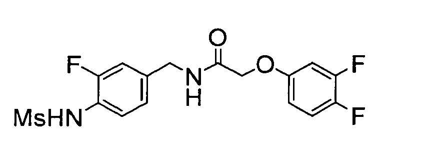

### Step 1: Synthesis of 3,4-difluorophenoxyacetic acid

To a solution of 3,4-difluorophenol (0.37 g, 2.84 mmol) in acetonitrile (10 mL) were added cesium carbonate (1.37 g, 4.2 mmol) and ethyl bromoacetate (0.34 mL, 3.01 mmol). The resulting mixture was stirred for 1.5 hours at room temperature, concentrated under reduced pressure, and diluted with EtOAc and water. The organic layer was washed with brine and concentrated under reduced pressure. The crude residue was diluted with THF (7 mL) and 1 N LiOH (5 mL), and then stirred for 1 hour at room temperature. The mixture was acidified with 6 N HCl, concentrated under reduced pressure, and diluted with EtOAc and water. The organic layer was washed with brine, dried over anhydrous magnesium sulfate, filtered, and concentrated under reduced pressure. The crude residue was then purified by recrystallization from EtOAc/hexane to afford 0.43 g (80.4%) as a white solid.
¹H NMR (300 MHz, CDCl₃): δ 7.10 (dd, 1 H, *J* = 9 Hz), 6.81 ~ 6.74 (m, 1 H), 6.66 ∼ 6.60 (m, 1 H), 4.66 (s, 2 H)

### Step 2: Synthesis of N-(3-fluoro-4-methanesulfonylaminobenzyl)-2-(3,4-difluorophenoxy) acetamide

To a suspension of 3-fluoro-4-methanesulfonylaminobenzylamine hydrochloride (100 mg, 0.39 mmol) in THF (5 mL) was added triethylamine (82 µL, 0.59 mmol). The mixture was stirred for 5 minutes, to which were added 3,4-difluorophenoxyacetic acid (79 mg, 0.42 mmol) and 4-(4,6-dimethoxy[1,3,5]triazin-2-yl)-4-methylmorpholinium chloride hydrate (DMTMM, 120 mg, 0.43 mmol). The mixture was stirred overnight at room temperature and was concentrated under reduced pressure. The residue was diluted with EtOAc and water. The organic layer was washed with 3N HCl, saturated sodium bicarbonate, and brine, dried over anhydrous magnesium sulfate, filtered, and concentrated under reduced pressure. The crude residue was purified by recrystallization from EtOAc/hexane to afford 88 mg (57.7%) as a white solid.
Melting point: 101 ~ 103°C; 1H NMR (300 MHz, CDCl₃): δ 7.53 (t, 1 H, *J* = 8.1 Hz), 7.16 ∼ 7.07 (m, 3 H), 6.88 (br, 1 H), 6.80 ∼ 6.73 (m, 1 H), 6.66 ∼ 6.61 (m, 1 H), 6.54 (br, 1 H), 4.53 (s, 1 H), 4.51 (s, 3 H), 3.03 (s, 3 H)

### Example 55: N-(3-Fluoro-4-methanesulfonylaminobenzyl)-2-(2, 4-di-tert-butylphenoxy)acetamide

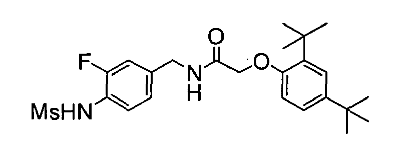

### Step 1: Synthesis of 2,4-di-tert-butylphenoxyacetic acid

To a solution of 2,4-di-*tert*-butylphenol (0.58 g, 2.81 mmol) in acetonitrile (10 mL) were added cesium carbonate (1.37 g, 4.2 mmol) and ethyl bromoacetate (0.34 mL, 3.01 mmol). The resulting mixture was stirred for 1.5 hours at room temperature, concentrated under reduced pressure, and diluted with EtOAc and water. The organic layer was washed with brine and concentrated under reduced pressure. The crude residue was diluted with THF (7 mL) and 1 N LiOH (5 mL), and then stirred for 1 hour at room temperature. The mixture was acidified with 6 N HCl, concentrated under reduced pressure, and diluted with EtOAc and water. The organic layer was washed with brine, dried over anhydrous magnesium sulfate, filtered, and concentrated under reduced pressure. The crude residue was then purified by recrystallization from EtOAc/hexane to afford 0.57 g (76.7%) as a white solid.
¹H NMR (300 MHz, CDCl₃): δ 7.36 (d, 1 H, *J* = 2.4 Hz), 7.17 (dd, 1 H, *J* = 8.4, 2.4 Hz), 6.68 (d, 1 H, *J* = 8.4 Hz), 4.69 (s, 2 H), 1.42 (s, 9 H), 1.30 (s, 9 H)

### Step 2: Synthesis of N-(3-fluoro-4-methanesulfonylaminobenzyl)-2-(2,4-di-tert-butylphenoxy)acetamide

To a suspension of 3-fluoro-4-methanesulfonylaminobenzylamine hydrochloride (100 mg, 0.39 mmol) in THF (5 mL) was added triethylamine (82 µL, 0.59 mmol). The mixture was stirred for 5 minutes, to which were added 2,4-di-tert-butylphenoxyacetic acid (111 mg, 0.42 mmol) and 4-(4,6-dimethoxy[1,3,5]triazin-2-yl)-4-methylmorpholinium chloride hydrate (DMTMM, 120 mg, 0.43 mmol). The mixture was stirred overnight at room temperature and was concentrated under reduced pressure.
The residue was diluted with EtOAc and water. The organic layer was washed with 3N HCl, saturated sodium bicarbonate and brine, dried over anhydrous magnesium sulfate, filtered, and concentrated under reduced pressure. The crude residue was purified by column chromatography (EtOAc:n-hexane = 1:2) to afford 140 mg (76.8%) as a foamy solid.
Melting point: 54 ∼ 56°C; ¹H NMR (300MHz, CDCl₃): δ 7.52 (t, 1 H, *J* = 8.1 Hz), 7.36 (d, 1 H, *J* = 2.7 Hz), 7.20 (dd, 1 H, *J* = 8.4, 2.4 Hz), 7.09 ∼ 7.03 (m, 2 H), 6.92 (br t, 1 H), 6.76 (d, 1 H, *J* = 8.4 Hz), 6.71 (br s, 1 H), 4.61 (s, 2 H), 4.53 (d, 2 H, *J* = 6 Hz), 3.01 (s, 3 H), 1.39 (s, 9 H), 1.30 (s, 9 H)

### Example 56: N-(3-Fluoro-4-methanesulfonylaminobenzyl)-2-(2,4-bis(trifluoromethyl)benzyloxy)acetamide

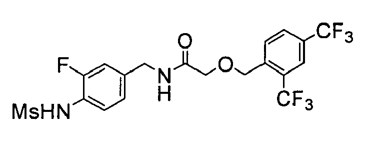

### Step 1: Synthesis of 2,4-bis(trifluoromethyl)benzyloxyacetic acid

To a solution of 2,4-bis(trifluoromethyl)benzyl alcohol (0.5 g, 2.05 mmol) in acetonitrile (10 mL) were added cesium carbonate (1.0 g, 3.07 mmol) and ethyl bromoacetate (0.25 mL, 2.25 mmol). The resulting mixture was stirred for 1.5 hours at room temperature, concentrated under reduced pressure, and diluted with EtOAc and water. The organic layer was washed with brine and concentrated under reduced pressure. The crude residue was diluted with THF (7 mL) and 1 N LiOH (5 mL), and then stirred for 1 hour at room temperature. The mixture was acidified with 6 N HCl, concentrated under reduced pressure, and diluted with EtOAc and water. The organic layer was washed with brine, dried over anhydrous magnesium sulfate, filtered, and concentrated under reduced pressure. The crude residue was then purified by column chromatography (EtOAc:n-hexane = 2:1) to afford 173 mg (28.0%) as an oil.
¹H NMR (300 MHz, CDCl₃): δ 10.63 (br, 1 H), 7.80 ∼ 7.83 (m, 3 H), 4.89 (s, 2 H), 4.30 (s, 2 H)

### Step 2: Synthesis of N-(3-fluoro-4-methanesulfonylaminobenzyl)-2-(2,4-bis(trifluoromethyl)benzyloxy) acetamide

To a suspension of 3-fluoro-4-methanesulfonylaminobenzylamine hydrochloride (145 mg, 0.57 mmol) in THF (5 mL) was added triethylamine (120 µL, 0.86 mmol). The mixture was stirred for 5 minutes, to which were added 2,4-bis(trifluoromethyl)benzyloxyacetic acid (173 mg, 0.57 mmol) and 4-(4,6-dimethoxy[1,3,5]triazin-2-yl)-4-methylmorpholinium chloride hydrate (DMTMM, 173 mg, 0.63 mmol). The mixture was stirred overnight at room temperature and was concentrated under reduced pressure. The residue was diluted with EtOAc and water.

The organic layer was washed with 3N HCl, saturated sodium bicarbonate, and brine, dried over anhydrous magnesium sulfate, filtered, and concentrated under reduced pressure. The crude residue was purified by recrystallization from EtOAc/hexane to afford 258 mg (89.7%) as a white solid.
Melting point: 123 ~ 125°C; ¹H NMR (300 MHz, CDCl₃): δ 7.94 (s, 1 H), 7.86 (d, 1 H, *J*= 8.4 Hz), 7.76 (d, 1 H, *J* = 7.8 Hz), 7.52 (t, 1 H, *J* = 8.4 Hz), 7.12 ∼ 7.06 (m, 2 H), 6.90 (br, 1H), 6.60 (br s, 1 H), 4.83 (s, 2 H), 4.48 (d, 2 H, *J* = 6.3 Hz), 4.14 (s, 2 H), 3.02 (s, 3 H)

### Example 57: N-(3-Fluoro-4-methanesulfonylaminobenzyl)-2-(4-(1,1-dimethylpropyl)phenoxy)acetamide

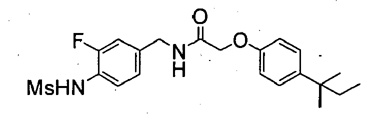

### Step 1: Synthesis of 4-(1,1-dimethylpropyl)phenoxyacetic acid

To a solution of 4-*tert*-amylphenol (0.46 g, 2.80 mmol) in acetonitrile (10 mL) were added cesium carbonate (1.37 g, 4.2 mmol) and ethyl bromoacetate (0.34 mL, 3.01 mmol). The resulting mixture was stirred for 1.5 hours at room temperature, concentrated under reduced pressure, and diluted with EtOAc and water. The organic layer was washed with brine and concentrated under reduced pressure. The crude residue was diluted with THF (7 mL) and 1 N LiOH (5 mL), and then stirred for 1 hour at room temperature. The mixture was acidified with 6 N HCl, concentrated under reduced pressure, and diluted with EtOAc and water. The organic layer was washed with brine, dried over anhydrous magnesium sulfate, filtered, and concentrated under reduced pressure. The crude residue was then purified by recrystallization from EtOAc/hexane to afford 0.57 g (91.6%) as a white solid.
¹H NMR (300 MHz, CDCl₃): δ 7.26 (d, 2 H, *J* = 8.7 Hz), 6.86 (d, 2 H, *J* = 8.7 Hz), 4.67 (s, 2 H), 1.61 (q, 2 H, *J* = 7.2 Hz), 1.25 (s, 6 H), 0.67 (t, 3 H, *J* = 7.2 Hz)

### Step 2: Synthesis of N-(3-fluoro-4-methanesulfonylaminobenzyl)-2-(4-(1,1-dimethylpropyl)phenoxy)acetamide

To a suspension of 3-fluoro-4-methanesulfonylaminobenzylamine hydrochloride (100 mg, 0.39 mmol) in THF (5 mL) was added triethylamine (82 µL, 0.59 mmol). The mixture was stirred for 5 minutes, to which were added 4-(1,1-dimethylpropyl)phenoxyacetic acid (93 mg, 0.42 mmol) and 4-(4,6-dimethoxy[1,3,5]triazin-2-yl)-4-methylmorpholinium chloride hydrate (DMTMM, 120 mg, 0.43 mmol). The mixture was stirred overnight at room temperature and was concentrated under reduced pressure. The residue was diluted with EtOAc and water. The organic layer was washed with 3N HCl, saturated sodium bicarbonate, and brine, dried over anhydrous magnesium sulfate, filtered, and concentrated under reduced pressure. The crude residue was purified by recrystallization from EtOAc/hexane to afford 143 mg (86.2%) as a white solid.
Melting point: 112 ~ 113°C; ¹H NMR (300 MHz, CDCl₃): δ 7.51 (t, 1 H, *J*= 8.1 Hz), 7.27 (d, 2 H, *J* = 9 Hz), 7.10 ~ 7.04 (m, 2 H), 7.00 (br, 1 H), 6.86 (d, 2 H, *J* = 9 Hz), 6.60 (br, 1 H), 4.56 (s, 2 H), 4.52 (d, 2 H, *J* = 6.3 Hz), 3.01 (s, 3 H), 1.61 (q, 2 H, *J* = 7.5 Hz), 1.26 (s, 6 H), 0.66 (t, 3 H, *J* = 7.5 Hz)

### Example 58: 2,2-Dimethyl-propionic acid 2-[(3-fluoro-4-methanesulfonylaminobenzylcarbamoyl)-methoxy]-5-trifluoromethyl-benzyl ester

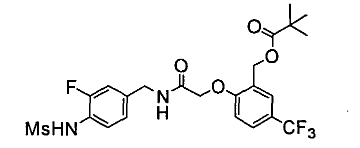

### Step 1: 2-[4-Trifluoromethylphenoxy]tetrahydro-2H-pyran

To a solution of 3,4-dihydro-2*H*-pyran (417 mg, 4.96 mmol) and HCl in dioxane (0.1 mL, 4 M) in CH₂Cl₂ (100 ml) was added dropwise 4-(trifluoromethyl)phenol (670 mg, 2.47 mol) at r.t. The reaction mixture was distinctly exothermic. After stirring overnight, the mixture was extracted with an aq solution of NaHCO₃ (20 ml) and the organic phase was dried (Na₂SO₄) and evaporated. The obtained residue was column-chromatographed to yield 2-[4-Trifluoromethylphenoxy]tetrahydro-2H-pyran (1000 mg, 98%).
¹H-NMR (300MHz, CDCl₃): δ 7.55 ∼ 7.52 (m, 2H), 7.13 (d, 2H, *J* = 9.0 Hz), 5.50 (m, 1 H), 3.90 ∼ 3.82 (m, 1 H), 3.65 ∼ 3.59 (m, 1 H), 2.04 ∼ 1.86 (m, 3H), 1.76 ~ 1.55 (m, 3H)

### Step 2: 2-(Tetrahydro-pyran-2-yloxy)-5-trifluoromethyl-benzadehyde

A 100 ml round-bottomed flask was filled with TMEDA (241 mg, 2.24 mmol) and BuLi in hexane (0.85 ml, 2.5 M, 2.29 mmol) was added to the stirred content over a period of 30 min at -10 °C. After 5 mins a molten 2-[4-Trifluoromethyl] phenoxy]tetrahydro-2*H-*pyran (400 mg, 1.62 mmol) was added dropwise at -10 °C over a period of 15 min, whereupon the lithium complex was precipitated. After 30 min, DMF (152 mg, 2.08 mol) was added. A cloudy solution was produced, which after 15 min was added dropwise over a period of 10 min to H₂O (5 ml) at a maximum temperature of 45 °C. There was a rapid evolution of gas. After stirring 10 min, the aqueous phase was separated, the mixture was extracted with EtOAc and dried over Na₂SO₄. The solvent was evaporated and the residue was column-chromatographed to yield 2-(Tetrahydro-pyran-2-yloxy)-5-trifluoromethyl-benzadehyde (379 mg, 85%).
¹H NMR (300 MHz, CDCl3): δ 10.54 (s, 1 H). 8.11 (d, 1H, *J* = 2.7 Hz), 7.75 (dd, 1H, *J* = 9.3 Hz, 2.4 Hz, 1 Harom), 7.36 (d, 1H, *J* = 9.3 Hz), 5.66 ∼ 5.65 (m, 1 H), 3.87 ~ 3.78 (m, 1 H), 3.71 ∼ 3.66 (m, 1 H), 2.13 ~ 1.92 (m, 3H), 1.82 ~ 1.62 (m, 3H).

### Step 3: [2-(Tetrahydro-pyran-2-yloxy)-5-trifluoromethyl-phenyl]-methanol

2-(Tetrahydro-pyran-2-yloxy)-5-trifluoromethyl-benzadehyde (100 mg, 0.36 mmol) was suspended in MeOH (2mL) and NaBH₄ (17 mg, 0.095 mmol) was added to the reaction mixture at 0 °C and then the resulting mixture was stirred for 90 mins at 0 °C ∼ r.t . The resulting mixture was diluted with EtOAc. The resulting solution was washed with water and then dried over anhyd. Na₂SO₄, filtered and concentrated under reduced pressure. The obtained residue was column-chromatographed to yield [2-(Tetrahydropyran-2-yloxy)-5-trifluoromethyl-phenyl]-methanol (100 mg, 99%).
¹H NMR (300 MHz, CDCl₃): δ 7.62 (d, 1 H, *J* = 1.8 Hz). 7.45 (dd, 1 H, *J* = 8.7 Hz, 2.4 Hz), 7.19 (d, 1 H, *J* = 8.7 Hz), 5.52 ∼ 5.50 (m, 1 H), 4.75 (d, 2H, *J* = 2.7 Hz), 3.86 ∼ 3.78 (m, 1 H), 3.66 ∼ 3.59 (m, 1 H), 2.13 ~ 1.82 (m, 3H), 1.78 ~ 1.54 (m, 3H).

### Step 4: (2-Hydroxymethyl-4-trifluoromethyl-phenoxy)-acetic acid ethyl ester

[2-(Tetrahydro-pyran-2-yloxy)-5-trifluoromethyl-phenyl]-methanol was suspended in HCl/dioxane (2 ml, 8 mmol, 4M) and then the resulting mixture was stirred for 12hrs at 0 °C ∼ r.t. The resulting mixture was diluted with EtOAc. The resulting solution was washed with water and then dried over anhyd. Na₂SO₄. filtered and concentrated under reduced pressure. To the obtained residue and bromoethylacetate (57 mg, 0.34 mmol) in CH₃CN (1ml) was added Cs₂CO₃ (122 mg, 0.37 mmol). The mixture was stirred for overnight at room temperature. The reaction mixture was diluted with EtOAc (2 ml) and then washed three times with H₂O (2 ml) and brine, dried over MgSO₄, filtered and concentrated under reduced pressure. The obtained residue was column-chromatographed to yield the (2-Hydroxymethyl-4-trifluoromethyl-phenoxy)-acetic acid ethyl ester (60 mg, 27 %).
¹H NMR (300 MHz, CDCl3): δ 7.61 (d, 1 H, *J* = 2.1 Hz). 7.52 (dd, 1 H, *J* = 8.4 Hz, 1.8 Hz), 6.84 (d, 1H, *J* = 8.4 Hz), 4.76 (s, 2H), 4.74 (s, 2H), 4.27 (q, 2H, d = 7.2 Hz), 1.30 (t, 3H, *J* = 2.1 Hz), 1.27 ~ 1.20 (m, 9H).

### Step 5: 2,2-Dimethyl-propionic acid 2-ethoxycarbonylmethoxy-5-trifluoromethyl-benzyl ester

(2-Hydroxymethyl-4-trifluoromethyl-phenoxy)-acetic acid ethyl ester (22 mg, 0.079 mmol) was suspended in pyridine (0.5 mL) and pivaloyl chloride (12 mg, 0.095 mmol) was added to the reaction mixture at 0 °C and then the resulting mixture was stirred for 60 mins at 0 °C. The resulting mixture was diluted with EtOAc. The resulting solution was washed with water and then dried over anhyd. Na₂SO₄, filtered and concentrated under reduced pressure. The obtained residue was column-chromatographed to yield 2,2-dimethyl-propionic acid 2-ethoxycarbonylmethoxy-5-trifluoromethyl-benzyl ester (21 mg, 87 %).

Step 6. 2,2-Dimethyl-propionic acid 2-carbonylmethoxy-5-trifluoromethyl-benzyl ester.

To 2,2-Dimethyl-propionic acid 2-ethoxycarbonylmethoxy-5-trifluoromethyl-benzyl ester (19 mg, 0.068mmol) in THF (0.5 ml) was added a solution of 0.5 N-LiOH (0.5 ml) and the mixture was stirred for 1.5 hrs at room temperature. The resulting residue was dissolved in H₂O (0.5 ml) and then washed three times with EtOAc (0.5 ml), acidified with 1N HCl to pH 1-2. The solution was extracted three times with methylene chloride (1 ml) and then dried over anhyd. Na₂SO₄ and concentrated in vacuo to give 2,2-Dimethyl-propionic acid 2-carbonylmethoxy-5-trifluoromethyl-benzyl ester (16 mg, 70 %).
¹H NMR (300 MHz, CDCl3): δ 7.79 (s, 1H, br), 7.55 ~ 7.53 (m, 2H), 6.83 ∼ 6.79 (m, 1H), 5.20 (s, 2H), 4.74 (d, 2H, *J* =2.1 Hz), 1.27 ~ 1.20 (m, 9H).

### Step 7: 2,2-Dimethyl-propionic acid 2-[(3-fluoro-4-methanesulfonylaminobenzylcarbamoyl)-methoxy]-5-trifluoromethyl-benzyl ester

3-fluoro-4-methanesulfonylaminobenzylamine hydrochloride (12.2 mg, 0.048 mmol) was suspended in THF (0.5 mL) and treated with triethylamine (5.81 mg) and then the resulting mixture was stirred for 10mins. 2,2-Dimethyl-propionic acid 2-carbonylmethoxy-5-trifluoromethyl-benzyl ester (16 mg, 0.048 mmol) was added to the reaction mixture followed by DMTMM (14.6 mg, 0.053 mmol) after 10 mins. The resulting mixture was stirred overnight at ambient temperature and then diluted with EtOAc. The resulting solution was washed successively with water, sat'd NaHCO₃ (x3), 1 N HCl and brine, and then dried over anhyd. MgSO₄, filtered and concentrated under reduced pressure. The crude residue was recrystallized from EtOAC/n-hexane to yield the title compound (20 mg, 78 %).
¹H NMR (300 MHz, CDCl₃): δ 7.84 (s, 1H, br), 7.57- 7.53 (m, 2H), 7.45∼7.40 (m, 1 H), 7.05 ∼ 6.99 (m, 2H), 6.88 (d, 1 H, *J* = 8.7 Hz), 6.52 (s, 1 H), 5.22 (s, 2H), 4.59 (s, 2H), 4.44 (d, 2H, *J* = 6.3 Hz), 2.94 (s, 3H), 1.10 ~ 1.01 (m, 9H).

### Example 59: N-(3-Fluoro-4-methanesulfonylaminobenzyl)-2-(4-chloro-3-fluorophenoxy)acetamide

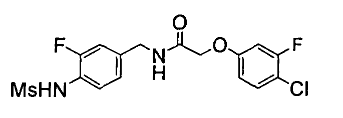

### Step 1: Synthesis of 3-chloro-4-fluorophenoxyacetic acid

To a solution of 3-chloro-4-fluorophenol (0.41 g, 2.80 mmol) in acetonitrile (10 mL) were added cesium carbonate (1.37 g, 4.2 mmol) and ethyl bromoacetate (0.34 mL, 3.01 mmol). The resulting mixture was stirred for 1.5 hours at room temperature, concentrated under reduced pressure, and diluted with EtOAc and water. The organic layer was washed with brine and concentrated under reduced pressure. The crude residue was diluted with THF (7 mL) and 1 N LiOH (5 mL), and then stirred for 1 hour at room temperature. The mixture was acidified with 6 N HCl, concentrated under reduced pressure, and diluted with EtOAc and water. The organic layer was washed with brine, dried over anhydrous magnesium sulfate, filtered, and concentrated under reduced pressure. The crude residue was then purified by recrystallization from EtOAc/hexane to afford 477mg (83.3%) as a white solid.
¹H NMR (300 MHz, CDCl₃): δ 7.31 (t, 1 H, *J* = 8.7 Hz), 6.75 (dd, 1 H, *J* = 10.3, 3 Hz), 6.67 (ddd, 1 H, *J* = 8.7, 3, 1.5 Hz), 4.67 (s, 2 H)

### Step 2: Synthesis of N-(3-fluoro-4-methanesulfonylaminobenzyl)-2-(4-chloro-3-fluorophenoxy) acetamide

To a suspension of 3-fluoro-4-methanesulfonylaminobenzylamine hydrochloride (100 mg, 0.39 mmol) in THF (5 mL) was added triethylamine (82 µL, 0.59 mmol). The mixture was stirred for 5 minutes, to which were added 3-chloro-4-fluorophenoxyacetic acid (85 mg, 0.42 mmol) and 4-(4,6-dimethoxy[1,3,5]triazin-2-yl)-4-methylmorpholinium chloride hydrate (DMTMM, 120 mg, 0.43 mmol). The mixture was stirred overnight at room temperature and was concentrated under reduced pressure. The residue was diluted with EtOAc and water. The organic layer was washed with 3N HCI, saturated sodium bicarbonate, and brine, dried over anhydrous magnesium sulfate, filtered, and concentrated under reduced pressure. The crude residue was purified by recrystallization from EtOAc/hexane to afford 130 mg (81.87%) as a white solid.
Melting point: 146 ~ 147.5°C; ¹H NMR (300 MHz, CDCl₃): δ 7.54 (t, 1 H, *J* = 8.1 Hz), 7.33 (t, 1 H, *J* = 8.7 Hz), 7.09 (d, 2 H, *J* = 9.3 Hz), 6.86 (br, 1 H), 6.76 (dd, 1 H, *J* = 10.5, 3.3 Hz), 6.68 (ddd, 1 H, *J* = 8.9, 2.7, 0.9 Hz), 6.50 (br s, 1 H), 4.53 (s, 3 H), 4.51 (s, 1 H), 3.03 (s, 3 H)

### Example 60: 2-(2'-Chloro-biphenyl-4-yloxy)-N-(3-fluoro-4-methanesulfonylaminobenzyl)-acetamide

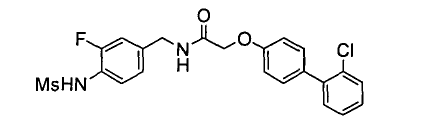

2-(4-Bromo-phenoxy)-N-(3-fluoro-4-methanesulfonylamino-benzyl)-acetamide (125 mg, 0.289 mmol), 2-chlorophenylboronic acid (46.1 mg, 0.295 mmol) and tetrakis(triphenylphosphine)palladium (0) (8.4 mg) were added in toluene and ethanol. 1 M K₂CO₃ (2mL) was added into the reaction mixture. The reaction mixture was heated to 100 °C. A reaction mixture was stirred overnight. A reaction solvent was removed in vacuo. A residue was extracted with MC (25 ml x 3) and H₂O. A combined organic layer was dried with MgSO₄, and then concentrated in vacuo. A residue was purified with column chromatography to yield the title compound (43 mg) as a white solid.
Mp: 102 - 103 °C: ¹H NMR (300MHz, CDCl₃): δ 7.52 (m, 1 H), 7.40 (m, 2H), 7.30 (m, 2H), 7.09 (m, 2H), 7.00 (m, 2H), 6.82 (m, 1H), 6.65 (br s, 1 H), 4.62 (s, 2H), 4.53 (m, 2H), 3.02 (s, 3H)

### Example 61: 2-(2'-Chloro-biphenyl-3-yloxy)-N-(3-fluoro-4-methanesulfonylaminobenzyl)-acetamide

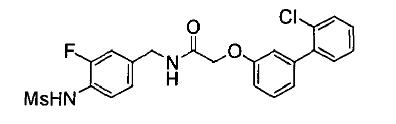

2-(3-Bromo-phenoxy)-N-(3-fluoro-4-methanesulfonylamino-benzyl)-acetamide (31 mg, 0.072 mmol), 2-chlorophenylboronic acid (12 mg, 0.0765 mmol), and tetrakis(triphenylphosphine)palladium (0) (5.1 mg) were added in toluene and ethanol. 1 M K₂CO₃ (2mL) was added into the reaction mixture. The reaction mixture was heated to 100 °C. The reaction mixture was stirred overnight. A reaction solvent was removed in vacuo. A residue was extracted with MC (25 ml x 3) and H₂O. A combined organic layer was dried with MgSO₄, and then concentrated in vacuo. A residue was purified with column chromatography to yield the title compound (21 mg) as a white solid.
Mp: 95 - 97°C: ¹H NMR (300MHz, CDCl₃): δ 7.66 (m, 1H), 7.51 (m, 3H), 7.39 (m, 1 H), 7.30 (m, 3H), 7.09 (m, 2H), 7.01 (m, 2H), 6.96 (m, 1 H), 6.55 (br s, 1H), 4.62 (s, 2H), 4.53 (d, 2H J = 6.1 Hz), 3.01 (s, 3H)

### Example 62: N-(3-Fluoro-4-methanesulfonylaminobenzyl)-2-(indan-5-yloxy)acetamide

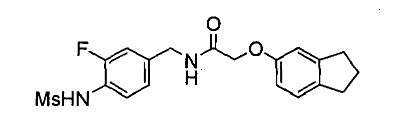

### Step 1: Synthesis of indan-5-yloxyacetic acid

To a solution of 5-indanol (0.38 g, 2.83 mmol) in acetonitrile (10 mL) were added cesium carbonate (1.37 g, 4.2 mmol) and ethyl bromoacetate (0.34 mL, 3.01 mmol). The resulting mixture was stirred for 1.5 hours at room temperature, concentrated under reduced pressure, and diluted with EtOAc and water. The organic layer was washed with brine and concentrated under reduced pressure. The crude residue was diluted with THF (7 mL) and 1 N LiOH (5 mL), and then stirred for 1 hour at room temperature. The mixture was acidified with 6 N HCl, concentrated under reduced pressure, and diluted with EtOAc and water. The organic layer was washed with brine, dried over anhydrous magnesium sulfate, filtered, and concentrated under reduced pressure. The crude residue was then purified by recrystallization from EtOAc/hexane to afford 433 mg (79.6%) as a white solid.
¹H NMR (300 MHz, CDCl₃): δ 7.13 (d, 1 H, *J* = 8.1 Hz), 6.80 (d, 1 H, *J* = 2.1 Hz), 6.71 (dd, 1.H, *J* = 8.4, 2.7 Hz), 4.66 (s, 2 H), 2.88 (t, 2 H, *J* = 7.8 Hz), 2.84 (t, 2 H, *J* = 7.2 Hz), 2.08 (quint, 2 H, *J* = 7.2 Hz)

### Step 2: Synthesis of N-(3-fluoro-4-methanesulfonylaminobenzyl)-2-(indan-5-yloxy)acetamide

To a suspension of 3-fluoro-4-methanesulfonylaminobenzylamine hydrochloride (100 mg, 0.39 mmol) in THF (5 mL) was added triethylamine (82 µL, 0.59 mmol). The mixture was stirred for 5 minutes, to which were added indan-5-yloxyacetic acid (80 mg, 0.42 mmol) and 4-(4,6-dimethoxy[1,3,5]triazin-2-yl)-4-methylmorpholinium chloride hydrate (DMTMM, 120 mg, 0.43 mmol). The mixture was stirred overnight at room temperature and was concentrated under reduced pressure. The residue was diluted with EtOAc and water. The organic layer was washed with 3N HCl, saturated sodium bicarbonate and brine, dried over anhydrous magnesium sulfate, filtered, and concentrated under reduced pressure. The crude residue was purified by recrystallization from EtOAc/hexane to afford 127 mg (82.4%) as a white solid.
Melting point: 118 ~ 120°C; ¹H NMR (300 MHz, CDCl₃): δ 7.52 (t, 1 H, *J=* 7.8 Hz), 7.14 (d, 1 H, *J* = 8.4 Hz), 7.07 (d, 2 H, *J* = 8.7 Hz), 6.97 (br, 1 H), 6.80 (d, 1 H, *J* = 2.1 Hz), 6.70 (dd, 1 H, *J* = 8.1, 2.4 Hz), 6.47 (br s, 1 H), 4.54 (s, 2 H), 4.51 (d, 2 H, *J* = 6.3 Hz), 3.02 (s, 3 H), 2.86 (q, 4 H, *J* = 7.8 Hz), 2.09 (quint, 2 H, *J* = 7.8 Hz)

### Example 63: N-(3-Fluoro-4-methanesulfonylaminobenzyl)-2-(2-cyclohexylphenoxy)acetamide

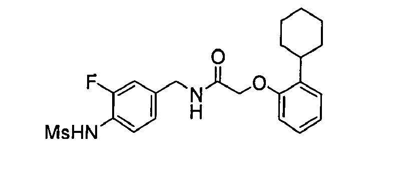

### Step 1: Synthesis of 2-cyclohexylphenoxyacetic acid

To a solution of 2-cyclohexylphenol (0.49 g, 2.78 mmol) in acetonitrile (10 mL) were added cesium carbonate (1.37 g, 4.2 mmol) and ethyl bromoacetate (0.34 mL, 3.01 mmol). The resulting mixture was stirred for 3 hours at room temperature, concentrated under reduced pressure, and diluted with EtOAc and water. The organic layer was washed with brine and concentrated under reduced pressure. The crude residue was diluted with THF (7 mL) and 1 N LiOH (5 mL), and then stirred for 1 hour at room temperature. The mixture was acidified with 6 N HCl, concentrated under reduced pressure, and diluted with EtOAc and water. The organic layer was washed with brine, dried over anhydrous magnesium sulfate, filtered, and concentrated under reduced pressure. The crude residue was then purified by recrystallization from EtOAc/hexane to afford 558 mg (85.7%) as a white solid.
¹H NMR (300 MHz, CDCl₃): δ 7.21 ∼ 7.18 (m, 1 H), 7.13 ∼ 7.07 (m, 1 H), 6.98 ∼ 6.93 (m, 1 H), 6.72 ∼ 6.69 (m, 1 H), 4.66 (s, 2 H), 3.02 ∼ 2.91 (m, 1 H), 1.9 ~ 1.75 (m, 4 H), 1.75 ~ 1.67 (m, 1 H), 1.48 ~ 1.32 (m, 4 H), 1.32 ~ 1.16 (m, 1 H)

### Step 2: Synthesis of N-(3-fluoro-4-methanesulfonylaminobenzyl)-2-(2-cyclohexylphenoxy)acetamide

To a suspension of 3-fluoro-4-methanesulfonylaminobenzylamine hydrochloride (100 mg, 0.39 mmol) in THF (5 mL) was added triethylamine (82 µL, 0.59 mmol). The mixture was stirred for 5 minutes, to which were added 2-cyclohexylphenoxyacetic acid (96 mg, 0.41 mmol) and 4-(4,6-dimethoxy[1,3,5]triazin-2-yl)-4-methylmorpholinium chloride hydrate (DMTMM, 120 mg, 0.43 mmol). The mixture was stirred overnight at room temperature and was concentrated under reduced pressure. The residue was diluted with EtOAc and water. The organic layer was washed with 3N HCl, saturated sodium bicarbonate, and brine, dried over anhydrous magnesium sulfate, filtered, and concentrated under reduced pressure. The crude residue was purified by column chromatography (EtOAc:n-hexane = 1:2) to afford product quantitatively as an oil.
¹H NMR (300 MHz, CDCl₃): δ 7.54 (t, 1 H, *J* = 8.1 Hz), 7.23 ∼ 7.08 (m, 4 H), 7.04 ∼ 6.98 (m, 1 H), 6.93 (br, 1 H), 6.82 ∼ 6.79 (m, 1 H), 6.74 (br s, 1 H), 4.57 (s, 2 H), 4.54 (d, 2 H, *J* = 6 Hz), 3.01 (s, 3 H),), 2.89 ~ 2.77 (m, 1 H), 1.88 ~ 1.64 (m, 5 H), 1.48 ∼ 1.16 (m, 5 H)

### Example 64: N-(3-fluoro-4-methanesulfonylamino-benzyl)-2-(4-Cyclohexylphenoxy)-acetamide

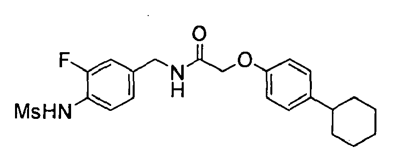

### Step 1: Synthesis of 4-cyclohexylphenoxyacetic acid

To a solution of 4-cyclohexylphenol (242 mg, 1.37 mmol) in acetonitrile (20 mL) were added cesium carbonate (502.1 mg, 1.54 mmol) and ethyl bromoacetate (0.30 mL). The resulting mixture was stirred overnight for at room temperature, concentrated under reduced pressure, and diluted with EtOAc and water. The organic layer was washed with brine and concentrated under reduced pressure. The crude residue was diluted with MeOH (25 mL) and 1 N KOH (5 mL), and then stirred for 1.5 hours at room temperature. The mixture was acidified with 6 N HCI, concentrated under reduced pressure, and diluted with EtOAc and water. The organic layer was washed with brine, dried over anhydrous magnesium sulfate, filtered, and concentrated under reduced pressure. The crude residue was then purified by recrystallization from EtOAc/hexane to afford 212 mg (64%) as a white solid.
¹H NMR (300 MHz, CDCl₃): δ 7.17 ~ 7.15 (m, 2H), 6.88 ∼ 6.85 (m, 2H), 4.66 (s, 2H), 2.48 (m, 1H), 1.865 ~ 1.83 (m, 4H), 1.76 ~ 1.72 (m, 2H), 1.37 ~ 1.34 (m, 4H)

### Step 2: Synthesis of N-(3-fluoro-4-methanesulfonylaminobenzyl)-2-(4-cyclohexylphenoxy)acetamide

To a suspension of 3-fluoro-4-methanesulfonylaminobenzylamine hydrochloride (114 mg, 0.447 mmol) in THF (5 mL) was added NMP (0.3 mL). The mixture was stirred for 20 minutes, to which were added 4-cyclohexylphenoxyacetic acid (104.5 mg, 0.447 mmol ol) and 4-(4,6-dimethoxy[1,3,5]triazin-2-yl)-4-methylmorpholinium chloride hydrate (DMTMM, 142 mg). The mixture was stirred overnight at room temperature and was concentrated under reduced pressure. The residue was diluted with EtOAc and water. The organic layer was washed with 3N HCl, saturated sodium bicarbonate and brine, dried over anhydrous magnesium sulfate, filtered, and concentrated under reduced pressure. The crude residue was purified by column chromatography (EtOAc:n-hexane = 1:1) to afford product (16 mg) as an oil.
¹H NMR (300 MHz, CDCl₃): δ 7.54 (t, 1H, J = 8.1 Hz), 7.17 ∼ 7.14 (m, 2H), 7.07 (m, 1H), 7.08 (m, 1H), 7.01 (m, 1H), 6.86-6.83 (m, 2H), 6.63 (br, 1 H), 4.54 (s, 2H), 4.51 (d, 2H, J = 6.3 Hz), 3.01 (s, 3H), 2.48 (m, 1 H), 1.85 ~ 1.82 (m, 4H), 1.76 ~ 1.72 (m, 2H), 1.41 ~ 1.26 (m, 4H)

### Experimental example: Biological potency test

### 1. ⁴⁵ Ca influx test

### 1) Separation of spinal dorsal root ganglia (DRG) in newborn rats and primary culture thereof

Neonatal (2-3 day old or younger than 2-3 day old) SD rats were put in ice for 5 minutes to anesthetize and disinfected with 70% ethanol. DRG of all part of spinal cord were dissected (Wood et al., 1988, J. Neurosci. 8, pp3208-3220) and collected in DME/F12 medium to which 1.2g/l sodium bicarbonate, 50mg/l gentamycin were added. The DRG were incubated sequentially at 37°C for 30 min in 200 U/ml collagenase and 2.5mg/ml trypsin, separately. The gangiia were washed twice with DME/F12 medium supplemented with 10% horse serum, triturated through a fire-polished Pasteur pipette, filtered through Nitex 80 membrane to obtain single cell suspension and the suspension was washed once more. This was subjected to centrifugation, then resuspended in cell culture medium at certain level of cell density. As the cell culture medium, DME/F12 medium supplemented with 10% horse serum was diluted with identical medium conditioned by C6 glioma cells 2 days on a confluent monolayer (1:1), and NGF (Nerve Growth Factor) was added to adjust 200ng/ml as final concentration. After the cells were grown 2 days in medium where cytosine arabinoside (Ara-C, 100 µM) was added to kill dividing nonneuronal cells, medium was changed to one without Ara-C. The resuspended cells were plated at a density of 1500-2000 neurons/well onto Terasaki plates previously coated with 10 µg/ml poly-D-ornithine.

### 2) ⁴⁵ Ca influx experiments

DRG nerve cells from the primary culture of 2 days were equilibrated by washing 4 times with HEPES (10mM, pH 7.4)-buffered Ca ²⁺, Mg²⁺-free HBSS (H-HBSS). The solution in each well was removed from the individual well. Medium containing the test compound plus capsaicin (final concentration 0.5 µM) and ⁴⁵Ca (final concentration 10 µCi/ml) in H-HBSS was added to each well and incubated at room temperature for 10 mins. Terasaki plates were washed five times with H-HBSS and dried at room temperature. To each well, 0.3% SDS (10 µl) was added to elute ⁴⁵Ca. After the addition of scintillation cocktail of into each well, the amount of ⁴⁵Ca influx into neuron was measured by counting radioactivity. Antagonistic activities of test compounds against vanilloid receptor were calculated as percent of the inhibition of maximal response of capsaicin at a concentration of 0.5 µM.

**[Table 1] Results of Calcium Influx Test**

| **Examples** | **Antagonist** |
|---|---|
| | **Calcium Uptake Test (IC₅₀, µM)** |
| **1** | 0.11 |
| **2** | 0.0086 |
| **3** | 0.18 |
| **4** | 0.081 |
| **5** | 0.034 |
| **6** | 0.019 |
| **7** | 0.13 |
| **8** | 0.11 |
| **9** | 0.33 |
| **10** | 0.16 |
| **11** | 0.096 |
| **12** | 0.071 |
| **13** | 0.16 |
| **14** | 0.76 |
| **15** | 0.68 |
| **16** | - |
| **17** | 0.21 |
| **18** | 0.12 |
| **19** | 1.5 |
| **20** | 0.19 |
| **21** | >10 |
| **22** | >10 |
| **23** | >10 |
| **24** | 1.3 |
| **25** | 2.3 |
| **26** | >10 |
| **27** | >10 |
| **28** | 4.3 |
| **29** | >10 |
| **30** | 3.4 |
| **31** | >10 |
| **32** | >10 |
| **33** | >10 |
| **34** | 8.7 |
| **35** | >10 |
| **36** | 1.2 |
| **37** | >10 |
| **38** | 2.6 |
| **39** | >10 |
| **40** | 1.8 |
| **41** | >10 |
| **42** | >10 |
| **43** | >10 |
| **44** | >10 |
| **45** | 3.7 |
| **46** | 1.7 |
| **47** | 0.15 |
| **48** | 0.18 |
| **51** | >10 |
| **52** | 0.069 |
| **53** | 0.074 |
| **54** | >10 |
| **55** | 5.6 |
| **56** | 5.1 |
| **57** | 0.13 |
| **58** | 3.0 |
| **59** | >10 |
| **60** | >10 |
| **61** | 4.5 |
| **62** | 82% @ 10 µM |
| **63** | 52% @ 10 µM |
| **64** | >10 |

### 2. Analgesic activity test: Mouse writhing test by inducing with phenyl-p-quinone

Male ICR mice (mean body weight 25g) were maintained in a controlled lighting environment (12 h on/ 12 h off) for experiment. Animals received an intraperitoneal injection of 0.3ml of the chemical irritant phenyl-p-quinone (dissolved in saline containing 5% ethanol to be a dose of 4.5mg/kg) and 6 mins later, the number of abdominal constrictions was counted in the subsequent 6 mins period. Animals (10 animals/group) received 0.2ml of test compounds solution in vehicle of ethanol/Tween 80/saline (10/10/80) intraperitoneally 30 min before the injection of phenyl-p-quinone. A reduction in the number of writhes responding to the test drug compound relative to the number responding in saline control group was considered to be indicative of an analgesic effect. Analgesic effect was calculated by % inhibition equation (% inhibition=(C-T)/C x 100), wherein C and T represent the number of writhes in control and compound-treated group, respectively (Table 2).

**[Table 2]**

| Test result of analgesic activity for writhing by phenyl-p-quinone | | |
|---|---|---|
| **Example** | **Dose (mg/kg)** | **Analgesic effect (%Inhibition)** |
| 2 | 0.3 | 51% |
| 3 | 1 | 39% |
| 5 | 1 | 59% |
| 6 | 0.1 | 41% |
| 12 | 1 | 27% |
| 18 | 1 | 33% |
| 24 | 10 | 28% |
| 52 | 1 | 49% |
| 53 | 1 | 49% |

### Industrial Applicability

As explained above, the compound according to the present invention is useful to preventing and treating of pain, migraine, arthralgia, neuralgia, neuropathies, nerve injury, skin disorder, urinary bladder hypersensitiveness, irritable bowel syndrome, fecal urgency, a respiratory disorder, irritation of skin, eye or mucous membrane, stomach-duodenal ulcer, inflammatory diseases, ear disease, and heart disease etc.

More specifically, the compound according to the present invention is useful to preventing and treating of acute pain, chronic pain, neuropathic pain, post-operative pain, rheumatic arthritic pain, osteoarthritic pain, postherpetic neuralgia, diabetic neuropathy, HIV-related neuropathy, neurodegeneration, stroke, neurotic/allergic/inflammatory skin disease, psoriasis, pruritus, prurigo, asthma, chronic obstructive pulmonary disease, urinary incontinence, inflammatory bowel disease, hyperacusis, tinnitus, vestibular hypersensitiveness, and myocardial ischemia.

## Claims

1. A compound of the formula (I), an isomer and/or a pharmaceutically acceptable salt thereof; 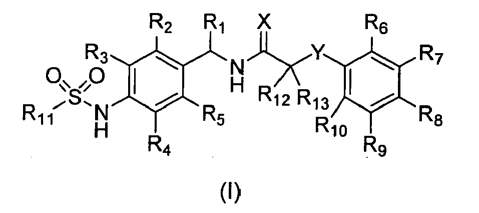 wherein,
X is O, S, or N-CN;
Y is O or S;
R₁ is hydrogen or C1-C5 alkyl;
R₂, R₃, R₄, and R₅ are independently hydrogen, halogen, nitro, cyano, carboxy, C1-C5 alkyl, C2-C5 alkenyl, C2-C5 alkynyl, halo (C1-C5) alkyl, C1-C5 alkylthio, C1-C5 alkoxy, or C1-C5 alkoxycarbonyl;
R₆, R₇, R₉, and R₁₀ are independently hydrogen, hydroxy, halogen, nitro, carboxy, C1-C5 alkyl, C2-C5 alkenyl, C2-C5 alkynyl, halo (C1-C5) alkyl, halo (C1-C5) alkoxy, C1-C5 alkylthio, C1-C5 alkylsulfonyl, C1-C5 alkoxy, C1-C5 alkylcarbonyl, C1-C5 alkoxycarbonyl, C2-C5 alkenyloxy, (C1-C5)alkoxy(C1-C5)alkyl, (C1-C5)alkoxycarbonyl(C1-C5)alkyl, (C1-C5)alkylcarbonyloxy(C1-C3)alkyl, (C1-C5)alkoxy(C1-C5)alkylamino, (C1-C5)alkoxy(C1-C5)alkoxy, C3-C6 cycloalkyl, C3-C5 cycloalkylmethyl, piperidinyl, or morpholinyl;
R₈ is halogen, C1-C5 alkyl, C2-C5 alkenyl, C2-C5 alkynyl, halo (C1-C5) alkyl, halo (C1-C5) alkoxy, C1-C5 alkylthio, C1-C5 alkylsulfonyl, C1-C5 alkoxy, C1-C5 alkylcarbonyl, C1-C5 alkoxycarbonyl, C2-C5alkenyloxy, (C1-C5)alkoxy(C1-C5)alkyl, (C1-C5)alkoxycarbonyl(C1-C5)alkyl, (C1-C5)alkoxy(C1-C5)alkylamino, (C1-C5)alkoxy(C1-C5)alkoxy, C3-C6 cycloalkyl, mono- or di- (C3-C5)cycloalkyl(C1-C3)alkyl, piperidinyl, morpholinyl, phenyl, pyridyl, or pyrimidinyl, wherein each (C3-C5)cycloalkyl may be unsubstituted or substituted with one or more methyl groups, and phenyl may be unsubstituted or substituted with one or more substituent selected from halogen, nitro, carboxy, C1-C5 alkyl, C2-C5 alkenyl, halo (C1-C5) alkyl, or C1-C5 alkoxy;
R₈ and R₉ may also form together -OCH₂O-, C2-C4 alkylene, or -NHN=N-;
R₁₁ is C1-C5 alkyl or C2-C5 alkenyl; and
R₁₂ and R₁₃ are hydrogen, halogen, or C1-C5 alkyl.

2. A compound according to claim 1, an isomer and/or a pharmaceutically acceptable salt thereof;
wherein,
X is O, S, or N-CN,
Y is O or S;
R₁ is hydrogen or C1-C3 alkyl;
R₂, R₃, R₄, and R₅ are independently hydrogen, halogen, nitro, cyano, methyl, ethyl, ethenyl, ethynyl, trifluoromethyl, methoxy, ethoxy, or methoxycarbonyl;
R₆, R₇, R₉, and R₁₀ are independently hydrogen, hydroxy, halogen, nitro, carboxy, C1-C5 alkyl, C2-C5 alkenyl, C2-C5 alkynyl, halo (C1-C5) alkyl, halo (C1-C5) alkoxy, C1-C5 alkylthio, C1-C5 alkylsulfonyl, C1-C5 alkoxy, C1-C5 alkylcarbonyl, C1-C5 alkoxycarbonyl, C2-C5alkenyloxy, (C1-C5)alkoxy(C1-C5)alkyl, (C1-C5)alkoxycarbonyl(C1-C5)alkyl, (C1-C5)alkylcarbonyloxy(C1-C3)alkyl, (C1-C5)alkoxy(C1-C5)alkylamino, (C1-C5)alkoxy(C1-C5)alkoxy, C3-C6 cycloalkyl, piperidinyl, or morpholinyl;
R₈ is halogen, C1-C5 alkyl, C2-C5 alkenyl, C2-C5 alkynyl, halo (C1-C5) alkyl, halo (C1-C5) alkoxy, C1-C5 alkoxy, C1-C5 alkylcarbonyl, C1-C5 alkoxycarbonyl, C2-C5alkenyloxy, (C1-C5)alkoxy(C1-C5)alkyl, C3-C6 cycloalkyl, mono- or di- (C3-C5)cycloalkyl(C1-C3)alkyl, piperidinyl, or morpholinyl, wherein each C3-C5 cycloalkyl may be unsubstituted or substituted with one or more methyl groups;
R₈ and R₉ may also form together -OCH₂O-, C2-C4 alkylene, or -NHN=N-;
R₁₁ is C1-C3 alkyl or C2-C3 alkenyl; and
R₁₂ and R₁₃ are hydrogen, halogen, or C1-C3 alkyl.

3. A compound of the formula (II), an isomer and/or a pharmaceutically acceptable salt thereof; 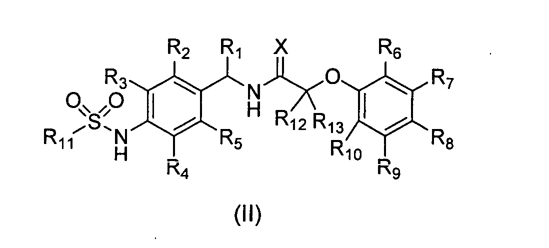 wherein,
X is O, S, or N-CN;
R₁ is hydrogen or C1-C5 alkyl;
R₂, R₃, R₄, and R₅ are independently hydrogen, halogen, nitro, cyano, methyl, ethyl, ethenyl, ethynyl, trifluoromethyl, methoxy, ethoxy, or methoxycarbonyl;
R₆, R₇, R₉, and R₁₀ are independently hydrogen, hydroxy, halogen, nitro, carboxy, C1-C5 alkyl, C2-C5 alkenyl, C2-C5 alkynyl, halo (C1-C5) alkyl, halo (C1-C5) alkoxy, C1-C5 alkylthio, C1-C5 alkylsulfonyl, C1-C5 alkoxy, C1-C5 alkylcarbonyl, C1-C5 alkoxycarbonyl, C2-C5alkenyloxy, (C1-C5)alkoxy(C1-C5)alkyl, (C1-C5)alkoxycarbonyl(C1-C5)alkyl, (C1-C5)alkylcarbonyloxy(C1-C3)alkyl, (C1-C5)alkoxy(C1-C5)alkylamino, (C1-C5)alkoxy(C1-C5)alkoxy, C3-C6 cycloalkyl, C3-C5 cycloalkylmethyl, piperidinyl, or morpholinyl;
R₈ is halogen, C1-C5 alkyl, C2-C5 alkenyl, C2-C5 alkynyl, halo (C1-C5) alkyl, halo (C1-C5) alkoxy, C1-C5 alkoxy, C1-C5 alkylcarbonyl, C1-C5 alkoxycarbonyl, C2-C5alkenyloxy, (C1-C5)alkoxy(C1-C5)alkyl, (C1-C5)alkoxycarbonyl(C1-C5)alkyl, (C1-C5)alkoxy(C1-C5)alkylamino, (C1-C5)alkoxy(C1-C5)alkoxy, C3-C6 cycloalkyl, mono- or di- (C3-C5)cycloalkyl(C1-C3)alkyl, piperidinyl, morpholinyl, or phenyl, wherein each (C3-C5)cycloalkyl may be unsubstituted or substituted with one or more methyl groups, and phenyl may be unsubstituted or substituted with one or more substituent selected from halogen, nitro, carboxy, C1-C5 alkyl, C2-C5 alkenyl, halo (C1-C5) alkyl, or C1-C5 alkoxy;
R₈ and R₉ may also form together -OCH₂O-, C2-C4 alkylene, or -NHN=N-;
R₁₁ is C1-C5 alkyl or C2-C5 alkenyl; and
R₁₂ and R₁₃ are hydrogen, halogen, or C1-C5 alkyl.

4. A compound according to anyone of claims 1 to 3, an isomer and/or a pharmaceutically acceptable salt thereof;
wherein,
X is O, S, or N-CN;
R₁ is hydrogen, methyl, or ethyl;
R₂, R₃, R₄, and R₅ are independently hydrogen, fluoro, chloro, bromo, nitro, cyano, methyl, ethyl, ethenyl, ethynyl, trifluoromethyl, methoxy, ethoxy, or methoxycarbonyl;
R₆, R₇, R₉, and R₁₀ are independently hydrogen, halogen, nitro, carboxy, methyl, ethyl, propyl, isopropyl, butyl, isobutyl, sec-butyl, t-butyl, ethenyl, propenyl, 2-propynyl, trifluoromethyl, pentafluoroethyl, heptafluoropropyl, hexafluoropropyl, trifluoromethoxy, methylthio, methoxy, ethoxy, propoxy, allyloxy, acetyl, t-butoxycarbonylmethyl, ethoxycarbonylethyl, methoxycarbonylmethyl, t-butylcarbonyloxymethyl, ethylcarbonyloxymethyl, isopropylcarbonyloxymethyl, methoxyethylamino, methoxyethoxy, C3-C6 cycloalkyl, piperidinyl, or morpholinyl;
R₈ is fluoro, chloro, bromo, iodo, C3-C5 alkyl, halo (C1-C5) alkyl, C2-C5 alkynyl, propenyl, butenyl, trifluoromethoxy, pentafluoroethoxy, ethoxy, propoxy, isopropoxy, acetyl, propionyl, allyloxy, methoxyethyl, C3-C6 cycloalkyl, mono-cyclopropylmethyl, dicyclopropylmethyl, 2,2-dimethylcyclopropyl, 2,3-dimethylcyclopropyl, piperidinyl, or morpholinyl;
R₈ and R₉ may also form together-OCH₂O-, C2-C4 alkylene, or NHN=N-;
R₁₁ is C1-C3 alkyl or C2-C3 alkenyl; and
R₁₂ and R₁₃ are hydrogen, halogen, or C1-C3 alkyl.

5. A compound according to anyone of claims 1 to 4, an isomer and/or a pharmaceutically acceptable salt thereof;
wherein,
X is O, S, or N-CN;
R₁ is hydrogen, methyl, or ethyl;
R₃ and R₄ are independently hydrogen, fluoro, chloro, bromo, nitro, cyano, methyl, ethyl, ethenyl, ethynyl, trifluoromethyl, methoxy, ethoxy, or methoxycarbonyl;
R₂, R₅, and R₁₀ are hydrogen;
R₆, R₇, and R₉ are independently hydrogen, halogen, nitro, carboxy, methyl, ethyl, propyl, isopropyl, butyl, isobutyl, sec-butyl, t-butyl, ethenyl, propenyl, 2-propynyl, trifluoromethyl, pentafluoroethyl, heptafluoropropyl, trifluoromethoxy, methylthio, methoxy, ethoxy, propoxy, allyloxy, acetyl, t-butoxycarbonylmethyl, ethoxycarbonylethyl, t-butylcarbonyloxymethyl, ethylcarbonyloxymethyl, isopropylcarbonyloxymethyl, methoxyethylamino, methoxyethoxy, cyclopropyl, cyclobutyl, piperidinyl, or morpholinyl;
R₈ is selected from C3-C5 alkyl, halo (C1-C5) alkyl, or C2-C5 alkynyl;
R₁₁ is methyl; and
R₁₂ and R₁₃ are hydrogen, fluoro, chloro, methyl, or ethyl.

6. A compound according to anyone of claims 1 to 5, an isomer and/or a pharmaceutically acceptable salt thereof;
wherein,
X is O, or N-CN;
R₁ is hydrogen, methyl, or ethyl;
R₃ and R₄ are independently hydrogen, fluoro, chloro, bromo, nitro, cyano, methyl, ethyl, ethenyl, ethynyl, trifluoromethyl, methoxy, ethoxy, or methoxycarbonyl;
R₂, R₅, and R₁₀ are hydrogen;
R₆, R₇, and R₉ are independently hydrogen, halogen, nitro, methyl, ethyl, propyl, isopropyl, butyl, isobutyl, sec-butyl, t-butyl, ethenyl, propenyl, 2-propynyl, trifluoromethyl, pentafluoroethyl, trifluoromethoxy, methylthio, methoxy, ethoxy, propoxy, allyloxy, acetyl, t-butoxycarbonylmethyl, ethoxycarbonylethyl, t-butylcarbonyloxymethyl, ethylcarbonyloxymethyl, isopropylcarbonyloxymethyl, methoxyethylamino, methoxyethoxy, cyclopropyl, or morpholinyl; R₈ is _ isopropyl, sec-butyl, t-butyl, isobutyl, t-amyl, trifluoromethyl, pentafluoroethyl, heptafluoropropyl, heptafluoro-iso-propyl, nonafluorobutyl, nonafluoro-t-butyl, 2-propynyl;
R₁₁ is methyl; and
R₁₂ and R₁₃ are hydrogen, methyl, or ethyl.

7. A compound according to anyone of claims 1 to 6, an isomer and/or a pharmaceutically acceptable salt thereof;
wherein,
X is O or N-CN;
R₁ is hydrogen or methyl;
R₃ and R₄ are independently hydrogen, fluoro, chloro, bromo, nitro, cyano, methyl, ethyl, , ethenyl, ethynyl, or trifluoromethyl;
R₂, R₅, and R₁₀ are hydrogen;
R₆, R₇, and R₉ are independently hydrogen, halogen, nitro, methyl, ethyl, propyl, isopropyl, butyl, isobutyl, sec-butyl, t-butyl, ethenyl, propenyl, 2-propynyl, trifluoromethyl, pentafluoroethyl, trifluoromethoxy, methylthio, methoxy, ethoxy, propoxy, allyloxy, acetyl, t-butoxycarbonylmethyl, ethoxycarbonylethyl, t-butylcarbonyloxymethyl, ethylcarbonyloxymethyl, methoxyethylamino, methoxyethoxy, cyclopropyl, morpholinyl;
R₈ is isopropyl, sec-butyl, t-butyl, isobutyl, t-amyl, trifluoromethyl, pentafluoroethyl, heptafluoropropyl, or 2-propynyl;
R₁₁ is methyl; and
R₁₂ and R₁₃ are hydrogen or methyl.

8. A compound according to anyone of claims 1 to 6, an isomer and/or a pharmaceutically acceptable salt thereof;
wherein,
X is O or N-CN;
R₁ is hydrogen or methyl;
R₃ and R₄ are independently hydrogen, fluoro, chloro, bromo, nitro, cyano, methyl, ethyl, ethenyl, ethynyl, trifluoromethyl, methoxy, ethoxy, or methoxycarbonyl;
R₂, R₅, and R₁₀ are hydrogen;
R₆ and R₉ are independently hydrogen, halogen, nitro, methyl, ethyl, propyl, isopropyl, butyl, isobutyl, sec-butyl, t-butyl, ethenyl, propenyl, 2-propynyl, trifluoromethyl, pentafluoroethyl, trifluoromethoxy, methylthio, methoxy, ethoxy, propoxy, allyloxy, acetyl, t-butoxycarbonylmethyl, t-butylcarbonyloxymethyl, ethylcarbonyloxymethyl, methoxyethylamino, methoxyethoxy, cyclopropyl, or morpholinyl;
R₇ is hydrogen or halogen;
R₈ is isopropyl, sec-butyl, t-butyl, isobutyl, pentafluoroethyl, or heptafluoropropyl;
R₁₁ is methyl; and
R₁₂ and R₁₃ are hydrogen or methyl.

9. A compound according to anyone of claims 1 to 6, an isomer and/or a pharmaceutically acceptable salt thereof;
wherein,
X is O;
R₁ is hydrogen or methyl;
R₃ is hydrogen, fluoro, chloro, bromo, nitro, cyano, methyl, ethyl, ethenyl, ethynyl, or methoxy;
R₄ is hydrogen, fluoro, chloro, methyl or ethyl;
R₂, R₅, R₁₀, and R₁₂ are hydrogen;
R₆ and R₉ are independently hydrogen, fluoro, chloro, bromo, iodo, nitro, methyl, ethyl, propyl, isopropyl, butyl, isobutyl, sec-butyl, t-butyl, ethenyl, propenyl, 2-propynyl, trifluoromethyl, pentafluoroethyl, trifluoromethoxy, methylthio, methoxy, ethoxy, propoxy, t-butylcarbonyloxymethyl, methoxyethylamino, methoxyethoxy, or morpholinyl;
R₇ is hydrogen or fluoro;
R₈ is isopropyl, sec-butyl, t-butyl, isobutyl, or pentafluoroethyl;
R₁₁ is methyl; and
R₁₃ is methyl or hydrogen.

10. A compound according to anyone of the preceding claims, an isomer and/or a pharmaceutically acceptable salt thereof;
wherein,
X is O;
R₁ is hydrogen or methyl;
R₃ is hydrogen, fluoro, chloro, methyl, ethyl, ethenyl, or ethynyl;
R₄ is hydrogen, fluoro, chloro, methyl or ethyl;
R₂, R₅, R₁₀, R₁₂, and R₁₃ are hydrogen;
R₆ and R₉ are independently hydrogen, fluoro, chloro, bromo, nitro, methyl, ethyl, ethenyl, trifluoromethyl, trifluoromethoxy, methoxy, ethoxy, methoxyethylamino, methoxyethoxy, or morpholinyl;
R₇ is hydrogen or fluoro;
R₈ is t-butyl or isopropyl; and
R₁₁ is methyl.

11. A compound according to anyone of claims 4-10, an isomer and/or a pharmaceutically acceptable salt thereof, wherein Y is O.

12. A compound according to claim 1 or 2, an isomer and/or a pharmaceutically acceptable salt thereof, wherein the compound is selected from the group consisting of
2-(4-tert-Butylphenoxy)-N-(4-methanesulfonylaminobenzyl)acetamide,
2-(4-tert-Butylphenoxy)-N-(3-fluoro-4-methanesulfonylaminobenzyl)acetamide,
2-(4-tert-Butylphenoxy)-N-(4-methanesulfonylamino-3-vinylbenzyl)acetamide,
2-(4-tert-Butylphenoxy)-N-(3-fluoro-4-methanesulfonylamino-5-vinylbenzyl)acetamide,
2-(4-tert-Butylphenoxy)-N-(3-ethynyl-5-fluoro-4-methanesulfonylaminobenzyl)acetamide,
2-(4-tert-Butylphenoxy)-N-(3-ethyl-5-fluoro-4-methanesulfonylaminobenzyl)acetamide,
2-(4-tert-Butylphenoxy)-N-(3-chloro-4-methanesulfonylamino-5-methylbenzyl)acetamide,
2-(4-tert-Butylphenoxy)-N-(5-fluoro-4-methanesulfonylamino-2-methylbenzyl)acetamide,
2-(4-tert-Butylphenoxy)-N-(3-chloro-4-methanesulfonylaminobenzyl)acetamide,
2-(4-tert-Butylphenoxy)-N-(3-fluoro-4-methanesulfonylamino-5-methylbenzyl) acetamide,
(R)-2-(4-tert-Butylphenoxy)-N-[1-(4-methanesulfonylaminophenyl)ethyl]acetamide,
(R)-2-(4-tert-Butylphenoxy)-N-[1-(3-fluoro-4-methanesulfonylaminophenyl)ethyl]acetamide,
(R)-2-(4-tert-Butylphenoxy)-N-(3-fluoro-4-methanesulfonylaminobenzyl)propionamide,
(R)-2-(4-tert-Butylphenoxy)-N-[1-(4-methanesulfonylaminophenyl)ethyl]-N'-cyanoacetamidine,
2-(4-tert-Butylphenoxy)-N-(3-fluoro-4-methanesulfonylaminobenzyl)-N'-cyanoacetamidine,
2-(4-tert-Butylphenylsulfanyl)-N-(3-ethynyl-5-fluoro-4-methanesulfonylaminobenzyl)acetamide,
2-[4-(*tert*-butyl)phenoxy]-N-(3-cyano-5-fluoro-4-methanesulfonylaminobenzyl)acetamide,
2-(4-tert-Butylphenoxy)-2-fluoro-N-(3-fluoro-4-methanesulfonylaminobenzyl)acetamide,
N-(3-Fluoro-4-methanesulfonylaminobenzyl)-2-(4-isopropylphenoxy)acetamide, and,
N-(3-Fluoro-4-methanesulfonylaminobenzyl)-2-(4-sec-butylphenoxy)acetamide.

13. A compound according to anyone of claims 1 to 7, an isomer or a pharmaceutically acceptable salt thereof, wherein the compound is selected from the group consisting of
2-(4-tert-Butylphenoxy)-N-(4-methanesutfonylaminobenzyl)acetamide,
2-(4-tert-Butylphenoxy)-N-(3-fluoro-4-methanesulfonylaminobenzyl)acetamide,
2-(4-tert-Butylphenoxy)-N-(4-methanesulfonylamino-3-vinylbenzyl)acetamide,
2-(4-tert-Butylphenoxy)-N-(3-fluoro-4-methanesulfonylamino-5-vinylbenzyl)acetamide,
2-(4-tert-Butylphenoxy)-N-(3-ethynyl-5-fluoro-4-methanesulfonylaminobenzyl)acetamide,
2-(4-tert-Butylphenoxy)-N-(3-chloro-4-methanesulfonylamino-5-methylbenzyl)acetamide,
(R)-2-(4-tert-Butylphenoxy)-N-[1-(4-methanesulfonylaminophenyl)ethyl]acetamide
(R)-2-(4-tert-Butylphenoxy)-N-[1-(3-fluoro-4-methanesulfonylaminophenyl)ethyl]acetamide,
2-[4-(tert-butyl)phenoxy]-N-3-cyano-5-fluoro-4-[(methanesulfonyl) amino]benzylacetamide,
N-(3-Fluoro-4-methanesulfonylaminobenzyl)-(4-isopropylphenoxy)acetamide, and
N-(3-Fluoro-4-methanesulfonylaminobenzyl)-(4-sec-butylphenoxy)acetamide.

14. A compound according to anyone of the preceding claims for use as a medicament.

15. A pharmaceutical composition comprising a compound, an isomer thereof, or a pharmaceutically acceptable salt thereof according to anyone of claims 1 to 13, as an active ingredient, together with a pharmaceutically acceptable carrier.

16. A pharmaceutical composition for preventing and treating a condition associated with the pathological stimulation and/or aberrant expression of vanilloid receptors, wherein said composition comprise a compound, an isomer thereof, or a pharmaceutically acceptable salt thereof according to anyone of claims 1 to 13; and a pharmaceutically acceptable carrier.

17. The pharmaceutical composition according to claims 15 or 16, for treating a condition selected from pain, inflammatory disease of the joints, urinary bladder hypersensitivity including urinary incontinence, stomach duodenal ulcer, irritable bowel syndrome (IBS) and inflammatory bowel disease (IBD), neurotic/allergic/inflammatory skin disease, psoriasis, asthma, chronic obstructive pulmonary disease, pruritus, and prurigo.

18. The pharmaceutical composition according to claim 17 wherein the pain is or is associated with a condition selected from osteoarthritis, rheumatoid arthritis, ankylosing spondylitis, diabetic neuropathic pain, post-operative pain, non-inflammatory musculoskeletal pain (including fibromyalgia, myofascial pain syndrome and back pain), migraine and other types of headaches.

19. The pharmaceutical composition according to anyone of claims 15-18 **characterized in that** it is adapted for oral administration.

20. A method for inhibiting vanilloid ligand from binding to vanilloid receptor in a patient, comprising contacting cells expressing vanilloid receptor in the patient with a compound, an isomer thereof, or a pharmaceutically acceptable salt thereof according to anyone of claims 1 to 13.

21. A method for preventing or treating a condition selected from pain, inflammatory disease of the joints, urinary bladder hypersensitivity including urinary incontinence, stomach duodenal ulcer, irritable bowel syndrome (IBS) and inflammatory bowel disease (IBD), neurotic/allergic/inflammatory skin disease, psoriasis, asthma, chronic obstructive pulmonary disease, pruritus, and prurigo, which comprises administering to a mammal including a person in need thereof a therapeutically effective amount of a compound, an isomer thereof, or a pharmaceutically acceptable salt thereof according to anyone of claims 1 to 13.

22. A method according to claim 21, wherein the pain is or is associated with a condition selected from osteoarthritis, rheumatoid arthritis, ankylosing spondylitis, diabetic neuropathic pain, post-operative pain, non-inflammatory musculoskeletal pain (including fibromyalgia, myofascial pain syndrome and back pain), migraine, and other types of headaches.

23. Use of a compound, an isomer thereof, or a pharmaceutically acceptable salt thereof according to anyone of claims 1 to 13 for the prevention or treatment of a condition that is associated with the aberrant expression and/or aberrant activation of a vanilloid receptor.

24. Use of a compound, an isomer thereof, or a pharmaceutically acceptable salt thereof according to anyone of claims 1 to 13, in preparation of a medicament for the prevention or treatment of a condition that is selected from pain, inflammatory disease of the joints, urinary bladder hypersensitivity including urinary incontinence, stomach duodenal ulcer, irritable bowel syndrome (IBS) and inflammatory bowel disease (IBD), neurotic/allergic/inflammatory skin disease, psoriasis, asthma, chronic obstructive pulmonary disease, pruritus, and prurigo.

25. Use of a compound according to claim 24, wherein the condition is pain, which is or which is associated with a condition selected from osteoarthritis, rheumatoid arthritis, ankylosing spondylitis, diabetic neuropathic pain, post-operative pain, non-inflammatory musculoskeletal pain (including fibromyalgia, myofascial pain syndrome and back pain), migraine, and other types of headaches.
